# FASCICULE DE BREVET EUROPEEN

(11) **EP 1 597 258 B1**
(45) Date de publication et mention de la délivrance du brevet: **05.11.2008**
(21) Numéro de dépôt: 04710901.2
(22) Date de dépôt: 13.02.2004
(51) Int. Cl.: C07D 473/34, A61K 31/52, A61P 31/10, A61P 25/28, A61P 35/00, A61P 25/34

(54) **NOUVEAUX DERIVES DE LA PURINE, LEUR PROCEDE DE PREPARATION, LEUR APPLICATION A TITRE DE MEDICAMENTS, COMPOSITIONS PHARMACEUTIQUES ET NOUVELLE UTILISATION**
NEUE PURINDERIVATE, DEREN HERSTELLUNGSVERFAHREN, DEREN VERWENDUNG ALS ARZNEIMITTEL, PHARMAZEUTISCHE ZUSAMMENSETZUNGEN UND NEUE VERWENDUNG
NOVEL PURINE DERIVATIVES, PREPARATION METHOD THEREOF, APPLICATION OF SAME AS MEDICAMENTS, PHARMACEUTICAL COMPOSITIONS AND NOVEL USE

(30) Priorité: 18.02.2003 FR 0301915
(43) Date de publication de la demande: 23.11.2005
(73) Titulaire: Aventis Pharma S.A., 92160 Antony (FR)
(72) Inventeur: BORDON-PALLIER, Florence, F-78280 Guyancourt (FR); HAESSLEIN, Jean-Luc, F-01480 Jassans-Riottier (FR)
(86) Numéro de dépôt international: PCT/FR2004/000330
(87) Numéro de publication internationale: WO 2004/073595

(56) Documents cités:
- WO-A-00/44750
- WO-A-92/12718
- WO-A-02/051843
- M. HAIDOUNE; R. MORNET: "Synthesis of 6-(Pyrrol-1-yl)purine and of some of its 9-Glycosides" J. HETEROCYCLIC CHEM., vol. 31, 1994, pages 1461-1464, XP002301729

## Description

La présente invention concerne de nouveaux dérivés de la purine, leur procédé de préparation, les nouveaux intermédiaires obtenus, leur application à titre de médicaments, les compositions pharmaceutiques les renfermant et la nouvelle utilisation de tels dérivés de la purine.

L'invention a ainsi pour objet de nouveaux dérivés de la purine.

L'article « M. Haidoune ; R. Mornet, J. Heterocuclic Chem., 31, 1994, pages 1461-1464 » décrit un procédé de synthèse de 6-(pyrrol-1-yl)purine.

WO 02/051843 décrit des dérivés de purine substitués en position 6 par une amine non-cyclique mais pouvant porter un hétérocycle azoté.

WO 92/12718 décrit des dérivés d'adénosine porteurs éventuellement d'une amine cyclique à 5 chaînons benzocondensée.

WO 00/44750 décrit des dérivés de purine substitués en position 6 par NHR₁ (c'est-à-dire une amine non-cyclique).

Les produits de la présente invention possèdent des propriétés inhibitrices de protéines kinases.

Parmi les kinases inhibées, on peut citer notamment les protéines-kinases cycline-dépendantes appelées 'cdk' notamment CDK1 et CDK2, GSK, GSK3Beta, CIV1, SARC, SRC kinase ()Abl kinase, CAM kinase II, casein kinase II, EGF-tyrosine kinase, IRK kinase, Map kinase (ERK 42), MEK1 kinase, PKA, Protein kinase p5611ck, Zap70, AKT ; FAK, JNK3, PLK1.
Les protéines kinase sont une famille d'enzyme qui catalysent la phosphorylation de groupes hydroxy de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, on peut citer notamment FAK (Focal Adhesion Kinase).

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999 ; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 : 1461-1469. 1999]. Phosphatidylinositol-3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91: 10148-10152. 1994; Ling et al. J. Cell. Biochem. 73 : 533-544. 1999]. Le complexe FAK/Src phosphoryle différents substrats comme la paxilline et p130CAS dans les fibroblastes [Vuori et al. Mol. Cell. Biol. 16: 2606-2613. 1996].

Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaires in vitro. Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucléotides anti-sens de FAK perdent leur adhésion et entrent en apoptose (Xu et al, Cell Growth Differ. 4:413-418. 1996). Il a également été démontré que FAK promeut la migration des cellules in vitro. Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112 : 2677-91, 1999]. La surexpression du domaine C-terminal de FAK (FRNK) bloque l'étirement des cellules adhérentes et réduit la migration cellulaire in vitro [Richardson A. and Parsons J.T. Nature. 380 : 538-540, 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires in vitro, suggère le rôle potentiels de FAK dans les processus néoplasiques. Une étude récente a effectivement démontré l'augmentation de la prolifération des cellules tumorales in vivo après induction de l'expression de FAK dans des cellules d'astrocytome humain [Cary L.A. et al. J. Cell Sci. 109 : 1787-94, 1996 ; Wang D et al. J. Cell Sci. 113 : 4221-4230, 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet 342 (8878) : 1024-1025. 1993 ; Owens et al. Cancer Research 55 : 2752-2755, 1995 ; Maung K. et al. Oncogene 18 : 6824-6828, 1999 ; Wang D et al. J. Cell Sci. 113 : 4221-4230, 2000].

L'étude des mécanismes moléculaires qui contrôlent le cycle cellulaire a permis de mettre en évidence le rôle des cdk ainsi définies : ces Cdk sont des régulateurs essentiels du cycle de division cellulaire les cdk sont des protéines constituées d'au moins deux sous-unités, une sous-unité catalytique (dont cdc2 est le prototype) et une sous-unité régulatrice (cycline). On connaît ainsi un certain nombre de cdk. Les cdk forment donc des complexes protéiques dont chacun est impliqué dans une phase du cycle cellulaire.
De nombreux documents de la littérature décrivent l'existence et le rôle des cdk et à titre d'exemple, on peut citer notamment le document WO 97/20842.

Plusieurs inhibiteurs de kinases ont été décrits comme la butyrolactone, le flavopiridol et la 2(2-hydroxyéthylamino)-6-benzylamino-9-méthylpurine appelée olomoucine).

De telles protéines kinases activatrices de Cdk sont notamment celles de champignons pathogènes qui causent des maladies infectieuses dans l'organisme humain.
Comme champignons pathogènes, dans le cadre de la présente invention, on peut citer Candida albicans mais par exemple et tout aussi bien : Candida stellatoidea, Candida tropicalis, Candida parapsilosis, Candida krusei, Candida pseudotropicalis, Candida quillermondii, Candida glabrata, Candida lusianiae ou Candida rugosa ou encore des mycètes du type Saccharomyces cerevisiae, du type Aspergillus ou Cryptococcus et notamment, par exemple, Aspergillus fumigatus, Coccidioides immitis, Cryptococcus neoformans, Histoplasma capsulatum, Blastomyces dermatitidis, Paracoccidioides brasiliens et Sporothrix schenckii ou encore des mycètes des classes des phycomycètes or eumycètes en particulier les sous-classes de basidiomycètes, ascomycètes, mehiascomycétales (levure) et plectascales, gymnascales (champignon de la peau et des cheveux) ou de la classe des hyphomycètes, notamment les sous-classes conidiosporales et thallosporales parmi lesquels les espèces suivantes : mucor, rhizopus, coccidioides, paracoccidioides (blastomyces, brasiliensis), endomyces (blastomyces), aspergillus, menicilium (scopulariopsis), trichophyton, epidermophton, microsporon, piedraia, hormodendron, phialophora, sporotrichon, cryptococcus, candida, geotrichum, trichosporon ou encore toropsulosis, pityriasis Versicolor ou Erythrasma.

Parmi de tels champignons pathogènes, on peut citer tout particulièrement Candida albicans.

On peut noter que les premières kinases activatrices de Cdk de champignons ont été identifiées chez Saccharomycès cerevisiae et Schizosaccharomycès pombe. L'activation des Cdk nécessite à la fois la fixation d'une molécule de cycline et la phosphorylation de la Cdk sur un résidu thréonine conservé, situé dans une région appelée 'boucle T'. Il a été montré que cette phosphorylation est effectuée par une kinase appelée 'kinase activatrice des Cdk' ou 'CAK'. A titre de complément d'information sur ces 'CAK', on peut citer les contenus des documents référencés comme suit :
- 'Solomon, Trends Biochem. Sci. 19, 496-500 (1994)
- 'Buck et al, EMBO J., 14(24), 6173-83 (1995)
- 'Damagnez et al, EMBO J., 14(24), 6164-72 (1995)

Chez la levure Saccharomycès cerevisiae, on a identifié une kinase responsable de l'activité CAK que l'on a appelée CIV1.

A titre de complément d'information sur ces 'CIV1', on peut citer les contenus des documents référencés comme suit :
- Thuret et al, Cell, 86(4), (1996)
- Kaldis et al, Cell, 86(4), 553-564 (1996),
- Espinosa et al, Science, 273(5282), 1714-1717 (1996)

Une telle activité CAK telle que définie ci-dessus essentielle pour la survie et la division cellulaire a été retrouvée et identifiée chez des champignons pathogènes telles que notamment Candida albicans : la séquence du gène codant pour cette protéine CIV1 chez Candida albicans appelée CaCIV1 et la protéine CACIV1 ont été identifiés. Une telle séquence et sa protéine sont clairement définies dans la demande de brevet française n° 9710287.

De tels inhibiteurs de protéines kinases peuvent notamment posséder des propriétés anti-prolifératives.

On a maintenant, et c'est notamment l'objet de la présente invention, trouvé des produits de formule (I) telle que définie ci-après qui possèdent des propriétés inhibitrices de protéines kinases CIV1 de champignons, ces protéines kinases étant activatrices de Cdk.

Ainsi la présente invention concerne des produits de formule (I) telle que définie ci-après qui peuvent posséder notamment des propriétés inhibitrices d'une telle protéine CIV1 que l'on peut trouver dans différents champignons tels que définis ci-dessus.

La présente invention est ainsi notamment relative à des produits de formule (I) telle que définie ci-après qui peuvent posséder plus particulièrement des propriétés inhibitrices de la protéine kinase CaCIV1 de Candida albicans telle que définie ci-dessus et décrite dans la demande de brevet française n° 9710287.

De tels inhibiteurs d'une protéine CIV1 essentielle pour la survie cellulaire de levures peuvent être utilisés comme agents antifongiques, soit en tant que médicaments soit sur le plan industriel.

Leurs propriétés inhibitrices permettent ainsi d'utiliser des produits de la présente invention comme fongicides pour traiter des maladies causées par de tels champignons pathogènes.

Le spectre des infections fongiques connues s'étend de l'attaque fongique de la peau ou des ongles à des infections mycotiques plus graves d'organes internes. De telles infections et les maladies qui en résultent sont identifiées comme des mycoses. Des substances antimycotiques à effets fongistatiques ou fongicides, sont utilisées pour le traitement de ces mycoses.

La présente invention concerne également le procédé de préparation de tels inhibiteurs, leur utilisation comme agent antifongique et les compositions pharmaceutiques contenant de tels inhibiteurs.

La présente invention a ainsi pour objet les produits de formule (I): 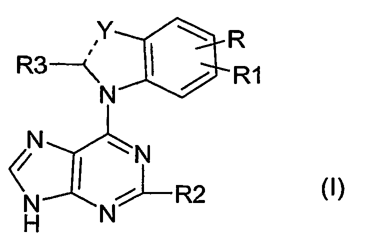 dans laquelle:
Y représente N, O, S, CHR3 ou =CR3
la ligne en pointillé sur le cycle indiquant que la liaison correspondante est simple ou double,
R et R1, identiques ou différents, représentent hydrogène, halogène, hydroxyle, alkyle, alcoxy, cyano, NO2, NR4R5, trifluorométhyle, trifluorométhoxy, aryle, hétéroaryle, -S(O)n-NR4R5, acyle, -NH-CO-alkyle ou -NH-CO-NH-phényle dans lesquels les radicaux alkyle et phényle sont éventuellement substitués par un ou plusieurs radicaux choisis parmi thiényle et phényle lui-même éventuellement substitué, ces radicaux phényle étant eux-même éventuellement substitués par un ou plusieurs radicaux choisis parmi les atomes d'halogène et les radicaux -NH2, -NHAlk et -N(Alk)2,
n représente un entier de 0 à 2,
R3 représente hydrogène, halogène, alkyle, cyano, NO2, NR4R5, trifluorométhyle, aryle,
R2 représente un radical R4, OR4, SR4 ou NR4R5 dans lesquels R4 représente un atome d'hydrogène ou un radical alkyle, cycloalkyle ou aryle,
NR4R5 étant tel que soit R4 et R5, identiques ou différents, sont choisis parmi les valeurs de R4 soit R4 et R5 forment ensemble avec l'atome d'azote auxquels ils sont liés un radical cyclique renfermant 4 à 6 chaînons renfermant un ou plusieurs hétéroatomes identiques ou différents choisis parmi N, 0 et S,
tous les radicaux alkyle, alcoxy, cycloalkyle, aryle et hétérocyclique définis ci-dessus étant éventuellement substitués par un ou plusieurs radicaux choisis parmi les atomes d'halogène, les radicaux hydroxyle, cyano, trifluorométhyle, trifluorométhoxy, alcoxy, aryle, hétérocyclique, les radicaux à fonction acide et isostère d'acide et les radicaux -NHR4, -NalkR4, -COR4, -COOR4, CONalkR4 et-CONHR4 dans lesquels R4 a la signification indiquée ci-dessus et alk représente un radical alkyle, tous les radicaux aryle et hétérocyclique définis ci-dessus étant de plus éventuellement substitués par un ou plusieurs radicaux alkyle, hydroxyalkyle et phénylalkyle, tous les radicaux aryle définis ci-dessus étant de plus éventuellement substitués par un radical dioxol,
tous les radicaux alkyle et alcoxy définis ci-dessus étant linéaires ou ramifiés et renfermant au plus 6 atomes de carbone,
tous les radicaux cycloalkyle définis ci-dessus renfermant au plus 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

La présente invention a ainsi pour objet les produits de formule (I) telle que définie ci-dessus dans laquelle :
Y représente N, O, S, CHR3 ou =CR3
la ligne en pointillé sur le cycle indiquant que la liaison correspondante est simple ou double,
R et R1, identiques ou différents, représentent hydrogène, halogène, hydroxyle, alkyle, alcoxy, cyano, NO2, NR4R5, trifluorométhyle, trifluorométhoxy, aryle, hétéroaryle,

   -S (O) n-NR4R5 ,
n représente un entier de 0 à 2,
R3 représente hydrogène, halogène, alkyle, cyano, NO2, NR4R5, trifluorométhyle, aryle,
R2 représente un radical R4, OR4, SR4 ou NR4R5 dans lesquels R4 représente un atome d'hydrogène ou un radical alkyle, cycloalkyle ou aryle,
NR4R5 étant tel que soit R4 et R5, identiques ou différents, sont choisis parmi les valeurs de R4 soit R4 et R5 forment ensemble avec l'atome d'azote auxquels ils sont liés un radical cyclique renfermant 4 à 6 chaînons renfermant un ou plusieurs hétéroatomes identiques ou différents choisis parmi N, 0 et S,
tous les radicaux alkyle, alcoxy, cycloalkyle, aryle et hétérocyclique définis ci-dessus étant éventuellement substitués par un ou plusieurs radicaux choisis parmi les atomes d'halogène, les radicaux hydroxyle, cyano, trifluorométhyle, trifluorométhoxy, alcoxy, aryle, hétérocyclique, les radicaux à fonction acide et isostère d'acide et les radicaux -NHR4, -NalkR4, -COR4 , -COOR4, - CONalkR4 et -CONHR4 dans lesquels R4 a la signification indiquée ci-dessus et alk représente un radical alkyle, tous les radicaux aryle et hétérocyclique définis ci-dessus étant de plus éventuellement substitués par un ou plusieurs radicaux alkyle, hydroxyalkyle et phénylalkyle, tous les radicaux aryle définis ci-dessus étant de plus éventuellement substitués par un radical dioxol,
tous les radicaux alkyle et alcoxy définis ci-dessus étant linéaires ou ramifiés et renfermant au plus 6 atomes de carbone,
tous les radicaux cycloalkyle définis ci-dessus renfermant au plus 6 atomes de carbone,
lesdits produits de formule (I) étant sous toutes les formes isomères possibles racémiques, énantiomères et diastéréo+isomères, ainsi que les sels d'addition avec les acides minéraux et organiques ou avec les bases minérales et organiques pharmaceutiquement acceptables desdits produits de formule (I).

Dans les produits de formule (I) et dans ce qui suit :
- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés,
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés,
- le terme radical acyle ou r-CO- désigne un radical linéaire ou ramifié renfermant au plus 12 atomes de carbone dans lequel le radical r représente un atome d'hydrogène, un radical alkyle, cycloalkyle, cycloalkényle, cycloalkyle, hétérocycloalkyle, aryle ou hétéroaryle, ces radicaux ayant les valeurs éventuellement substituées indiquées ci-dessus ou ci-après : ainsi le radical acyle représente notamment CO-alkyle, CO-aryle ou CO-héréroaryle. On cite par exemple les radicaux formyle, acétyle, propionyle, butyryle ou benzoyle, ou encore valéryle, hexanoyle, acryloyle, crotonoyle, carbamoyle, pyrrolidinylcarboxy ou encore furylcarboxy
- le terme atome d'halogène désigne de préférence l'atome de chlore, mais peut aussi représenter un atome de fluor, de brome ou d'iode,
- le terme radical cycloalkyle désigne les radicaux cyclopropyle, cyclobutyle et tout particulièrement les radicaux cyclopentyle et cyclohexyle,
- le terme radical aryle désigne les radicaux insaturés, monocycliques ou constitués de cycles condensés, carbocycliques. Comme exemples de tel radical aryle, on peut citer les radicaux phényle ou naphtyle.
- le terme radical hétérocyclique désigne un radical saturé ou insaturé constitué au plus de 6 chaînons tel que l'un ou plusieurs des chaînons représente un atome d'oxygène, de soufre ou d'azote : un tel radical hétérocyclique désigne ainsi un radical carbocyclique interrompu par un ou plusieurs hétéroatomes choisis parmi les atomes d'oxygène, d'azote ou de soufre étant entendu que les radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents. On peut citer notamment le radical dioxolane, dioxane, dithiolane, thiooxolane, thiooxane, pipérazinyle, pipérazinyle substitué par un radical alkyle, linéaire ou ramifié, renfermant au plus 4 atomes de carbone, thiényle tel que 2-thiényle et 3-thiényle, furyle tel que 2-furyle, pyridyle tel que 2-pyridyle, 3-pyridyle et 4-pyridyle pyrimidyle, pyrrolyle, thiazolyle, isothiazolyle, diazolyle, triazolyle, tétrazolyle, thiadiazolyle, thiatriazolyle, oxazolyle, oxadiazolyle, 3- ou 4-isoxazolyle ; on peut citer également des groupes hétérocycliques condensés contenant au moins un hétéroatome choisi parmi le soufre, l'azote et l'oxygène, par exemple benzothiényle tel que 3-benzothiényle, benzofuryle, benzopyrrolyle, benzimidazolyle, benzoxazolyle, thionaphtyle, indolyle ou purinyle. On peut citer tout particulièrement les radicaux thiényle tel que 2-thiényle et 3-thiényle, furyle tel que 2-furyle, tétrahydrofuryle, thiényle, tétrahydrothienyle, pyrrolyle, pyrrolinyle et pyrrolidinyle.
- le terme fonction acide ou isostère d'acide désigne le radical carboxy libre, salifié ou estérifié, le radical tétrazolyle libre ou salifié, ou les radicaux : -SO3H, -PO(OH)2, NH SO2-CF3, -NH-SO2-NH-V, NH-SO2-NH-CO-V, NH-CO-V, -NH-CO-NH-V, -NH-CO-NH-SO2-V, -SO2-NH-, -SO2-NH-CO-V, -SO2-NH-CO-NH-V, -CO-NH-V, -CO-NH-OH, -CO-NH-SO2-V
dans lesquels V représente un radical alkyle ou alkényle, linéaire ou ramifié, renfermant au plus 6 atomes de carbone ou un radical phényle, ces radicaux alkyle, alkényle et phényle que représente V étant éventuellement substitués par les substituants indiqués ci-dessus pour les radicaux alkyle et phényle des produits de formule (I).

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, béta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant même formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus. La présente invention a particulièrement pour objet les produits de formule (I) telle que définie ci-dessus répondant à la formule (Ia): 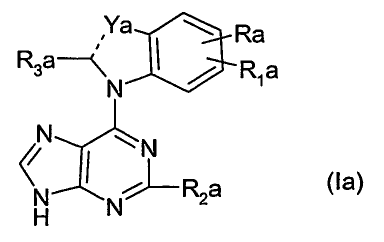 dans laquelle:
Ya représente N, O, S, CHR3a ou =CR3a
la ligne en pointillé sur le cycle indiquant que la liaison correspondante est simple ou double,
Ra et R1a, identiques ou différents, représentent hydrogène, halogène, hydroxyle, alkyle, alcoxy, cyano, NO2, NR4aR5a, trifluorométhyle, trifluorométhoxy, phényle, hétéroaryle,

   -S (O) n-NR4aR5a,
n représente un entier de 0 à 2,
R3a représente hydrogène, halogène, alkyle, cyano, NO2, amino, alkylamino, dialkylamino, trifluorométhyle et phényle,
R2a représente un radical R4a, OR4a, SR4a ou NR4aR5a dans lesquels R4a représente un atome d'hydrogène ou un radical alkyle, cycloalkyle ou phényle,
NR4aR5a étant tel que soit R4a et R5a, identiques ou différents, sont choisis parmi les valeurs de R4a soit R4a et R5a forment ensemble avec l'atome d'azote auxquels ils sont liés un radical pipéridyle, morpholinyle, pyrrolidinyle ou pipérazinyle éventuellement substitué, tous les radicaux alkyle, alcoxy, cycloalkyle, phényle, phénylalkyle et hétérocyclique définis ci-dessus étant éventuellement substitués par un ou plusieurs radicaux choisis parmi les atomes d'halogène, les radicaux hydroxyle, cyano, trifluorométhyle, trifluorométhoxy, alcoxy, phényle, hétérocyclique tels que par exemple tétrahydropyranyle, pipéridyle éventuellement substitué sur N ou C par un radical carboxy libre, salifié ou estérifié par un radical alkyle, les radicaux SO3H, PO(OH)2, NH-SO2-CF3, NH-SO2-NH-V et NH-SO2-NH-CO-V dans lesquels V représente un radical phényle, alkyle ou alkényle, les radicaux alkényle étant linéaires ou ramifiés renfermant au plus 6 atomes de carbone, et les radicaux -NalkR4a, -NHR4a, -COR4a, -COOR4a , -CONalkR4a et-CONHR4a dans lesquels R4a a la signification indiquée ci-dessus et alk représente un radical alkyle,
tous les radicaux phényle et hétérocyclique définis ci-dessus étant de plus éventuellement substitués par un ou plusieurs radicaux alkyle, hydroxyalkyle et phénylalkyle,
tous les radicaux phényle définis ci-dessus étant de plus éventuellement substitués par un radical dioxol,
tous les radicaux alkyle et alcoxy définis ci-dessus étant linéaires ou ramifiés et renfermant au plus 6 atomes de carbone,
tous les radicaux cycloalkyle définis ci-dessus renfermant 5 ou 6 atomes de carbone,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Ia).

On peut noter que lorsque R2a représente un radical NR4aR5a, R2a représente notamment le radical NH-Rya dans lequel Rya représente le radical : 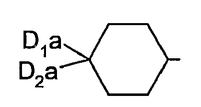 avec D1a et D2a soit, identiques ou différents, sont choisis parmi l'atome d'hydrogène, le radical hydroxyle, les radicaux alkyle, alcoxy linéaires ou ramifiés renfermant au plus 6 atomes de carbone et les radicaux NHR5a, soit forment ensemble le radical =O ou =N-OR4a,
R4a représente l'atome d'hydrogène, un radical alkyle, cycloalkyle ou phényle,
R5a représente l'atome d'hydrogène, un radical alkyle, cycloalkyle ou le radical -COOtBu (Boc).

La présente invention a plus particulièrement pour objet les produits de formule (I) telle que définie ci-dessus répondant à la formule (Ib): 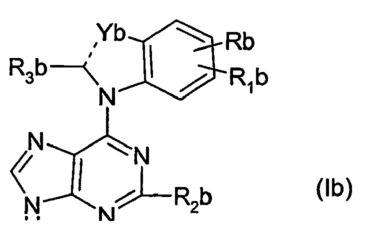 dans laquelle :
Yb représente N, CHR3b ou =CR3b
la ligne en pointillé sur le cycle indiquant que la liaison correspondante est simple ou double,
Rb et R1b, identiques ou différents, représentent hydrogène, halogène, hydroxyle, alkyle, alcoxy, cyano, NO2, trifluorométhyle, trifluorométhoxy, phényle,

   -S (O) n-NR4bR5b,
n représente un entier de 0 à 2,
R3b représente hydrogène, halogène, alkyle, cyano, NO2, amino, alkylamino, dialkylamino, trifluorométhyle et phényle,
R2b représente un radical R4b ou NR4bR5b dans lesquels R4b représente un atome d'hydrogène ou un radical alkyle, cycloalkyle ou phényle,
NR4bR5b étant tel que soit R4b et R5b, identiques ou différents, sont choisis parmi les valeurs de R4b soit R4b et R5b forment ensemble avec l'atome d'azote auxquels ils sont liés un radical pipéridyle, morpholinyle, pyrrolidinyle éventuellement substitués,
tous les radicaux alkyle, alcoxy, cycloalkyle, phényle, phénylalkyle, hétérocyclique comme pipéridyle, morpholinyle et pyrrolidinyle définis ci-dessus étant éventuellement substitués par un ou deux radicaux choisis parmi les atomes d'halogène, les radicaux hydroxyle, cyano, trifluorométhyle, trifluorométhoxy, alcoxy, phényle, tétrahydropyrannyle, pipéridyle, eux-mêmes éventuellement substitués sur N ou C par un radical carboxy libre, salifié ou estérifié par un radical alkyle), et les radicaux -NalkR4a, -NHR4a et -COOR4a dans lesquels R4a a la signification indiquée ci-dessus et alk représente un radical alkyle,
tous les radicaux phényle et hétérocyclique définis ci-dessus étant de plus éventuellement substitués par un ou plusieurs radicaux alkyle, hydroxyalkyle et phénylalkyle, tous les radicaux alkyle et alcoxy définis ci-dessus étant linéaires ou ramifiés et renfermant au plus 4 atomes de carbone,
tous les radicaux cycloalkyle définis ci-dessus renfermant 5 ou 6 atomes de carbone,
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Ib).

On peut noter que lorsque R2b représente un radical NR4bR5b, R2b représente notamment le radical NH-Ryb dans lequel Ryb représente le radical : 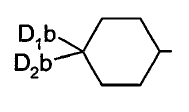 avec D1b et D2b soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux NHR5b, soit forment ensemble le radical =O ou =N-OR4b,
R4b représente l'atome d'hydrogène, un radical alkyle renfermant au plus 4 atomes de carbone, phényle, -CH2-phényle ou le radical cycloalkyle renfermant au plus 6 atomes de carbone éventuellement substitué par le radical -NHR3b,
R5b représente l'atome d'hydrogène, un radical alkyle, cycloalkyle renfermant au plus 6 atomes de carbone ou le radical -COOtBu (Boc).

La présente invention a encore plus particulièrement pour objet les produits de formule (I) telle que définie ci-dessus répondant à la formule (Ic) : 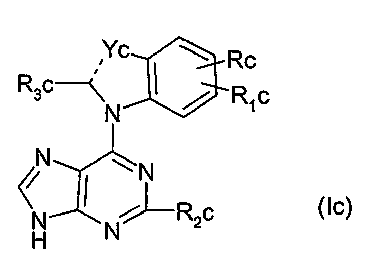 dans laquelle:
Yc représente N, CH2 ou CH=,
la ligne en pointillé sur le cycle indiquant que la liaison correspondante est simple ou double,
Rc et R1c, identiques ou différents, représentent hydrogène, halogène, hydroxyle, alkyle, alcoxy, phénylalcoxy, phénylalkyle, cyano, NO2, trifluorométhyle, trifluorométhoxy, phényle, -S(O)n-NH2 éventuellement substitué sur l'atome d'azote par un ou deux radicaux alkyle et n représente un entier de 0 à 2,
R3c représente hydrogène, halogène, alkyle, cyano, NO2, trifluorométhyle et phényle,
R2c représente un radical NR4cR5c dans lesquels soit R4c et R5c, identiques ou différents, sont tels que R4c représente un atome d'hydrogène ou un radical alkyle, cycloalkyle, phényle ou phénylalkyle,
les radicaux alkyle, cycloalkyle, phényle et phénylalkyle étant éventuellement substitués par un ou plusieurs radicaux choisis parmi hydroxyle, amino, carboxy libre, salifié ou estérifié par un radical alkyle, tétrahydropyrannyle, pipéridyle, éventuellement substitué sur N ou C par un radical carboxy libre, salifié ou estérifié par un radical alkyle,
et R5c représente un atome d'hydrogène ou un radical alkyle,
soit R4c et R5c forment ensemble avec l'atome d'azote auxquels ils sont liés un radical pipéridyle, morpholinyle ou pyrrolidinyle, ces radicaux éventuellement substitués par un radical alkyle, hydroxyalkyle, amino, monoalkylamino ou dialkylamino, tous les radicaux alkyle et alcoxy définis ci-dessus étant linéaires ou ramifiés renfermant au plus 4 atomes de carbone
lesdits produits de formule (Ic) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Ic).

On peut noter que lorsque Ryc représente le radical : 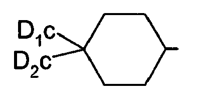 avec D1c et D2c soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH2, -NH-COOtBu ou -NHalkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone.
Ryd représente notamment le radical : 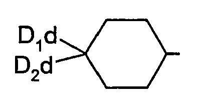 avec D1d et D2d, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH2, -NH-COOtBu ou -NHalkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone. Rye représente notamment le radical : 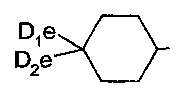 avec D1e et D2e représentent l'un l'atome d'hydrogène et l'autre le radical -NH2 éventuellement substitué par un radical -COOtBu ou -alkyle dans lequel le radical alkyle linéaire ou ramifié renferme au plus 4 atomes de carbone.

La présente invention a tout particulièrement pour objet les produits de formule (I) telle que définie ci-dessus répondant à la formule (Id): 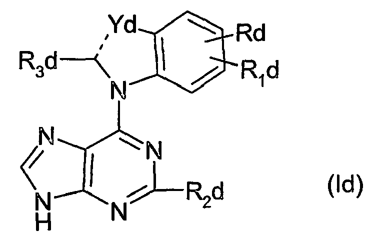 dans laquelle:
Yd représente N, CH2 ou CH=,
la ligne en pointillé sur le cycle indiquant que la liaison correspondante est simple ou double,
Rd et R1d, identiques ou différents, représentent hydrogène, halogène, alkyle, alcoxy, phénylalcoxy, NO2, dialkylaminosulfonyle,-NH2, trifluorométhyle, -CO-CH3, -NH-CO-alkyle ou -NH-CO-NH-phényle dans lesquels le radical alkyle est éventuellement substitué par un radical thiényle ou phényle et le radical phényle est éventuellement substitué par un ou plusieurs radicaux choisis parmi les atomes d'halogène et les radicaux -NH2, -NHAlk et -N(Alk)2,
R3d représente hydrogène ou alkyle,
R2d représente un radical NR4dR5d dans lesquels soit R4d et R5d, identiques ou différents, sont tels que R4d représente un atome d'hydrogène, un radical alkyle linéaire ou ramifié renfermant 1 à 4 atomes de carbone et éventuellement substitué par un ou deux hydroxyle, un radical cycloalkyle éventuellement substitué par un radical amino ou hydroxyle, phényle ou phénylalkyle (1à4C) avec phényle éventuellement substitué par un radical carboxy libre, salifié ou estérifié par un radical alkyle, tétrahydropyranalkyle (ex 28), pipéridylalkyle (ex 31, 36) éventuellement substitué sur N ou C par un radical carboxy
et R5d représente un atome d'hydrogène ou un radical alkyle,
soit R4d et R5d forment ensemble avec l'atome d'azote auxquels ils sont liés un radical pipéridyle éventuellement substitué par un radical amino, morpholinyle, pyrrolidinyle (ex 34) éventuellement substitué par un radical hydroxyalkyle,
tous les radicaux alkyle et alcoxy définis ci-dessus étant linéaires ou ramifiés renfermant au plus 4 atomes de carbone
lesdits produits de formule (Id) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Id).

La présente invention a ainsi tout particulièrement pour objet les produits de formule (I) telle que définie ci-dessus répondant à la formule (Id) dans laquelle:
Yd représente N, CH2 ou CH=,
la ligne en pointillé sur le cycle indiquant que la liaison correspondante est simple ou double,
Rd et R1d, identiques ou différents, représentent hydrogène, halogène, alkyle, alcoxy, phénylalcoxy, NO2, dialkylaminosulfonyle
R3d représente hydrogène ou alkyle,
R2d représente un radical NR4dR5d dans lesquels soit R4d et R5d, identiques ou différents, sont tels que R4d représente un atome d'hydrogène, un radical alkyle linéaire ou ramifié renfermant 1 à 4 atomes de carbone et éventuellement substitué par un ou deux hydroxyle, un radical cycloalkyle éventuellement substitué par un radical amino ou hydroxyle, phényle ou phénylalkyle (1 à 4 C) avec phényle éventuellement substitué par un radical carboxy libre, salifié ou estérifié par un radical alkyle, tétrahydropyranalkyle, pipéridylalkyle éventuellement substitué sur N ou C par un radical carboxy, et R5d représente un atome d'hydrogène ou un radical alkyle, soit R4d et R5d forment ensemble avec l'atome d'azote auxquels ils sont liés un radical pipéridyle éventuellement substitué par un radical amino, morpholinyle, pyrrolidinyle éventuellement substitué par un radical hydroxyalkyle, tous les radicaux alkyle et alcoxy définis ci-dessus étant linéaires ou ramifiés renfermant au plus 4 atomes de carbone lesdits produits de formule (Id) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Id).

La présente invention a tout particulièrement pour objet les produits de formule (I) telle que définie ci-dessus dont les noms suivent :
- le Dichlorhydrate de trans-N-[6-(5,6-dichloro-2,3-dihydro-1H-indol-1-yl)-9H-purin-2-yl]-1,4-cyclohexanediamine
- le Dichlorhydrate de trans-N-[6-(1H-benzimidazol-1-yl)-9H-purin-2-yl]-1,4-cyclohexanediamine
- le Trans-N-[6-(5,6-diméthyl-1H-benzimidazol-1-yl)-9H-purin-2-yl]-1,4-cyclohexanediamine
- le Chlorhydrate de trans-N-[6-(5,6-dichloro-1H-benzimidazol-1-yl)-9H-purin-2-yl]-1,4-cyclohexanediamine
- le Dichlorhydrate de trans-N-[6-(5-méthoxy-1H-benzimidazol-1-yl)-9H-purin-2-yl]-1,4-cyclohexanediamine
- le Dichlorhydrate de trans-N-[6-(1H-indol-1-yl)-9H-purin-2-y1]-1,4-cyclohexanediamine
- le Trans-N-[6-[6-(phénylméthoxy)-1H-benzimidazol-1-yl]-9H-purin-2-yl]-1,4-cyclohexanediamine
- le Trans-N-[6-[5-(phénylméthoxy)-1H-benzimidazol-1-yl]-9H-purin-2-yl]-1,4-cyclohexanediamine
- le Trans-4-[[6-(1H-benzimidazol-1-yl)-9H-purin-2-yl]amino]-cyclohexanol
- le Dichlorhydrate de trans-N-[6-(2,3-dihydro-5-nitro-1H-indol-1-yl)-9H-purin-2-yl]-1,4-cyclohexanediamine
- le Trans-N-[6-[2,3-dihydro-6-(trifluorométhyl)-1H-indol-1-yl)-9H-purin-2-yl]-1,4-cyclohexanediamine (Ex 40)
- le Trans-N-[1-[2-[(4-aminocyclohexyl)amino]-9H-purin-6-yl]-2,3-dihydro-1H-indol-5-yl]-2-thiophèneacétamide (Ex 41)
- le Trans-N-[6-(6-nitro-2,3-dihydro-1H-indol-1-yl)-9H-purin-2-yl]-1,4-cyclohexanediamine (Ex 44)

La présente invention a également pour objet un procédé de préparation des produits de formule (I), telle que définie ci-dessus caractérisé en ce que l'on soumet le composé de formule (II) : 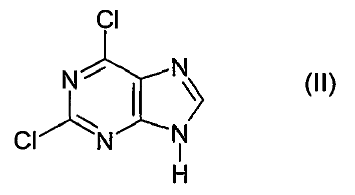 que l'on soumet à l'action d'un composé de formule (III) : 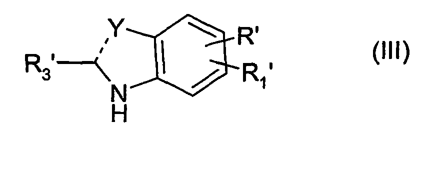 dans laquelle R', R1'et R3' ont les significations indiquées respectivement à la revendication 1 pour R, R1 et R3, dans lesquelles les éventuelles fonctions réactives sont éventuellement protégées par des groupements protecteurs, et Y a la signification indiquée à la revendication 1,
pour obtenir un produit de formule (IV) : 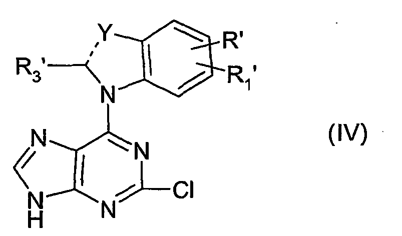 dans laquelle R', R1', R3' et Y ont les significations indiquées ci-dessus,
produit de formule (IV) que l'on soumet à une réaction avec un composé de formule (V) :

R2'-H (V)

dans laquelle R2' a la signification indiquée à la revendication 1 pour R2 dans laquelle les éventuelles fonctions réactives sont éventuellement protégées par des groupements protecteurs,
pour obtenir un produit de formule (I'): 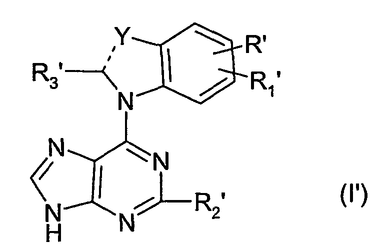 dans laquelle R', R1', R2', R3' et Y' ont les significations indiquées ci-dessus,
les produits de formule (I') ayant la signification indiquée à la revendication 1 pour les produits de formule (I) dans laquelle les éventuelles fonctions réactives sont éventuellement protégées par des groupements protecteurs,
produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

Les produits de formule (I') ayant la signification indiquée ci-dessus pour les produits de formule (I) dans laquelle les éventuelles fonctions réactives sont éventuellement protégées par des groupements protecteurs, produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Dans des conditions préférentielles de mise en oeuvre de l'invention, le procédé décrit ci-dessus peut être réalisé de la façon suivante :
Le produit de formule (II) est donc la dichloro 2,6 purine qui est un produit commercialisé.

Le produit de formule (II) est soumis à l'action du produit de formule (III) telle que définie ci-dessus notamment dans un alcool tel que le butanol à une température d'environ 80°C ou à une température d'environ 75°C pendant environ 2 ou 3 heures ou dans le DMF pour donner un produit de formule (IV) telle que définie ci-dessus.

Le produit ainsi obtenu de formule (IV) telle que définie ci-dessus est soumis à l'action d'un composé de formule (V) dans les conditions définies dans la partie expérimentale et notamment comme indiqué ci-après.

Le composé de formule (V) peut notamment représenter un composé de formule (XIV), (XV) ou (XVI) telles que définies ci-après : 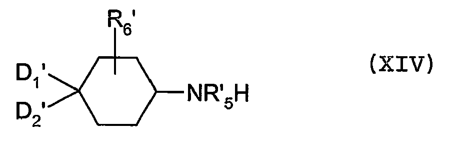 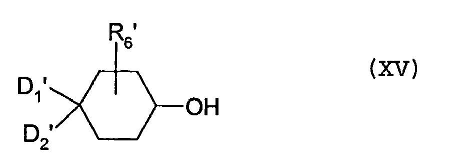 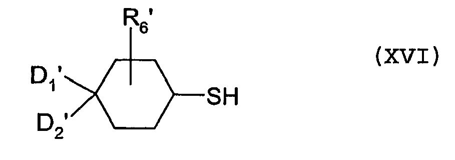 composés de formule (XIV), (XV) ou (XVI) dans lesquelles D1', D2' et R5' ont les significations indiquées à la revendication 1 respectivement pour D1, D2 et R5 dans lesquelles les éventuelles fonctions réactives sont éventuellement protégées par des groupements protecteurs et R6' représentant un ou plusieurs substituants que peut porter le radical cycloalkyle, dans lesquelles les éventuelles fonctions réactives sont éventuellement protégées par des groupements protecteurs.
Le produit de formule (V) soit R2-H peut notamment représenter le produit (XIV) : 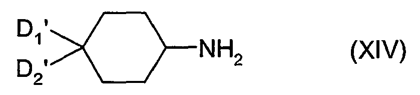 dans laquelle D1' et D2' ont les significations indiquées ci-dessus respectivement pour D1 et D2 dans lesquelles les éventuelles fonctions réactives sont éventuellement protégées par des groupements protecteurs.

Ainsi parmi les produits de formule (I') on trouve notamment les produits de formule (I") : 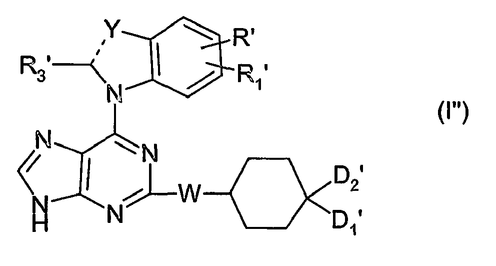 dans laquelle R', R1', R3', D1' et D2' et Y' ont les significations indiquées ci-dessus, dans lesquelles les éventuelles fonctions réactives sont éventuellement protégées par des groupements protecteurs, et W représente NH, S ou O, que l'on peut préparer notamment en faisant réagir un produit de formule (IV) tel que défini ci-dessus avec un produit de formule (XIV), (XV) ou (XVI) tels que définis ci-dessus.

La réaction du produit de formule (IV) avec un composé de formule (XIV), (XV) ou (XVI) telles que définies ci-dessus pour donner un produit de formule (I') peut notamment être réalisée selon une réaction de condensation qui le cas échéant peut être réalisée à une température d'environ 140°C sans solvant : une telle réaction de condensation peut être suivie d'une réaction de salification en présence d'acide chlorhydrique par exemple ou encore d'acide tartrique, citrique ou méthane sulfonique, dans un alcool tel que par exemple l'éthanol ou le méthanol pour donner des produits de formule (I') telle que définie ci-dessus.

La fonction amine des composés de formule (I') telle que définie ci-dessus, peut être protégée par un groupe tel que Boc ou CH2-phényle et peut alors être libérée dans les conditions usuelles connues de l'homme du métier.

La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse.

Les réactions de réduction ou oxydation du produit de formule (I') en produit de formule (I) peuvent être réalisées selon les méthodes usuelles connues de l'homme du métier.

Selon les valeurs de Y', R', R1', R2', R3', D1' et D2', les produits de formules (I') constituent ou non des produits de formule (I) et peuvent donner des produits de formule (I) ou être transformés en d'autres produits de formule (I) en étant soumis à une ou plusieurs des réactions a) à k) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, benzyloxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal ou le diéthylthiocétal ou l'éthylènedithiocétal,
- les fonctions acides des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acides peuvent être protégées par exemple sous forme d'esters formés avec les esters facilement clivables tels que les esters benzyliques ou ter butyliques ou des esters connus dans la chimie des peptides.

Les réactions auxquelles les produits de formule (I') telle que définie ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'oxone, le périodate de sodium dans un solvant tel que par exemple le chlorure de méthylène ou le dioxanne à la température ambiante.

L'obtention de la fonction sulfoxyde peut être favorisée par un mélange équimolaire du produit renfermant un groupement alkylthio et du réactif tel que notamment un peracide.

L'obtention de la fonction sulfone peut être favorisée par un mélange du produit renfermant un groupement alkylthio avec un excès du réactif tel que notamment un peracide.
d) La réaction de transformation d'une fonction cétone en oxime peut être réalisée dans les conditions usuelles connues de l'homme de métier, telle que notamment une action en présence d'une hydroxylamine éventuellement O-substituée dans un alcool tel que par exemple l'éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.

Les éventuelles fonctions carboxy libre des produits décrits ci-dessus peuvent être, si désiré, réduites en fonction alcool notamment par des dérivés de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
J. Organometallic Chemistry 33, 337 (1971) KOZIMA S.& coll.

On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.

Il est entendu que les réactions décrites ci-dessus peuvent être effectuées comme indiqué ou encore, le cas échéant, selon d'autres méthodes usuelles connues de l'homme du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou paratoluène sulfonique, formique ou trifluoroacétique ou encore par une hydrogénation catalytique.

Le groupement phtalimido peut être éliminé par l'hydrazine.

On trouvera une liste de différents groupements protecteurs utilisables par exemple dans le brevet FR 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier.
k) Les éventuelles formes optiquement actives des produits décrits ci-dessus peuvent être préparées par dédoublement des racémiques selon les méthodes usuelles connues de l'homme du métier.

Le produit de départ de formule (II) soit la dichloro 2,6 purine est connu et commercialisé.

Parmi les produits de départ de formules (III) et (V), certains sont connus et peuvent être obtenus commercialement ou peuvent être préparés selon les méthodes usuelles connues de l'homme du métier.

Parmi les produits de départ commerciaux de formules (III), (V), (XIV), (XV) et (XVI), on peut citer par exemple, les produits suivants :
Comme produits commerciaux de formule (XIV), on peut citer le trans-1,4-diaminocyclohexane, le trans-4-aminocyclohexanol ou encore la benzylamine, la paraméthoxybenzylamine ou la parafluorobenzylamine.

On peut encore notamment préparer certains produits de départ à partir de produits commerciaux par exemple en les soumettant à une ou plusieurs des réactions décrites ci-dessus en a) à k), réalisées dans les conditions également décrites ci-dessus.

On peut citer encore à titre d'exemple :
des exemples de produits de formule (II) telle que définie ci-dessus, sont donnés ci-après dans la préparation des exemples de la présente invention.

La partie expérimentale ci-après donne des exemples de tels produits de départ.

La présente invention a enfin pour objet à titre de produits industriels nouveaux, les composés de formules (IV) tel que notamment le produit 6-(1H-benzimidazol-1-yl)-2-chloro-9H-purine.

Des illustrations de telles réactions définies ci-dessus sont données dans la préparation des exemples décrits ci-après.

Les produits de formule (I) tels que définis ci-dessus ainsi que leurs sels d'addition avec les acides présentent d'intéressantes propriétés pharmacologiques.

Les produits de la présente invention tels que définis ci-dessus, possèdent des propriétés inhibitrices de kinases d'une grande sélectivité.

Les cdk jouent un rôle central dans l'initiation, le développement et l'achèvement des évènements du cycle cellulaire et ainsi, les molécules inhibitrices de cdk sont susceptibles de limiter des proliférations cellulaires non désirées telles que celles observées dans les cancers, psoriasis, croissance de champignons, de parasites (animaux, protistes) : de telles molécules inhibitrices de cdk sont ainsi également susceptibles d'intervenir dans la régulation de maladies neurodégénératives telles que la maladie d'Alzheimer.

Des kinases particulièrement sensibles aux effets inhibiteurs des dérivés de la présente invention sont notamment les cdk1, cdk2, cdk4, cdk5 et cdk7.

Les produits de la présente invention sont donc doués de propriétés antimitotiques.

Les produits de la présente invention possèdent en plus de leurs propriétés inhibitrices spécifiques de kinases, des effets cellulaires intéressants tels que des propriétés antiprofilératives et notamment des effets sur l'apoptose.

On sait par des travaux décrits dans la littérature tel que dans WO 97/20842, que des rapports existent entre le cycle cellulaire et l'apoptose. Parmi les voies conduisant à l'apoptose, certaines sont dépendantes de kinases.

Les produits de la présente invention sont notamment utiles pour la thérapie de tumeurs.

Les produits de l'invention peuvent également ainsi augmenter les effets thérapeutiques d'agents anti-tumoraux couramment utilisés.

Les produits de formule (I) de la présente invention possèdent donc tout particulièrement des propriétés antimitotiques et anti-neurodégénératives.

Les produits de formule (I) telle que définie ci-dessus peuvent donc être utilisés comme inhibiteurs de tyrosines kinases : de telles tyrosines kinases peuvent appartenir à diverses familles telles que par exemple la famille Src dans laquelle se trouvent notamment Src, Fyn, Lck, Yes, Fgr, Hck et Yrk ou encore par exemple les familles Csk, Btk, Abl, Fak, Jak, Syk, Fps, Zap 70, EGF, PDGF et CSF. Une telle liste de protéines tyrosines kinases n'est pas exhaustive.

Parmi ces protéines tyrosines kinases, on peut noter que l'on distingue des protéines tyrosines kinases associées à des récepteurs telles que par exemple EGF, PDGF ou CSF et des protéines tyrosines kinases cytoplasmiques parmi lesquelles notamment Src, Fyn, Lck, Yes, Fgr, Hck et Yrk ou encore Csk, Btk, Abl, Fak, Jak, Syk, Fps et Zap 70.

Les produits de formule (I) telle que définie ci-dessus peuvent en outre être utilisés pour inhiber le domaine catalytique (tyrosine kinase) de la protéine Src, la méthode consistant en l'administration au patient dont le traitement requiert l'inhibition du domaine catalytique (tyrosine kinase) de la protéine Src, une quantité inhibitrice d'un ou plusieurs produits de formule (I) telle que définie ci-dessus.

Les produits de formule (I) telle que définie ci-dessus sont tout particulièrement des inhibiteurs du domaine catalytique Src : de tels inhibiteurs sont ainsi notamment capables d'inhiber l'adhésion des ostéoclastes sur la surface de l'os et ainsi la résorption osseuse par les ostéoclastes.

Les maladies de l'os dont le traitement ou la prévention nécessite l'emploi des produits de formule (I) telle que définie ci-dessus, sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, la maladie de Paget, l'ostéopénie induite par l'immobilisation. En outre les produits de formule (I) telle que définie ci-dessus peuvent être utilisés pour soulager, empêcher ou traiter les désordres de l'os qui sont causés par les traitements, par les glucocorticoïdes, les thérapies liées à la prise de stéroïdes ou de corticostéroïdes ou par les déficiences d'hormones sexuelles mâles ou femelles.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

Les produits de formule (I) telle que définie ci-dessus, par leur affinité avec le domaine catalytique (tyrosine kinase) de Src, peuvent également être utilisés dans d'autres applications thérapeutiques. Par exemple on sait que les plaquettes et les neurones sont des tissus qui expriment la protéine Src également. En outre plusieurs protéines de cette famille étant majoritairement exprimées dans le système hématopoïétique, de nombreuses applications dans le traitement de l'immunité, de l'infection, de l'allergie et des maladies auto-immunes sont envisageables.
Enfin, les produits de formule (I) telle que définie ci-dessus peuvent également être utilisés pour inhiber le domaine catalytique (tyrosine kinase) de protéines autres que Src, la méthode consistant en l'administration au patient dont le traitement requiert l'inhibition du domaine catalytique (tyrosine kinase), une quantité inhibitrice d'un ou plusieurs produits de formule (I) telle que définie ci-dessus.

De telles protéines contenant le domaine catalytique (tyrosine kinase) autres que Src peuvent donc être choisies notamment parmi Fyn, Lck, Yes, Fgr, Hck, Yrk, Csk, Btk, Abl, Fak, Jak, Syk, Fps, Zap 70, EGF, PDGF et CSF.Une telle liste de protéines tyrosines kinases n'est pas exhaustive

les produits de formule (I) telle que définie ci-dessus peuvent également être utilisés pour inhiber le domaine catalytique Sérine /thréonine kinase notamment parmi les CDK

Les produits de formule (I) telle que définie ci-dessus peuvent ainsi être utilisés dans le traitement de maladies telles que les maladies prolifératives, le cancer, la resténose, l'inflammation; les allergies ou les maladies cardiovasculaires.

Les produits de la présente invention tels que définis ci-dessus, possèdent des propriétés inhibitrices de protéines kinases comme indiqué ci-dessus et notamment de CIV1 telle que définie ci-dessus.

Les CIV1 jouent un rôle central dans l'entrée dans le cycle cellulaire par l'activation des Cdk et ainsi, les molécules inhibitrices de CIV1 sont susceptibles de limiter des proliférations cellulaires non désirées telles que celles observées dans la croissance de champignons.

Les produits de formule (I) de la présente invention peuvent donc posséder des propriétés antimitotiques.

Ces propriétés justifient leur application en thérapeutique et l'invention a particulièrement pour objet à titre de médicaments, les produits de formule (I)telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a plus particulièrement pour objet à titre de médicaments, les produits tels que définis par la formule (Id) telle que définie ci-dessus.

L'invention a tout particulièrement pour objet, à titre de médicaments, les produits décrits ci-après dans les exemples et notamment les produits de formule (I) telle que définie ci-dessus, répondant aux formules suivantes :
- le Dichlorhydrate de trans-N-[6-(5,6-dichloro-2,3-dihydro-1H-indol-1-yl)-9H-purin-2-yl]-1,4-cyclohexanediamine
- le Dichlorhydrate de trans-N-[6-(1H-benzimidazol-1-yl)-9H-purin-2-yl]-1,4-cyclohexanediamine
- le Trans-N-[6-(5,6-diméthyl-1H-benzimidazol-1-yl)-9H-purin-2-yl]-1,4-cyclohexanediamine
- le Chlorhydrate de trans-N-[6-(5,6-dichloro-1H-benzimidazol-1-yl)-9H-purin-2-yl]-1,4-cyclohexanediamine
- le Dichlorhydrate de trans-N-[6-(5-méthoxy-1H-benzimidazol-1-yl)-9H-purin-2-yl]-1,4-cyclohexanediamine
- le Dichlorhydrate de trans-N-[6-(1H-indol-1-yl)-9H-purin-2-yl]-1,4-cyclohexanediamine
- le Trans-N-[6-[6-(phénylméthoxy)-1H-benzimidazol-1-yl]-9H-purin-2-yl]-1,4-cyclohexanediamine
- le Trans-N-[6-[5-(phénylméthoxy)-1H-benzimidazol-1-yl]-9H-purin-2-yl]-1,4-cyclohexanediamine
- le Trans-4-[[6-(1H-benzimidazol-1-yl)-9H-purin-2-yl]amino]- cyclohexanol
- le Dichlorhydrate de trans-N-[6-(2,3-dihydro-5-nitro-1H-indol-1-yl)-9H-purin-2-yl]- 1,4-cyclohexanediamine
- le Trans-N-[6-[2,3-dihydro-6-(trifluorométhyl)-1H-indol-1-yl)-9H-purin-2-yl]-1,4-cyclohexanediamine( Ex 40)
- le Trans-N-[1-[2-[(4-aminocyclohexyl)amino]-9H-purin-6-yl]-2,3-dihydro-1H-indol-5-yl]-2-thiophéneacétamide (Ex 41)
- le Trans-N-[6-(6-nitro-2,3-dihydro-1H-indol-1-yl)-9H-purin-2-yl]-1,4-cyclohexanediamine (Ex 44)

Les médicaments, objet de l'invention, trouvent, par exemple, comme antimitotiques, leur emploi dans la chimiothérapie des cancers ou encore dans le traitement de psoriasis, de parasitoses telles que celles dues à des protistes ou à des champignons ou encore dans le traitement de la maladie d'Alzheimer ou dans le traitement de l'apoptose neuronale.

Les médicaments, objet de l'invention, trouvent notamment leur emploi dans le traitement de maladies dues à des champignons telles que les candidoses, aspergilloses, histoplasmoses et coccidoidoses.

L'invention s'étend aux compositions pharmaceutiques contenant à titre de principe actif l'un au moins des médicaments tels que définis ci-dessus.

De telles compositions peuvent notamment être utiles pour traiter les infections fongiques topiques et systémiques.

Les compositions pharmaceutiques indiquées ci-dessus peuvent être administrées par voie buccale, rectale, par voie parentérale ou par voie locale en application topique sur la peau et les muqueuses ou par injection par voie intraveineuse ou intramusculaire. Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels, les préparations en aérosols, les ovules vaginales et les capsules gynécologiques. Ces compositions sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie sera variable selon le produit utilisé, le sujet traité et l'affection en cause, peut être, par exemple, de 0,05 à 5 g par jour chez l'adulte ou de préférence de 0,1 à 2 g par jour.

L'invention a donc particulièrement pour objet les compositions pharmaceutiques telles que définies ci-dessus caractérisées en ce qu'elles sont utilisées comme médicaments.

L'invention a ainsi notamment pour objet l'utilisation des produits de formule (I) telle que définie ci-dessus et/ou de leurs sels pharmaceutiquement acceptables, pour la préparation de médicaments destinés à la prévention ou au traitement de maladies fongiques telles que des mycoses dues à des champignons choisis notamment parmi les champignons définis ci-dessus.

L'invention a plus précisément pour objet l'utilisation des produits de formule (I) telle que définie ci-dessus et/ou de leurs sels pharmaceutiquement acceptables, pour la préparation de médicaments destinés à la prévention ou au traitement de maladies fongiques telles que notamment les candidoses, aspergilloses, histoplasmoses et coccidoidoses.

L'invention a particulièrement pour objet l'utilisation des produits de formule (I) tels que définis ci-dessus et/ou de leurs sels pharmaceutiquement acceptables pour la préparation de médicaments destinés à la prévention ou au traitement de maladies causées par Candida albicans et notamment destinés à la prévention ou au traitement de la candidose systémique.

L'invention a ainsi pour objet les produits de formule (I) telle que définie ci-dessus ayant des propriétés antifongiques comme inhibiteurs de protéines kinases CIV1 de Candida albicans.

L'invention a ainsi pour objet les compositions pharmaceutiques renfermant à titre de principe actif au moins un inhibiteur de protéines kinases CIV1 de Candida albicans tels que définis ci-dessus.

La présente invention a notamment pour objet l'utilisation des compositions telles que définies ci-dessus comme agents antifongiques.

La présente invention a ainsi pour objet les compositions pharmaceutiques telles que définies ci-dessus caractérisées en ce qu'elles sont utilisées comme médicaments antimitotiques, en particulier pour la chimiothérapie de cancers ou encore pour le traitement de psoriasis, de parasitoses telles que celles dues à des champignons ou à des protistes ou de la maladie d'Alzheimer.

La présente invention a ainsi pour objet les compositions pharmaceutiques telles que définies ci-dessus caractérisées en ce qu'elles sont utilisées comme médicaments antineurodégénératifs notamment anti-apoptose neuronale.

La présente invention a également pour objet l'utilisation des produits de formule (I) telle que définie ci-dessus, et/ou de leurs sels pharmaceutiquement acceptables, pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

La présente invention a également pour objet l'utilisation des produits de formule (I) telle que définie ci-dessus, et/ou de leurs sels pharmaceutiquement acceptables tels que définis ci-dessus pour la préparation de médicaments destinés à la prévention ou au traitement de maladies liées à un dérèglement de la sécrétion et/ou de l'activité de protéines tyrosines kinases et des Sérine /thréonine kinases

La présente invention a également pour objet l'utilisation des produits de formule (I) telle que définie ci-dessus, et/ou de leurs sels pharmaceutiquement acceptables pour la préparation de médicaments destinés au traitement ou à la prévention de l'immunité, de l'infection, de l'allergie, et des maladies auto-immunes.

La présente invention a également pour objet l'utilisation des produits de formule (I) telle que définie ci-dessus, et/ou de leurs sels pharmaceutiquement acceptables pour la préparation de médicaments destinés au traitement ou à la prévention de maladies telles que les maladies prolifératives, le cancer, la resténose, l'inflammation, les allergies ou les maladies cardiovasculaires.

La présente invention concerne aussi un procédé de criblage de produits antifongiques selon la présente invention, caractérisé en ce qu'il comprend une étape où l'on mesure l'activité d'une protéine kinase déterminée puis l'on sélectionne les produits ayant un effet inhibiteur sur cette activité déterminant ainsi les propriétés antifongiques des produits selon la présente invention.

Les exemples suivants de produits de formule (I) selon la présente invention illustrent l'invention sans toutefois la limiter.

### Partie expérimentale

### EXEMPLE 1 : Dichlorhydrate de trans-N-[6-(5,6-dichloro-2,3-dihydro-1H-indol-1-yl)-9H-purin-2-yl]-1,4-cyclohexanediamine

### Stade 1 : 2-chloro-6-(2,3-dihydro-1H-indol-1-yl)-9H-purine

On mélange 189 mg de dichloro-2,6-purine,4 ml de butanol, 143 mg (1,2 équivalent) d'indoline et porte à une température de 80°C environ 17 heures. On laisse revenir à température ambiante. On essore et lave à l'éther éthylique, on sèche sous vide et on obtient 262 mg de produit attendu sous forme de cristaux couleur beige.

### Stade 2 : Dichlorhydrate de trans-N-[6-(5,6-dichloro-2,3-dihydro-1H-indol-1-yl)-9H-purin-2-yl]-1,4-cyclohexanediamine

On porte 800 mg de trans-1,4-diaminocyclohexane à sa température de fusion (70°C) on ajoute en une fois 190 mg de produit obtenu au stade 1 ci-dessus et on chauffe alors à 140°C pendant environ 6 heures.
On laisse revenir à température ambiante. On purifie par chromatographie sur silice avec pour éluant un mélange MeOH-NH40H 98-2. On ajoute 4 ml d'éthanol et 4 ml d'HCl-EtOH (acide chlorhydrique-éthanol) et lave à l'éthanol. On sèche sous vide à 50°C. On obtient 67 mg de produit attendu.

### EXEMPLE 2 : Dichlorhydrate de trans-N-[6-(1H-benzimidazol-1-yl)-9H-purin-2-yl]-1,4-cyclohexanediamine

### Stade 1 : 6-(1H-benzimidazol-1-yl)-2-chloro-9H-purine

On procède comme à l'exemple 1 en partant de 1,89 g de dichloro-2,6-purine, 40 ml de butanol, 1,30 g de benzimidazole. On obtient 1,1 g de produit attendu

### Stade 2 : Dichlorhydrate de trans-N-[6-(1H-benzimidazol-1-yl)-9H-purin-2-yl]-1,4-cyclohexanediamine

On porte 2,52 g de trans-1,4-diaminocyclohexane à sa température de fusion (70°C) on ajoute 865 mg de produit obtenu au stade 1 ci-dessus et on chauffe alors à 140°C pendant environ 5 heures.
On laisse revenir à température ambiante. On purifie par chromatographie sur silice avec pour éluant un mélange CH2Cl2-MeOH-NH4OH : 78-20-2. On ajoute 10 ml d'éthanol et 6 ml d'HCl-EtOH (acide chlorhydrique-éthanol). On évapore à sec puis on empâte dans l'éther éthylique. On sèche sous vide.
On obtient 397 mg de produit attendu.

### EXEMPLE 3 : 6-(1H-benzimidazol-1-yl)-9H-purin-2-amine

On mélange 500 mg de 2 amino 6-purine, 5 ml de butanol, 383 mg de benzimidazole et porte à une température de 90°C environ 48 heures, puis 140°C 48 heures. On purifie par chromatographie sur silice avec pour éluant un mélange CH2Cl2-MeOH-NH4OH : 95-5-0,3.

On empâte dans le méthanol. On obtient 250 mg de produit attendu.

### EXEMPLE 4 : 6-(1H-benzimidazol-1-yl)-N,N-diméthyl-9H-purin-2-amine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 6-(1H-benzimidazol-1-yl)-N,N-diméthyl-9H-purin-2-amine

On mélange 300 mg de produit obtenu au stade 1 ci-dessus avec 3 ml de DMF(diméthyl formamide) et 0,17 ml (1,1 équivalent) de TEA (TriÉthyl Amine). On chauffe à 90°C pendant 2 jours. On essore le précipité. On purifie par chromatographie sur silice avec pour éluant un mélange CH2Cl2-MeOH-NH4OH : 90-9-9. On obtient 298 mg de produit attendu.

### EXEMPLE 5 : Trans-N-[6-(5,6-diméthyl-1H-benzimidazole-1-yl)-9H-purin-2-yl]-1,4-cyclohexanediamine

### Stade 1 : 2-chloro-6-(5,6-diméthyl-1H-benzimidazol-1-yl)-9H-purine

On mélange 283 mg de dichloro-2,6-purine,5 ml de butanol, 219 mg de 5,6 diméthyl benzimidazole et porte à une température de 100°C environ 17 heures. On laisse revenir à température ambiante. On essore et lave à l'isopropanol, on sèche sous vide à 50°C et on obtient 194 mg de produit attendu sous forme de cristaux couleur crème.

### Stade 2 : Trans-N-[6-(5,6-diméthyl-1H-benzimidazol-1-yl)-9H-purin-2-yl]-1,4-cyclohexanediamine

On porte 570 mg de trans1,4-diaminocyclohexane à sa température de fusion (70°C) on ajoute en une fois 149 mg de produit obtenu au stade 1 ci-dessus et on chauffe alors à 140°C pendant environ 18 heures.
On laisse revenir à température ambiante. On purifie par chromatographie sur silice avec pour éluant un mélange MeOH-NH4OH : 98-2. On obtient 40 mg de produit attendu sous forme de cristaux de couleur beige.

### EXEMPLE 6 : 3-[[6-(1H-benzimidazol-1-yl)-9H-purin-2-yl]amino]-benzoate d'éthyle

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu

### Stade 2 : 3-[[6-(1H-benzimidazol-1-yl)-9H-purin-2-yl]amino]-benzoate d'éthyle

On mélange 300 mg de produit obtenu au stade 1 ci-dessus avec 0,83 ml (5 équivalent) de 3 amino benzoate d'éthyle et 0,166 mg de NaI et on chauffe alors à 140°C pendant environ 4 jours.
On laisse revenir à température ambiante et on agite à température ambiante pendant 2 jours. On purifie par chromatographie sur silice avec pour éluant un mélange CH2Cl2-MeOH-NH4OH : 95-5-0,3 On obtient 25,2 mg de produit attendu.

### EXEMPLE 7 : Trans-N-[6-(5-chloro-2,3-dihydro-1H-indol-1-yl)-9H-purin-2-yl]-1,4-cyclohexanediamine

### Stade 1 : 2-chloro-6-(5-chloro-2,3-dihydro-1H-indol-1-yl)-9H-purine

On procède comme au stade 1 de l'exemple 1 à partir de 756 mg de dichloro-2,6-purine, 12 ml de butanol et 737 mg de 5-chloro-2,3-dihydro-1H-indole.
On porte à une température de 80°C environ 20 heures.
On laisse revenir à température ambiante. On essore et lave à l'isopropyle et sèche. on obtient ainsi 1,67 g de produit attendu

### Stade 2 : Trans-N-[6-(5-chloro-2,3-dihydro-1H-indol-1-yl)-9H-purin-2-yl]-1,4-cyclohexanediamine

On procède comme au stade 2 de l'exemple 1 à partir de 1,14 g de trans 1,4-diaminocyclohexane et 306 mg du produit obtenu au stade 1 ci-dessus ; on chauffe alors à 120°C pendant environ 6 heures.
On obtient 140 mg de produit attendu sous forme de cristaux de couleur beige.

### EXEMPLE 8 : Chlorhydrate de trans-N-[6-(5,6-dichloro-1H-benzimidazol-1-yl)-9H-purin-2-yl]-1,4-cyclohexanediamine

### Stade 1 : 2-chloro-6-(5,6-dichloro-1H-benzimidazol-1-yl)-9H-purine

On mélange 567 mg de dichloro-2,6-purine,6 ml de butanol, 617 mg de 5,6-dichlorobenzimidazole et porte à une température de 100°C environ 24 heures. On laisse revenir à température ambiante. On essore et lave à l'isopropanol, on sèche sous vide à 50°C et on obtient 548 mg de produit attendu sous forme de cristaux couleur grise/noire.

### Stade 2 : Chlorhydrate de trans-N-[6-(5,6-dichloro-1H-benzimidazol-1-yl)-9H-purin-2-yl]-1,4-cyclohexanediamine

On porte 570 mg de trans 1,4-diaminocyclohexane à sa température de fusion (70°C) on ajoute en une fois 170 mg de produit obtenu au stade 1 ci-dessus et on chauffe alors à 110°C pendant environ 24 heures. On laisse revenir à température ambiante. On purifie par chromatographie sur silice avec pour éluant un mélange MeOH-NH4OH : 98-2. On dissout dans l'éthanol puis on ajoute HCl-AcOEt (acide chlorhydrique-acétate d'éthyle). On essore et sèche sous vide à 50°C. On obtient 34 mg de produit attendu sous forme de cristaux de couleur marron.

### EXEMPLE 9 : 6-(1H-benzimidazol-1-yl)-N-(phénylméthyl-9H-purin-2-amine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu

### Stade 2 : 6-(1H-benzimidazol-1-yl)-N-(phénylméthyl-9H-purin-2-amine

On mélange 300 mg de produit obtenu au stade 1 ci-dessus avec 2 ml de DMSO et 0,27 g (5 équivalent) de n-butylamine et on chauffe alors à 120°C pendant environ 48 heures. On laisse revenir à température ambiante. On purifie par chromatographie sur silice avec pour éluant un mélange CH2Cl2-MeOH-NH4OH : 90-9-1. On évapore et on empâte dans le chlorure de méthylène. On obtient 351 mg de produit attendu.

### EXEMPLE 10 : 6-(1H-benzimidazol-1-yl)-N-butyl-9H-purin-2-amine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu

### Stade 2 : 6-(1H-benzimidazol-1-yl)-N-butyl-9H-purin-2-amine

On mélange 200 mg de produit obtenu au stade 1 ci-dessus avec 3 ml de DMSO et 0,6 ml (5 équivalent) de benzylamine et on chauffe alors à 120°C pendant environ 60 heures. On laisse revenir à température ambiante. On purifie par chromatographie sur silice avec pour éluant un mélange CH2Cl2-MeOH-NH4OH : 90-9-1. On évapore et on empâte dans le chlorure de méthylène On essore et sèche sous vide à 50°C. On obtient 105 mg de produit attendu.

### EXEMPLE 11 : 2-[[6-(1H-benzimidazol-1-yl)-9H-purin-2-yl]amino]-éthanol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu

### Stade 2 : 2-[[6-(1H-benzimidazol-1-yl)-9H-purin-2-yl]amino]-éthanol

On mélange 210 mg de produit obtenu au stade 1 ci-dessus avec 2 ml de DMSO et 0,3 ml d'éthanolamine et on chauffe alors à 120°C pendant environ 48 heures sous agitation. On laisse revenir à température ambiante. On purifie par chromatographie sur silice avec pour éluant un mélange CH2Cl2-MeOH-NH4OH : 90-9-1. On évapore et on empâte dans le chlorure de méthylène-méthanol (5-5). On essore et sèche sous vide à 50°C. On obtient 118 mg de produit attendu.

### EXEMPLE 12 : 6-(1H-benzimidazol-1-yl)-N-méthyl-9H-purin-2-amine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 6-(1H-benzimidazol-1-yl)-N-méthyl-9H-purin-2-amine

On mélange 200 mg de produit obtenu au stade 1 ci-dessus avec 2 ml de DMSO et 0,115 ml (5 équivalent) de méthylamine et on chauffe alors à 120°C pendant environ 18 heures. On laisse revenir à température ambiante. On purifie par chromatographie sur silice avec pour éluant un mélange CH2Cl2-MeOH-NH4OH : 90-9-1. On évapore et on empâte dans le chlorure de méthylène. On essore et sèche sous vide à 50°C. On obtient 190 mg de produit attendu.

### EXEMPLE 13 : 6-(1H-benzimidazol-1-yl)-N-cyclohexyl-9H-purin-2-amine

### Stade 1 : 6-(1H-benzimidazol-1-yl)-2-chloro-9H-purine

On mélange 8g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 6-(1H-benzimidazol-1-yl)-N-cyclohexyl-9H-purin-2-amine

On mélange 200 mg de produit obtenu au stade 1 ci-dessus avec 2 ml de DMSO et 0,42 ml de cyclohexylamine et on chauffe alors à 110°C pendant environ 48 heures. On laisse revenir à température ambiante. On purifie par chromatographie sur silice avec pour éluant un mélange CH2Cl2-MeOH-NH4OH : 90-9-1. On obtient 84,7 mg de produit attendu.

### EXEMPLE 14 : 2,2'-[[6-(1H-benzimidazol-1-yl)-9H-purin-2-yl]imino]bis-éthanol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 2,2'-[[6-(1H-benzimidazol-1-yl)-9H-purin-2-yl]imino]bis-éthanol

On mélange 200 mg de produit obtenu au stade 1 ci-dessus avec 3 ml de DMSO et 0,388 mg (5 équivalent) de diéthanolamine et on chauffe alors à 120°C pendant environ 48 heures. On laisse revenir à température ambiante. On purifie par chromatographie sur silice avec pour éluant un mélange CH2Cl2-MeOH-NH4OH : 90-9-1. On évapore et on empâte dans le chlorure de méthylène. On essore et sèche sous vide à 50°C. On obtient 88,2 mg de produit attendu.

### EXEMPLE 15 : Dichlorhydrate de trans-N-[6-(5-méthoxy-1H-benzimidazol-1-yl)-9H-purin-2-yl]-1,4-cyclohexanediamine

### Stade 1 : 2-chloro-6-(5-méthoxy-1H-benzimidazol-1-yl)-9H-purine

On mélange 567 mg de dichloro-2,6-purine, 15 ml de butanol, 999 mg de 5-OCH3-benzimidazole et porte à une température de 120°C environ 24 heures. On laisse revenir à température ambiante. On essore et lave avec H2C12, on sèche sous vide à 50°C. On reprend dans H2O+ ; on ajoute 1 ml de NH4OH, puis on extrait avec CH2Cl2, on ajoute une solution aqueuse saturée de NaCl. On sèche sur sulfate de sodium et on amène à sec. On obtient 60 mg de produit attendu sous forme de cristaux couleur blanche.

### Stade 2 : Dichlorhydrate de trans-N-[6-(5-méthoxy-1H-benzimidazol-1-yl)-9H-purin-2-yl]-1,4-cyclohexanediamine

On porte 969 mg de trans1,4-diaminocyclohexane à sa température de fusion (70°C) on ajoute en une fois 5100 mg de produit obtenu au stade 1 ci-dessus (sans nom) et 10 ml de DMSO et on chauffe alors à 120°C pendant environ 72 heures. On laisse revenir à température ambiante. On purifie par chromatographie sur silice avec pour éluant un mélange MeOH-NH4OH : 98-2. On dissout dans l'éthanol puis on ajoute HCl-AcOEt. On essore et sèche sous vide à 60°C. On purifie par chromatographie sur silice avec pour éluant un mélange CH2Cl2-MeOH-NH4OH : 75-23-2. On obtient 258 mg de produit attendu sous forme de cristaux de couleur beige.

### EXEMPLE 16 : Dichlorhydrate de trans-N-[6-(1H-indol-1-yl)-9H-purin-2-yl]-1,4-cyclohexanediamine

### Stade 1 : 2-chloro-6-(1H-indol-1-yl)-9H-purine

On chauffe 236 mg de produit obtenu au stade 1 de l'exemple 1 dans 20 ml de dioxane sur siliparite avec 227 mg de DDQ ( Dichloro Dicyano-Benzoquinone) pendant 60 heures à 80°C. On évapore le dioxane puis on purifie par chromatographie sur silice avec pour éluant un mélange CH2Cl2-MeOH : 95-5. On obtient 142 mg de produit attendu sous forme de cristaux couleur beige.

### Stade 2 : Dichlorhydrate de trans-N-[6-(1H-indol-1-yl)-9H-purin-2-yl]-1,4-cyclohexanediamine

On porte 1,31 g de trans 1,4-diaminocyclohexane à sa température de fusion (70°C) on ajoute 310 mg de produit obtenu au stade 1 ci-dessus et on chauffe alors à 140°C pendant environ 18 heures. On laisse revenir à température ambiante. On purifie par chromatographie sur silice avec pour éluant un mélange MeOH-NH4OH : 98-2. On ajoute 4 ml d'éthanol puis 2 ml d'acide chlorhydrique-éthanol (8N). On essore puis on lave à l'éthanol. On sèche sous vide. On obtient 23 mg de produit attendu sous forme de cristaux de couleur beige.

### EXEMPLE 17 : 6-(1H-benzimidazol-1-yl)-N-phényl-9H-purin-2-amine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 6-(1H-benzimidazol-1-yl)-N-phényl-9H-purin-2-amine

On mélange 300 mg de produit obtenu au stade 1 ci-dessus avec 0,52 ml d'aniline et on chauffe alors à 140°C pendant environ 72 heures. On laisse revenir à température ambiante 48 heures. On empâte en chlorure de méthylène on essore. On obtient 48 mg de produit attendu.

### EXEMPLE 18 : 2-[[6-(1H-benzimidazol-1-yl)-9H-purin-2-yl]amino]-1,3-propanediol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 2-[[6-(1H-benzimidazol-1-yl)-9H-purin-2-yl]amino]-1,3-propanediol

On mélange 200 mg de produit obtenu au stade 1 ci-dessus avec 3 ml de DMSO et 336 mg (5 équivalent) de 2-amino-1,3-propanediol et on chauffe alors à 120°C pendant environ 72 heures. On laisse revenir à température ambiante. On purifie par chromatographie sur silice avec pour éluant un mélange CH2Cl2-MeOH-NH4OH : 90-9-1. On évapore et on empâte dans le chlorure de méthylène-méthanol : 50-50. On essore et sèche sous vide à 50°C. On obtient 91,4 mg de produit attendu.

### EXEMPLE 19 : Trans-N-[6-[6-(phénylméthoxy)-1H-benzimidazol-1-yl]-9H-purin-2-yl]-1,4-cyclohexanediamine

### Stade 1 : 2,6-dichloro-9-[[2-(triméthylsilyl)éthoxy] méthyl]-9H-purine

On mélange 945 mg de dichloro-2,6-purine, 15 ml de DMF (DiMéthyl Formamide) et 288 mg de NaH, on laisse 2 heures à température ambiante. On ajoute alors 1,06 ml de 2-chlorométhoxyléthyl-triméthylsilanne. On agite 18 heures à température ambiante. On évapore le DMF. On reprend en chlorure de méthylène. On lave avec une solution saturée de bicarbonate de sodium, puis une fois avec H2O et enfin avec une solution saturée de NaCl. On sèche sur Na2SO4 et on évapore à sec. On purifie par chromatographie sur silice avec pour éluant un mélange CHCl₃-AcOEt : 5-5. On obtient 876 mg de produit attendu sous forme d'huile de couleur jaune.

### Stade 2 : 2-chloro-6-[6-(phénylméthoxy)-1H-benzimidazol-1-yl]-9-[[2-(triméthylsilyl)éthoxy]méthyl]-9H-purine

On mélange 860 mg de produit obtenu au stade 1 ci-dessus avec 605 mg de 5-(phénylméthoxy)-1H-benzimidazole (éther benzylique en 5 du benzimidazole) et 15 ml de butanol. On chauffe à 120°C pendant 24 heures. On essore, lave avec de l'H2O puis sèche sous vide à 50°C. On obtient 667 mg de 2-chloro-6-[6-(phénylméthoxy)-1H-benzimidazol-1-yl]-9-[[2-(triméthylsilyl)éthoxy] méthyl]-9H-purine sous forme de cristaux de couleur beige.

### Stade 3 : Trans-N-[6-[6-(phénylméthoxy)-1H-benzimidazol-1-yl]-9H-purin-2-yl]-1,4-cyclohexanediamine

On porte 1 g de trans 1,4-diaminocyclohexane à sa température de fusion (70°C) on ajoute 15 ml de DMSO, 530 mg de produit obtenu au stade 1 ci-dessus et on chauffe alors à 120°C pendant environ 21 heures. On purifie par chromatographie sur silice avec pour éluant un mélange CH2Cl2-MeOH-NH4OH : 85-13,5-1,5. On obtient 38 mg de produit attendu sous forme de cristaux de couleur beige.

### EXEMPLE 20 : Trans-N-[6-[5-(phénylméthoxy)-1H-benzimidazol-1-yl]-9H-purin-2-yl]-1,4-cyclohexanediamine

### Stade 1 : 2,6-dichloro-9-[[2-(triméthylsilyl)éthoxy] méthyl]-9H-purine

On mélange 945 mg de dichloro-2,6-purine, 15 ml de DMF (DiMéthyl Formamide) et 288 mg de NaH, on laisse 2 heures à température ambiante. On ajoute alors 1,06 ml de 2-chlorométhoxyléthyl-triméthylsilanne. On agite 18 heures à température ambiante. On évapore le DMF. On reprend en chlorure de méthylène. On lave avec une solution saturée de bicarbonate de sodium, puis une fois avec H2O et enfin avec une solution saturée de NaCl. On sèche sur Na2SO4 et on évapore à sec. On purifie par chromatographie sur silice avec pour éluant un mélange CHCl3-AcOEt : 5-5. On obtient 876 mg de produit attendu sous forme d'huile de couleur jaune.

### Stade 2 : 2-chloro-6-[5-(phénylméthoxy)-1H-benzimidazol-1-yl]-9-[[2-(triméthylsilyl)éthoxy]méthyl]-9H-purine

On mélange 860 mg de produit obtenu au stade 1 ci-dessus avec 605 mg de 5-(phénylméthoxy)-1H-benzimidazole (éther benzylique en 5 du benzimidazole) et 15 ml de butanol. On chauffe à 120°C pendant 24 heures. On essore, lave avec de l'H2O puis sèche sous vide à 50°C. On obtient 667 mg de 2-chloro-6-[5-(phénylméthoxy)-1H-benzimidazol-1-yl]-9-[[2-(triméthylsilyl)éthoxy] méthyl]-9H-purine sous forme de cristaux de couleur beige.

### Stade 3 : Trans-N-[6-[5-(phénylméthoxy)-1H-benzimidazol-1-yl]-9H-purin-2-yl]-1,4-cyclohexanediamine

On porte 1 g de trans 1,4-diaminocyclohexane à sa température de fusion (70°C) on ajoute 15 ml de DMSO, 530 mg de produit obtenu au stade 1 ci-dessus et on chauffe alors à 120°C pendant environ 21 heures. On purifie par chromatographie sur silice avec pour éluant un mélange CH2Cl2-MeOH-NH4OH : 85-13,5-1,5. On obtient 39 mg de produit attendu sous forme de cristaux de couleur beige.

### EXEMPLE 21 : Dichlorhydrate de trans-N-[6-(1H-benzimidazol-1-yl)-9H-purin-2-yl]-1,2-cyclohexanediamine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : Dichlorhydrate de trans-N-[6-(1H-benzimidazol-1-yl)-9H-purin-2-yl]-1,2-cyclohexanediamine

On mélange 200 mg de produit obtenu au stade 1 ci-dessus avec 3 ml de DMSO et 420 mg (5 équivalent) de (1S,2S)-(-)-1,2-diaminocyclohexane et on chauffe alors à 120°C pendant environ 4 jours. On laisse revenir à température ambiante. On purifie par chromatographie sur silice avec pour éluant un mélange CH2Cl2-MeOH-NH4OH : 95-5-0,33. On ajoute 4 ml d'éthanol et 4 ml d'HCl-EtOH (acide chlorhydrique-éthanol). On évapore à sec puis on empâte dans l'éther éthylique. On sèche sous vide. On obtient 116 mg de produit attendu.

### EXEMPLE 22 : [1-[6-(1H-benzimidazol-1-yl)-9H-purin-2-yl]-4-piperidinyl]-carbamate de 1,1-diméthyléthyle

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : [1-[6-(1H-benzimidazol-1-yl)-9H-purin-2-yl]-4-piperidinyl]-carbamate de 1,1-diméthyléthyle

On mélange 200 mg de produit obtenu au stade 1 ci-dessus avec 3 ml de DMSO et 740 mg (5 équivalent) de Boc-4-aminopiperidine et on chauffe alors à 120°C pendant environ 4 heures. On laisse revenir à température ambiante. On purifie par chromatographie sur silice avec pour éluant un mélange CH2Cl2-MeOH-NH4OH : 90-9-1. On évapore à sec puis on empâte dans le dichlorométhane. On sèche sous vide. On obtient 252 mg de produit attendu.

### EXEMPLE 23 : Dichlorhydrate de cis-N-[6-(1H-benzimidazol-1-yl)-9H-purin-2-yl]-1,2-cyclohexanediamine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : Dichlorhydrate de cis-N-[6-(1H-benzimidazol-1-yl)-9H-purin-2-yl]-1,2-cyclohexanediamine

On mélange 200 mg de produit obtenu au stade 1 ci-dessus avec 3 ml de DMSO et 0,5 ml (5 équivalent) de Cis-1,2-diaminocyclohexane et on chauffe alors à 120°C pendant environ 3 jours. On laisse revenir à température ambiante. On purifie par chromatographie sur silice avec pour éluant un mélange CH2Cl2-MeOH-NH4OH : 85-15-1,5. On ajoute 3 ml d'éthanol et 3 ml d'HCl-EtOH (acide chlorhydrique-éthanol). On obtient 34,3 mg de produit attendu.

### EXEMPLE 24 : Trans-4-[[6-(1H-benzimidazol-1-yl)-9H-purin-2-yl]amino]-cyclohexanol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : Trans-4-[[6-(1H-benzimidazol-1-yl)-9H-purin-2-yl]amino]-cyclohexanol

On mélange 200 mg de produit obtenu au stade 1 ci-dessus avec 3 ml de DMSO et 586 mg (5 équivalents) de Trans-4-aminocyclohexanol et on chauffe alors à 120°C pendant environ 4 jours. On laisse revenir à température ambiante. On purifie par chromatographie sur silice avec pour éluant un mélange CH2Cl2-MeOH-NH4OH : 85-15-1,5. On obtient 45 mg de produit attendu.

### EXEMPLE 25 : Bis(trifluoroacétate) de 1-[6-(1H-benzimidazol-1-yl)-9H-purin-2-yl]-4-piperidinamine

On mélange 100 mg de produit obtenu à l'exemple 22 avec 3 ml de chlorure de méthylène et 1,5 ml d'acide trifluoroacétique à 10 % d'anisole. On agite à température ambiante pendant 5 heures. On concentre à sec et on co-évapore au toluène puis au chlorure de méthylène. On purifie par chromatographie sur silice avec pour éluant un mélange CH2Cl2-MeOH-NH4OH : 90-9-1. On obtient 128 mg de produit attendu.

### EXEMPLE 26 : Dichlorhydrate de trans-N-[6-(2-méthyl-1H-indol-1-yl)-9H-purin-2-yl]-1,4-cyclohexanediamine

### Stade 1 : 6-(2-méthyl-1H-indol-1-yl)-2-chloro-9H-purine

On procède comme au stade 1 de l'exemple 1 en mélangeant 189 mg de dichloro-2,6-purine, 5 ml de butanol et 0,16 g de 2-méthyl indoline. On chauffe à 130°C pendant environ 1 heure et on laisse revenir à température ambiante. On essore et on lave à l'isopropanol. On sèche et on obtient ainsi 174 mg de produit attendu.

### Stade 2 : Dichlorhydrate de trans-N-[6-(2-méthyl-1H-indol-1-yl)-9H-purin-2-yl]-1,4-cyclohexanediamine

On procède comme au stade 2 de l'exemple 1 en partant de 638 mg de trans 1,4-diaminocyclohexane, de 161 mg du produit obtenu au stade 1 ci-dessus, on chauffe à 120°C pendant 29 heures. On purifie sur silice avec pour éluant MeOH-NH4OH : 98-2 et on obtient ainsi 190 mg de produit sous forme de résine couleur marron, on dissout dans l'éthanol et on ajoute HCl-AcOEt, le chlorhydrate précipite, on ajoute 3 ml d'AcOEt, on essore et on sèche sous vide à 50°C.

On obtient ainsi 222 mg du produit attendu sous forme de cristaux de couleur beige.

### EXEMPLE 27 : (2S)-2-[[6-(1H-benzimidazol-1-yl)-9H-purin-2-yl]amino]-1-butanol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : (2S)-2-[[6-(1H-benzimidazol-1-yl)-9H-purin-2-yl]amino]-1-butanol

On mélange 200 mg de produit obtenu au stade 1 ci-dessus avec 3 ml de DMSO et 350 µl (5 équivalents) (+/-)-2-amino-1-butanol et on chauffe alors à 120°C pendant environ 2 jours. On laisse revenir à température ambiante. On purifie par chromatographie sur silice avec pour éluant un mélange CH2Cl2-MeOH-NH4OH : 95-5-0,3. On obtient 40 mg de produit attendu.

### EXEMPLE 28 : 6-(1H-benzimidazol-1-yl)-N-[(tétrahydro-2H-pyran-4-yl)méthyl]-9H-purin-2-amine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 6-(1H-benzimidazol-1-yl)-N-[(tétrahydro-2H-pyran-4-yl)méthyl]-9H-purin-2-amine

On mélange 250 mg de produit obtenu au stade 1 ci-dessus avec 3 ml de DMSO et 531 mg µl de 4-aminométhyl tétrahydropyrane et on chauffe alors à 120°C pendant environ 2 jours. On laisse revenir à température ambiante. On purifie par chromatographie sur silice avec pour éluant un mélange CH2Cl2-MeOH-NH4OH : 90-9-1. On obtient 142 mg de produit attendu.

### EXEMPLE 29 : Trans-N-[6-(2-méthyl-1H-indol-1-yl)-9H-purin-2-yl]-1,4-cyclohexanediamine

### Stade 1 : 6-(2-méthyl-1H-indol-1-yl)-2-chloro-9H-purine

On procède comme au stade 1 de l'exemple 1 en mélangeant 378 mg de dichloro-2,6-purine, 10 ml de butanol et 0,32 de diméthyl indoline. On chauffe à 100°C pendant environ 5 heures et on laisse revenir à température ambiante. On essore et on lave à l'isopropanol. On sèche et on obtient ainsi 423 mg de produit attendu.

### Stade 2 : 6-(2-méthyl-1H-indol-1-yl)-2-chloro-1H-purine

On mélange 140 mg de produit obtenu au stade 1 ci-dessus avec 170 mg de DDQ (Dichloro Dicyano-Benzoquinone) et 5 ml de dioxane. On porte à 80-90°C puis on laisse revenir à température ambiante. On filtre, on rince et on sèche. On purifie sur silice avec pour éluant CHCl3-EtOh-AcOEt : 90-5-5. On obtient ainsi 170 mg du produit attendu.

### Stade 3 : Trans-N-[6-(2-méthyl-1H-indol-1-yl)-9H-purin-2-yl]-1,4-cyclohexanediamine

On procède comme au stade 2 de l'exemple 1 en partant de 684 mg de trans 1,4-diaminocyclohexane, de 166 mg du produit obtenu au stade 2 ci-dessus, on chauffe à 140°C pendant 6 heures On purifie sur silice avec pour éluant MeOH-NH4OH : 98-2 et on obtient ainsi 51 mg de produit attendu sous forme de cristaux de couleur beige.

### EXEMPLE 30 : Dichlorhydrate de trans-N-[6-(2,3-dihydro-5-nitro-1H-indol-1-yl)-9H-purin-2-yl]-1,4-cyclohexanediamine

### Stade 1 : 2-chloro-6-(2,3-dihydro-5-nitro-1H-indol-1-yl)-9H-purine

On mélange 567 mg de dichloro-2,6-purine, 10 ml de butanol et 590 mg de 5-nitroindole. On chauffe à 80°C pendant environ 17 heures et on laisse revenir à température ambiante. On purifie par chromatographie sur silice avec pour éluant un mélange CHCl3-éthanol : 95-5.

On obtient ainsi 834 mg de produit attendu sous forme de cristaux de couleur jaunâtre.

### Stade 2 : Trans-N-[6-(2,3-dihydro-5-nitro-1H-indol-1-yl)-9H-purin-2-yl]-1,4-cyclohexanediamine, dihydrochloride

On mélange 421 mg du produit obtenu au stade 1 ci-dessus avec 1,14 g de trans 1,4-diaminocyclohexane, et 10 ml de DMSO. On chauffe à 120°C pendant environ 29 heures. On sèche sous vide à 50°C et on purifie par chromatographie sur silice avec pour éluant un mélange CH2Cl2-MeOH-NH4OH : 85-13, 5-1, 5. On empâte dans l'éthanol puis on ajoute 2 ml de HCl-Éthanol. On sèche sous vide.

On obtient ainsi 113 mg de produit attendu sous forme de cristaux de couleur jaune moutarde.

### EXEMPLE 31 : 4-[[[6-(1H-benzimidazol-1-yl)-9H-purin-2-yl]amino]méthyl]-1-piperidinecarboxylate de 1,1-diméthyléthyle

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : 4-[[[6-(1H-benzimidazol-1-yl)-9H-purin-2-yl]amino]méthyl]-1-piperidinecarboxylate de 1,1-diméthyléthyle

On mélange 200 mg de produit obtenu au stade 1 ci-dessus avec 5 ml de DMSO et 790 mg (5 équivalent) de 4-amine méthyl-N-Boc-piperidine et on chauffe alors à 100°C pendant environ 3 jours. On laisse revenir à température ambiante. On concentre à sec le DMSO et on reprend en CH2Cl2. On purifie par chromatographie sur silice avec pour éluant un mélange CH2Cl2-MeOH-NH4OH : 95-5-0,33. On obtient 56 mg de produit attendu.

### EXEMPLE 32 : Trans-N-[6-(5-bromo-2,3-dihydro-1H-indol-1-yl)-9H-purin-2-yl]-1,4-cyclohexanediamine

### Stade 1 : 6-(5-bromo-2,3-dihydro-9H-indol-1-yl)-2-chloro-9H-purine

On procède comme au stade 1 de l'exemple 7 en utilisant 950 mg de 5-bromo-2,3-dihydro-1H-indole à la place de 737 mg de 5-chloro-2,3-dihydro-1H-indole. On obtient ainsi 1,32 g de produit attendu

### Stade 2 : Trans-N-[6-(5-bromo-2,3-dihydro-1H-indol-1-yl)-9H-purin-2-yl]-1,4-cyclohexanediamine

On procède comme au stade 2 de l'exemple 7 à partir de 350 mg du produit obtenu au stade 1 ci-dessus à la place de 306 mg du produit obtenu au stade 1 de l'exemple 7. On obtient 35 mg de produit attendu sous forme de cristaux de couleur marron.

### EXEMPLE 33 : Dichlorhydrate de 6-(1H-benzimidazol-1-yl)-2-(4-morpholinyl)-9H-purine

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : Dichlorhydrate de 6-(1H-benzimidazol-1-yl)-2-(4-morpholinyl)-9H-purine

On mélange 230 mg de produit obtenu au stade 1 ci-dessus avec 3 ml de DMSO et 370 mg (5 équivalents) de morpholine et on chauffe alors à 120°C pendant environ 16 heures. On laisse revenir à température ambiante. On concentre à sec le DMSO. On empâte dans CH2Cl2 (chlorure de méthylène). On sèche sous vide. On reprend dans 5 ml de HCl (8M) et 10 ml d'éthanol on concentre à sec et on obtient 209 mg de produit attendu.

### EXEMPLE 34 : (2S)-1-[6-(1H-benzimidazol-1-yl)-9B-purin-2-yl]-2-pyrrolidineméthanol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : (2S)-1-[6-(1H-benzimidazol-1-yl)-9H-purin-2-yl]-2-pyrrolidineméthanol

On mélange 250 mg de produit obtenu au stade 1 ci-dessus avec 2 ml de DMSO et 470 mg de (S)-(+)-2-pyrrolidine méthanol. On chauffe alors à 110°C pendant environ 16 heures. On laisse revenir à température ambiante. On empâte dans CH2Cl2 (chlorure de méthylène) On sèche sous vide. On obtient 200 mg de produit attendu.

### EXEMPLE 35 : (2R)-1-[6-(1H-benzimidazol-1-yl)-9H-purin-2-yl]-2-pyrrolidineméthanol

### Stade 1 : 6-(1H-benzimidazol-1-yl)-2-chloro-9H-purine

On mélange 8 g de dichloro-2,6-purine, 150 ml de butanol, 5,5 g de benzimidazole et porte à une température de 80°C environ 17 heures. On laisse revenir à température ambiante. On essore et lave à l'éther éthylique, on sèche sous vide et on obtient 3 g de produit attendu.

### Stade 2 : (2R)-1-[6-(1H-benzimidazol-1-yl)-9H-purin-2-yl]-2-pyrrolidineméthanol

On mélange 250 mg de produit obtenu au stade 1 ci-dessus avec 2 ml de DMSO et 400 ml (4 équivalent) de (R)-(-)-2- pyrrolidine méthanol et on chauffe alors à 120°C pendant environ 5 heures. On laisse revenir à température ambiante. On concentre à sec le DMSO et on reprend en CH2C12 puis on essore. On obtient 258 mg de produit attendu.

### EXEMPLE 36 : Bis(trifluoroacétate) de 6-(1H-benzimidazol-1-yl)-N-(4-piperidinylméthyl)-9H-purin-2-amine

On mélange 35 mg du produit de l'exemple 31 avec 1 ml de CH2CL2 et 0,5 ml de TFA 10 % d'anisole. On laisse agiter 2 heures à température ambiante puis on concentre à sec en présence de toluène et de CH2Cl2. On obtient ainsi 40 mg de produit attendu.

### EXEMPLE 37 : Dichlorhydrate de trans-1-[2-[(4-aminocyclohexyl)amino]-9H-purin-6-yl]-2,3-dihydro-N,N-diméthyl-1H-indole-5-sulfonamide

### Stade 1 : 1-(2-chloro-9H-purin-6-yl)-2,3-dihydro-N,N-diméthyl-9H-indole-5-sulfonamide

On procède comme au stade 1 de l'exemple 7 en utilisant 1,08 g de 2,3-dihydro-N,N-diméthyl-1H-indole-5-sulfonamide à la place de 737 mg de 5-chloro-2,3-dihydro-1H-indole. On porte à une température de 80°C environ 20 heures. On obtient ainsi 1,628 g de produit attendu.

### Stade 2 : Dichlorhydrate de trans-1-[2-[(4-aminocyclohexyl)amino]-9H-purin-6-yl]-2,3-dihydro-N,N-diméthyl-1H-indole-5-sulfonamide

On procède comme au stade 2 de l'exemple 7 à partir de 379 mg du produit obtenu au stade 1 ci-dessus à la place de 306 mg du produit obtenu au stade 1 de l'exemple 7.

On obtient 150 mg de produit attendu sous forme de cristaux de couleur crème.

### EXEMPLE 38 : Dichlorhydrate de trans-N-[6-(5-fluoro-2,3-dihydro-1H-indol-1-yl)-9H-purin-2-yl]-1,4-cyclohexane-diamine

### Stade 1 : 2-chloro-6-(5-fluoro-2,3-dihydro-1H-indol-1-yl)-9H-purine

On procède comme au stade 1 de l'exemple 7, en utilisant 658 mg de 5-fluoro-2,3-dihydro-1H-indole à la place de 737 mg de 5-chloro-2,3-dihydro-1H-indole. On obtient ainsi 1,088 g de produit attendu.

### Stade 2 : Dichlorhydrate de trans-N-[6-(5-fluoro-2,3-dihydro-1H-indol-1-yl)-9H-purin-2-yl]-1,4-cyclohexanediamine

On procède comme au stade 2 de l'exemple 7 à partir de 290 mg du produit obtenu au stade 1 ci-dessus à la place de 306 mg du produit obtenu au stade 1 de l'exemple 7.

On obtient 87 mg de produit attendu sous forme de cristaux de couleur beige.

| | Nomenclature | Exemples |
|---|---|---|
| 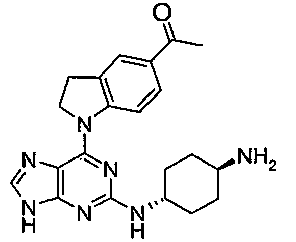 | Trans-1-[1-[2-[(4-aminocyclohexyl)amino]-9H-purin-6-yl]-2,3-dihydro-1H-indol-5-yl]-éthanone | **Exemple 39 840694** |
| 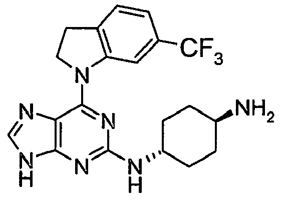 | *Trans*-*N*-[6-[2,3-dihydro-6-(trifluorométhyl)-1*H*-indol-1-yl)-9*H*-purin-2-yl]-1,4-cyclohexanediamine | **Exemple 40** |
| 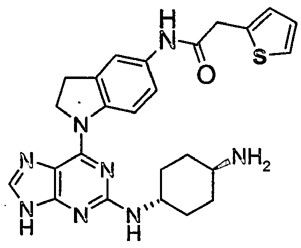 | Trans-N-[1-[2-[(4-aminocyclohexyl)amino]-9H-purin-6-yl]-2,3-dihydro-1H-indol-5-yl]-2-thiophèneacétamide | **Exemple 41** |
| 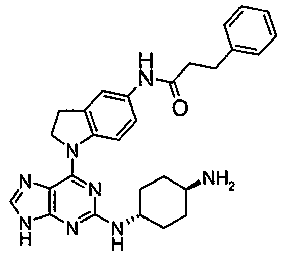 | Trans-N-[1-[2-[(4-aminocyclohexyl)amino]-9H-purin-6-yl]-2,3-dihydro-1H-indol-5-yl]-benzènepropanamide | **Exemple 42** |
| 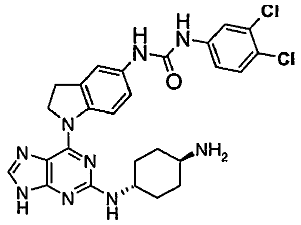 | Trans-N-[1-[2-[(4-aminocyclohexyl)amino]-9H-purin-6-yl]-2,3-dihydro-1H-indol-5-yl]-N'-(3,4-dichlorophényl)-urée | **Exemple 43** |
| 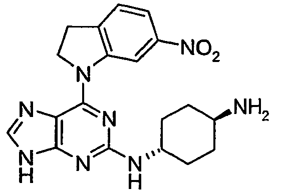 | *Trans*-*N*-[6-(6-nitro-2,3-dihydro-1*H*-indol-1-yl)-9*H*-purin-2-yl]-1,4-cyclohexanediamine | **Exemple 44** |
| 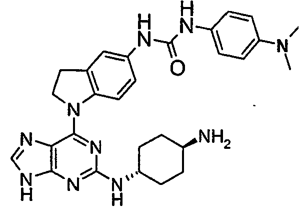 | Trans-N-[1-[2-[(4-aminocyclohexyl)amino]-9H-purin-6-yl]-2,3-dihydro-1H-indol-5-yl]-N'-[4-(diméthylamino)phényl]-urée | **Exemple 45** |
| 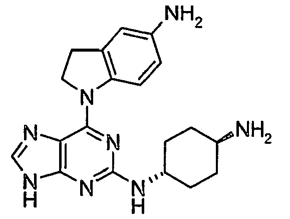 | *Trans*-*N*-[6-(5-amino-2,3-dihydro-1*H*-indol-1-yl)-9*H*-purin-2-yl]-1,4-cyclohexanediamine | **Exemple 46** |

### EXEMPLE 39 : Trans-1-[1-[2-[(4-aminocyclohexyl)amino]-9H-purin-6-yl]-2,3-dihydro-1H-indol-5-yl]-éthanone

### Stade 1 : 2-chloro-1-[1-(9E-purin-6-yl)-2,3-dihydro-1E-indol-5-yl]-éthanone

On mélange 1,10 g de dichloro-2,6-purine, 12 ml de butanol, 1,13 g d'acétylindoline et porte à une température de 90°C environ 3,5 heures. On laisse revenir à température ambiante. On essore et lave à l'éther éthylique, on sèche sous vide et on obtient 1,94 g de produit attendu.

### Stade 2 : Trans-1-[1-[2-[(4-aminocyclohexyl)amino]-9H-purin-6-yl]-2,3-dihydro-1H-indol-5-yl]-éthanone

On mélange 1,88g de produit obtenu au stade 1 ci-dessus avec 4,8g de Trans 1,4 di-aminocyclohexane et on chauffe alors à 140°C pendant environ 72 heures. On laisse revenir à température ambiante 48 heures. On empâte dans l'eau, on sèche sous vide à 50°C. On obtient 2,7g de produit attendu sous forme de poudre beige.

### EXEMPLE 40 : Trans-N-[6-[2,3-dihydro-6-(trifluorométhyl)-1H-indol-1-yl)-9H-purin-2-yl]-1,4-cyclohexanediamine

### Stade 1 : 2-chloro-6-[2,3-dihydro-6-(trifluorométhyl)-1H-indol-1-yl]-9H-purine

On mélange 756mg de dichloro-2,6-purine, 8 ml de butanol, 897 mg de 6-(trifluoromethyl)indoline et porte à une température de 90°C environ 23 heures. On laisse revenir à température ambiante. On essore et lave à l'éther éthylique, on sèche sous vide à 50°C et on obtient 1,256 g de produit attendu sous forme de cristaux beiges.

### Stade 2 : Trans-N-[6-[2,3-dihydro-6-(trifluorométhyl)-1H-indol-1-yl]-9H-purin-2-yl]-1,4-cyclohexanediamine

On mélange 340 mg de produit obtenu au stade 1 ci-dessus avec 800 mg de trans-1-4 diaminocyclohexane et on chauffe alors à 140°C pendant environ 8 heures. On laisse revenir à température ambiante. On purifie par chromatographie sur silice avec pour éluant un mélange CH2Cl2-MeOH-NH4OH : 85-15-1,5. On récupère 275 mg de produit on empâte dans HCL/Etanol ethanol : 50-50. On essore et sèche sous vide à 50°C. On obtient 244 mg de produit attendu.

### EXEMPLE 41 : Trans-N-[1-[2-[(4-aminocyclohexyl) amino]-9H-purin-6-yl]-2,3-dihydro-1H-indol-5-yl]-2-thiophèneacétamide

### Stade 1 : 2-chloro-6-(2,3-dihydro-5-nitro-1H-indol-1-yl)-9H-purine

On mélange 37,8 g de dichloro-2,6-purine, 700 ml de butanol, 32,8 g de 5-nitroindoline et porte à une température de 90°C environ 3 jours. On laisse revenir à température ambiante. On essore et lave à l'éther éthylique, on sèche sous vide à 50°C et on obtient 57,3 g de produit attendu sous forme de cristaux beiges.

### Stade 2 : trans-N-[6-(2,3-dihydro-5-nitro-1H-indol-1-yl)-9H-purin-2-yl]-1,4-cyclohexanediamine

On mélange 56 g de produit obtenu au stade 1 ci-dessus avec 140 g de trans-1-4 diaminocyclohexane et on chauffe alors à 140°C pendant environ 55 heures. On laisse revenir à température ambiante. On purifie par chromatographie sur silice avec pour éluant un mélange CH2Cl2-MeOH-NH4OH : 85-15-1,5. On récupère 19,7 g de produit.

### Stade 3 : Trans-6-(2,3-dihydro-5-nitro-1H-indol-1-yl)-2-[[4-[[(1,1-diméthyléthoxy)carbonyl]amino]cyclohexyl] amino]-9H-purine-9-carboxylate de 1,1-diméthyléthyle

On mélange 9,46 g de produit obtenu au stade 2 ci-dessus avec 160 ml de chloroforme et 2,4 ml de triéthylamine. On refroidit à 0°C puis ajoute en une fois 31,43 g de Boc₂O, laisse 10 minutes à 0°C puis porte au reflux 3 heures. On laisse revenir à température ambiante, ajoute 70 ml d'eau puis extrait par 150 ml de dichlorométhane. La phase organique est lavée par 70 ml de solution aqueuse saturée en NaCl, sèche sur sulfate de sodium, filtre et évapore à sec. On obtient ainsi 12,92 g de produit attendu

### Stade 4 : Trans-6-(5-amino-2,3-dihydro-1H-indol-1-yl)-2-[[4-[[(1,1-diméthyléthoxy)carbonyl]amino]cyclohexyl] amino]-9H-purine-9-carboxylate de 1,1-diméthyléthyle

Dans un ballon à hydrogéner on introduit 12,65 g de produit obtenu au stade 3 ci-dessus dans 130 ml de méthanol ; on ajoute 500 mg de Pd/C et agite sous pression de 1400 mbar d'hydrogène pendant 12 heures. On filtre sur clarcel puis évapore le solvant. Le résidu est chromatographié sur 600 g de silice, éluant dichlorométhane/Acétate d'éthyle 60/40.

On obtient ainsi 6 g de produit attendu.

### Stade 5 : Trans-6-[2,3-dihydro-5-[(2-thiénylacétyl) amino]-1H-indol-1-yl]-2-[[4-[[(1,1-diméthyléthoxy) carbonyl]amino]cyclohexyl]amino]-9H-purine-9-carboxylate de 1,1-diméthyléthyle

On mélange 40 mg de produit obtenu au stade 4 avec 11,4 mg de chlorure de 2-thiophène acétyle, 25 µl de diisopropyléthylamine et 2 ml de dichlorométhane. On agite 1 heure à température ambiante, puis lave à la saumure, sèche sur sulfate de magnésium et évapore à sec.

### Stade 6 : Trans-N-[1-[2-[(4-aminocyclohexyl)amino]-9H-purin-6-yl]-2,3-dihydro-1H-indol-5-yl]-2-thiophéne-acétamide

Le produit obtenu au stade 5 est dissous dans 2 ml de méthanol. On ajoute 2 ml d'une solution 2N d'acide chlorhydrique dans le méthanol. On agite 48 heures à température ambiante, puis évapore à sec. Le résidu est chromatographié sur une colonne LCMSprep Xterra, éluant Acétonitrile/tampon hydrogénocarbonate d'ammonium à 0,2 % en gradient. On récupère ainsi 6 mg de produit attendu, exemple 41.

### EXEMPLE 42 : Trans-N-[1-[2-[(4-aminocyclohexyl)amino]-9H-purin-6-yl]-2,3-dihydro-1E-indol-5-yl]-benzènepropanamide

### Stade 1 : Trans-6-[2,3-dihydro-5-[(1-oxo-3-phénylpropyl) amino]-1H-indol-1-yl]-2-[[4-[[(1,1-diméthyléthoxy) carbonyl]amino]cyclohexyl]amino]-9H-purine-9-carboxylate de 1,1-dimethyléthyle

On mélange 40 mg de produit obtenu au stade 4 de l'exemple 41 avec 11,97 mg de chlorure d'hydrocinnamoyle, 25µl de diisopropyléthylamine et 2 ml de dichlorométhane. On agite 1 heure à température ambiante, puis lave à la saumure, sèche sur sulfate de magnésium et évapore à sec.

### Stade 2 : Trans-N-[1-[2-[(4-aminocyclohexyl)amino]-9H-purin-6-yl]-2,3-dihydro-1H-indol-5-yl]-benzènepropanamide

Le produit obtenu au stade 1 est dissous dans 2 ml de méthanol. On ajoute 2 ml d'une solution 2N d'acide chlorhydrique dans le méthanol. On agite 48 heures à température ambiante, puis évapore à sec. Le résidu est chromatographié sur une colonne LCMSprep Xterra, éluant Acétonitrile/tampon hydrogénocarbonate d'ammonium à 0,2 % en gradient. On récupère ainsi 4 mg de produit attendu, exemple 42.

### EXEMPLE 43 : Trans-N-[1-[2-[(4-aminocyclohexyl)amino]-9H-purin-6-yl]-2,3-dihydro-1H-indol-5-yl]-N'-(3,4-dichlorophényl)-urée

### Stade 1 : Trans-6-[5-[[[(3,4-dichlorophényl)amino] carbonyl]amino]-2,3-dihydro-1H-indol-1-yl]-2-[[4-[[(1,1-diméthyléthoxy)carbonyl]amino]cyclohéxyl]amino]-9H-purine-9-carboxylate de 1,1-diméthyléthyle

On mélange 80 mg de produit obtenu au stade 4 de l'exemple 41 avec 37,3 mg d'isocyanate de 3,4-dichlorophényle et 5 ml de dichlorométhane. On agite 2 heures à température ambiante, rajoute 0,5 ml d'eau et évapore à sec. Purification sur LCMSprep colonne Xterra, éluant acétonitrile/tampon hydrogénocarbonate d'ammonium pH9. On obtient 35 mg de produit attendu.

### Stade 2 : Trans-N-[1-[2-[(4-aminocyclohéxyl)amino]-9H-purin-6-yl]-2,3-dihydro-1H-indol-5-yl]-N'-(3,4-dichlorophényl)-urée

Le produit obtenu au stade 1 est dissous dans 5 ml de méthanol. On ajoute 5 ml d'une solution 2N d'acide chlorhydrique dans le méthanol. On agite 48 heures à température ambiante, puis évapore à sec. Le résidu est chromatographié sur une colonne LCMSprep Xterra, éluant Acétonitrile/tampon hydrogénocarbonate d'ammonium à 0,2 % en gradient. On récupère ainsi 30 mg de produit attendu, exemple 43.

### EXEMPLE 44 : Trans-N-[6-(6-nitro-2,3-dihydro-1H-indol-1-yl)-9H-purin-2-yl]-1,4-cyclohexanediamine

### Stade 1 : 2-chloro-(6-nitro-2,3-dihydro-1H-indol-1-yl)-9H-purine

On mélange 378 mg de dichloro-2,6-purine, 7 ml de butanol, 328 mg de 6-nitroindoline et porte à une température de 90°C environ 3 jours. On laisse revenir à température ambiante. On essore et lave à l'éther éthylique, on sèche sous vide à 50°C et on obtient 573 mg de produit attendu sous forme de cristaux beiges.

### Stade 2 : Trans-N-[6-(6-nitro-2,3-dihydro-1H-indol-1-yl)-9H-purin-2-yl]-1,4-cyclohexanediamine

On mélange 560 mg de produit obtenu au stade 1 ci-dessus avec 1,40 g de trans-1-4 diaminocyclohexane et on chauffe alors à 140°C pendant environ 55 heures. On laisse revenir à température ambiante. On purifie par chromatographie sur silice avec pour éluant un mélange CH2Cl2-MeOH-NH4OH : 85-15-1,5. On récupère 197 mg de produit auquel on ajoute 10 ml de méthanol/CH2Cl2 et 6 ml HCL/Ethanol. On essore et sèche sous vide à 35°C. On obtient 27 mg de produit attendu.

### EXEMPLE 45 : Trans-N-[1-[2-[(4-aminocyclohexyl)amino]-9H-purin-6-yl]-2,3-dihydro-1H-indol-5-yl]-N'-[4-(diméthylamino)phényl]-urée

### Stade 1 : Trans-6-[5-[[[[4-(diméthylamino)phényl]amino] carbonyl]amino]-2,3-dihydro-1H-indol-1-yl]-2-[[4-[[(1,1-diméthyléthoxy)carbonyl]amino]cyclohéxyl]amino]-9H-purine-9-carboxylate de 1,1-diméthyléthyle

On mélange 80 mg de produit obtenu au stade 4 de l'exemple 41 avec 32,2 mg d'isocyanate de 4-diméthyl-aminophényle et 5 ml de dichlorométhane. On agite 20 heures à température ambiante, rajoute 0,5 ml d'eau et évapore à sec. Purification sur LCMSprep colonne Xterra, éluant acétonitrile/tampon hydrogénocarbonate d'ammonium pH9. On obtient 55 mg de produit attendu.

### Stade 2 : Trans-N-[1-[2-[(4-aminocyclohéxyl)amino]-9H-purin-6-yl]-2,3-dihydro-1H-indol-5-yl]-N'-[4-(diméthylamino)phényl]-urée

Le produit obtenu au stade 1 est dissous dans 5 ml de méthanol. On ajoute 5 ml d'une solution 2N d'acide chlorhydrique dans le méthanol. On agite 48 heures à température ambiante, puis évapore à sec. Le résidu est chromatographié sur une colonne LCMSprep Xterra, éluant Acétonitrile/tampon hydrogénocarbonate d'ammonium à 0,2 % en gradient. On récupère ainsi 42 mg de produit attendu, exemple 45.

### EXEMPLE 46 : Trans-N-[6-(5-amino-2,3-dihydro-1H-indol-1-yl)-9H-purin-2-yl]-1,4-cyclohexanediamine

200 mg de produit obtenu au stade 4 de l'exemple 41 est dissous dans 5 ml de méthanol. On ajoute 5 ml d'une solution 2N d'acide chlorhydrique dans le méthanol. On agite 48 heures à température ambiante, puis évapore à sec. Le résidu est chromatographié sur une colonne de silice, éluant dichlorométhane, méthanol, ammoniaque 85/15/1,5. Le produit obtenu est repris dans 10 ml de mélange méthanol/dichlorométhane 50/50, on rajoute 6 ml de solution HCl 1M dans l'acétate d'éthyle, laisse cristalliser, filtre et récupère ainsi 87 mg de produit attendu.

### EXEMPLE 47 : COMPOSITION PHARMACEUTIQUE

On a préparé des comprimés répondant à la formule suivante :
Produit de l'exemple 1 0,2 g
Excipient pour un comprimé terminé à 1 g
   (détail de l'excipient : lactose, talc, amidon, stéarate de magnésium).

### PARTIE PHARMACOLOGIQUE:

Les protéines utilisées dans les tests décrits ci-après sont obtenues selon les méthodes usuelles connues de l'homme du métier.

### 1) Test d'inhibition de l'activité de CIV-CDK (CIV1)

### a) Préparation des réactifs

### -(1)- cocktail enzyme 3X

967 µl Tampon [Tris-HCl 50 mM - 0,1M NaCl - pH 7,5]
- 0,1 % BSA + 30 µl Cdk2 (1 mg/ml)
+ 3 µl CaCiv1 (0,4 mg/ml)

### -(2)- cocktail Inhibiteurs 3X

préparer une gamme 3X d'inhibiteur en 3 % DMSO-Tampon [Tris-HCl 50 mM - 0,1M NaCl - pH 7,5] -0,1 % BSA

EXEMPLE de gamme : 200 ; 100 ; 30 ; 20 ; 10 ; 3 ; 2 ; 1 ; 0,3 ; 0 µM

### -(3)- cocktail ATP 3X

12,2 µl [³³P]ATP + 305 µl Tampon 10X kinase + 700 µl Eau Tampon 10X kinase = 0,5M Tris - 0,1 M MgCl2 - 1 mM Na3VO4
- 10 mM DTT - 15 µM ATP - pH 7,5 + 1 comprimé d'inhibiteurs de protéases (Complete EDTA Free^{™}) pour 5 ml de Tampon).

### b) Réalisation du test

1)- mélanger 30 µl du cocktail enzyme 3X (1) avec 30 µl du cocktail Inhibiteur 3X (2)
2) - ajouter 30 µl du cocktail ATP 3X (3) (début de la réaction)
3)- Incuber 30 mn à Température ambiante (20 à 25°C)
4)- arrêter la réaction en ajoutant 250 µl de Tampon [Tris-HCl 50 mM - 0,1 M NaCl - pH 7,5] - 0,1 % BSA - 25 mM EDTA
5)- répartir 100 µl dans une plaque Coatée avec des anticorps dirigés contre le substrat de la réaction
6)- incuber 60 mn à T° ambiante en agitation douce puis laver 3 fois avec 300 µl
   Tampon [Tris-HCl 50 mM - 0,1 M NaCl - pH 7,5] - 0,05 % Tween20-
9)- Mettre la plaque à sécher 30 mn à 37°C
10)- Mettre à compter dans un compteur à scintillation

### c) Résultats obtenus exprimés en IC 50 exprimés en micromolaire

| Produit | IC50 en micromolaire |
|---|---|
| Exemple 1 | 0,8 |
| Exemple 2 | 0,5 |
| Exemple 5 | 1,1 |
| Exemple 7 | 3,6 |
| Exemple 8 | 1,3 |
| Exemple 15 | 0,3 |
| Exemple 16 | 0,31 |
| Exemple 18 | 3,9 |
| Exemple 19 | 0,78 |
| Exemple 20 | 1,7 |
| Exemple 24 | 0,39 |
| Exemple 27 | 4,3 |
| Exemple 30 | 0,1 |
| Exemple 39 | 3,6 |
| Exemple 40 | 0,2 |
| Exemple 41 | 0,95 |
| Exemple 42 | 3,3 |
| Exemple 43 | 4,4 |
| Exemple 44 | 0,02 |
| Exemple 45 | 1,3 |
| Exemple 46 | 2,00 |

### 2) Test d'inhibition de l'activité de SRC kinase

L'inhibition est déterminée par polarisation de fluorescence, la kinase Abl phosphoryle un peptide détecté par l'ajout d'un anticorps anti phospho-peptide couplé à un marqueur fluorescent.

Le test est fait dans un volume final de 50 µl ; tous les réactifs sont préparés dans un tampon :
■ 25 mHEPES/NaOH pH 7,6
■ 5 mM MgCl2
■ 2 mM MnCl2
■ 50 µM Na2VO4

5 µl de l'inhibiteur concentré 10 fois sont ajoutés à 25 µL d'enzyme (12 U/ml final), (Upstate Biotechnology ref 14-117), après 5 minutes d'incubation à température ambiante 10 µL de PolyGluTyr 4/1 (150 ng/ml final) et 10 µL d'ATP (5 µM final) sont ajoutés. La détection se fait après 20 minutes d'incubation à température ambiante.

### ➢ Résultats obtenus exprimés en pourcentage d'inhibition à 20 µM

| | |
|---|---|
| Exemple 8 | 98 % |
| Exemple 15 | 99 % |
| Exemple 20 | 99 % |
| Exemple 24 | 99 % |
| Exemple 27 | 95 % |
| Exemple 30 | 99 % |
| Exemple 32 | 100 % |
| Exemple 38 | 95 % |

### 3) Test d'inhibition de l'activité de CDK2

L'inhibition de l'activité kinase de la Cyclin Dependent kinase 2 (CDK2) est déterminée par la mesure de la phosphorylation de son peptide substrat.

Le test est fait dans un volume final de 50 µl.

Le tampon d'incubation est le suivant :
■ 50 mHepes/NaOH pH 7,5
■ 10 mM MgCl2
■ 1 mM DTT

5 µl de l'inhibiteur concentré 10 fois sont ajoutés à 25 µl de tampon d'incubation contenant 0,8 U/µL final de l'enzyme ; puis 10 µL de tampon d'incubation contenant 0,0025 mg/ml de substrat peptidique sont ajoutés et enfin 10 µL de tampon d'incubation contenant 2 mM ATP et de l'ATP radio-marqué [33P]ATP (0,25 µCi) sont ajoutés. La réaction est arrêtée après 10 minutes d'incubation à 37°C.

### ➢ Résultats obtenus exprimés en pourcentage d'inhibition à 20 µM

| | |
|---|---|
| Exemple 2 | 97 % |
| Exemple 27 | 97 % |
| Exemple 30 | 99 % |

### 4) Test d'inhibition de l'activité de CDK1

L'inhibition de l'activité kinase de la Cyclin Dependent kinase 1 (CDK1) est déterminée par la mesure de la phosphorylation de son peptide substrat.

Le test est fait dans un volume final de 50 µl.
Le tampon d'incubation est le suivant :
■ 50 mM Tris/HCl pH 7,5
■ 10 mM MgCl2
■ 100 µM Na2VO4
■ 2 mM DTT
■ 40 mM Beta-glycerophosphate
■ 0,1 mg/ml BSA
5 µl de l'inhibiteur concentré 10 fois sont ajoutés à 25 µl de tampon d'incubation contenant 0,04 U/µL final de l'enzyme ; puis 10 µL de tampon d'incubation contenant 12,5 µM final de substrat peptidique sont ajoutés et enfin 10 µL de tampon d'incubation contenant 50 µM ATP et de l'ATP radio-marqué [33P]ATP (0,5 µCi) sont ajoutés. La réaction est arrêtée après 40 minutes d'incubation à température ambiante.

### ➢ Résultats obtenus exprimés en pourcentage d'inhibition à 20 µM

| | |
|---|---|
| Exemple 8 | 93 % |
| Exemple 30 | 99 % |

### 5) Test d'inhibition de l'activité de Abl

L'inhibition de l'activité kinase est déterminée par polarisation de fluorescence, la kinase Abl phosphoryle un peptide détecté par l'ajout d'un anticorps anti-phospho-peptide couplé à un marqueur fluorescent.

Le test est fait dans un volume final de 50 µl ; tous les réactifs sont préparés dans un tampon :
■ 20 mHepes/NaOH pH 7,5
■ 5 mM MgCl2
■ 100 µM Na2VO4
■ 1 mM DTT
■ 100 µM EDTA/NaOH
■ 0,01 % Brij35
5 µl de l'inhibiteur concentré 10 fois sont ajoutés à 25 µL d'enzyme (1000 U/ml final), (Calbiochem ref 102555), après 5 minutes d'incubation à température ambiante 10 µL de PolyGT (400 ng/ml final) et 10 µL d'ATP (5 µM final) sont ajoutés. La détection se fait après 15 minutes d'incubation à 30°C.

### ➢ Résultats obtenus exprimés en pourcentage d'inhibition à 20 µM

| | |
|---|---|
| Exemple 24 | 100 % |
| Exemple 2 | 100 % |
| Exemple 32 | 100 % |

### 6) Test d'inhibition de l'activité de ZAP kinase

L'inhibition de l'activité kinase est déterminée par polarisation de fluorescence, la kinase ZAP phosphoryle un peptide détecté par l'ajout d'un anticorps anti-phospho-peptide couplé à un marqueur fluorescent.

Le test est fait dans un volume final de 50 µl ; tous les réactifs sont préparés dans un tampon :
■ 20 m Tris/HCl pH 7,7
■ 7 mM MnCl2
■ 50 µM Na2VO4
5 µl de l'inhibiteur concentré 10 fois sont ajoutés à 25 µL d'enzyme (0,7 µg/ml final), (Panvera ref P2782), après 5 minutes d'incubation à température ambiante 10 µL de PolyGT (300 ng/ml final) et 10 µL d'ATP (0,25 µM final) sont ajoutés. La détection se fait après 15 minutes d'incubation à température ambiante.

### ➢ Résultats obtenus exprimés en pourcentage d'inhibition à 20 µM

| | |
|---|---|
| Exemple 8 | 98 % |
| Exemple 20 | 95 % |
| Exemple 24 | 96 % |

### 7) Test d'inhibition de l'activité de Caseine kinase II

L'inhibition de l'activité kinase est déterminée par la mesure de la phosphorylation de son peptide substrat.

Le test est fait dans un volume final de 50 µl.
Le tampon d'incubation est le suivant :
■ 30 mM MES pH 6,9
■ 15 mM MgCl2
■ 195 mM KCl
■ 7,25 mM DTT
5 µl de l'inhibiteur concentré 10 fois sont ajoutés à 25 µl de tampon d'incubation contenant 1 U/µL final de l'enzyme (Tebu,SE-124) ; puis 10 µL de tampon d'incubation contenant 60 µM final de substrat peptidique sont ajoutés et enfin 10 µL de tampon d'incubation contenant 25 µM ATP et de l'ATP radio-marqué [33P]ATP (0,25 µCi) sont ajoutés. La réaction est arrêtée après 30 minutes d'incubation à température ambiante.

### ➢ Résultats obtenus exprimés en pourcentage d'inhibition à 20 µM

| | |
|---|---|
| Exemple 8 | 93 % |
| Exemple 30 | 97 % |

### 8) Test d'inhibition de l'activité de CAM kinase II

L'inhibition de l'activité kinase est déterminée par la mesure de la phosphorylation de son peptide substrat.

Le test est fait dans un volume final de 50 µl.
Le tampon d'incubation est le suivant :
■ 20 mM MOPS pH 7,4
■ 5 mM MgCl2
■ 5 mM CaCl2
■ 1 mM DTT
■ 100 µM Na2VO4
■ 25 mM Beta-glycerophosphate
5 µl de l'inhibiteur concentré 10 fois sont ajoutés à 25 µl de tampon d'incubation contenant 0,2 µg/µL final de l'enzyme (Tebu, SE-134) ; puis 10 µL de tampon d'incubation contenant 5 µM final de substrat et 1600 U/ml final de calmoduline sont ajoutés et enfin 10 µL de tampon d'incubation contenant 20 µM ATP et de l'ATP radio-marqué [33P]ATP (0,05 µCi) sont ajoutés. La réaction est arrêtée après 40 minutes d'incubation à température ambiante.

### ➢ Résultats obtenus exprimés en pourcentage d'inhibition à 20 µM

| | |
|---|---|
| Exemple 30 | 98 % |

### 9) Test d'inhibition de l'activité de EGF tyr kinase

L'inhibition de l'activité kinase est déterminée par la mesure de la phosphorylation de son peptide substrat.

Le test est fait dans un volume final de 100 µl.
Le tampon d'incubation est le suivant :
■ 5 mM Hepes/Tris pH 7,4
■ 2 % Glycerol
■ 0,2 % tritonX100
10 µl de l'inhibiteur concentré 10 fois sont ajoutés à 20 µl de tampon d'incubation contenant 1 U/µL final de l'enzyme (Tebu, SE-124) ; puis 30 µL de tampon d'incubation contenant 0,43 mg/ml final de substrat peptidique sont ajoutés ainsi que 20 µl de tampon d'incubation contenant 0,4 µM d'enzyme et enfin 20 µL de tampon 6 mMtris/HCl pH 7,4, 15 mM MgCl2 contenant 10 µM ATP et de l'ATP radio-marqué [33P]ATP (0,25 µCi) sont ajoutés. La réaction est arrêtée après 60 minutes d'incubation à 30°C.

### ➢ Résultats obtenus exprimés en pourcentage

d'inhibition à 20 µM

| | |
|---|---|
| Exemple 20 | 100 % |

### 10) Test d'inhibition de l'activité de AKT

L'inhibition de l'activité kinase est déterminée par polarisation de fluorescence, la kinase AKT phosphoryle un peptide détecté par l'ajout d'un anticorps anti phospho-peptide couplé à un marqueur fluorescent.

Le test est fait dans un volume final de 30 µl; tous les réactifs sont préparés dans un tampon :
■ 50 m HEPES pH 7,5
■ 0,03 % triton X100
■ 10 mM MgCl2
■ 5 % Glycerol
■ 1 mM DTT
10 µl de l'inhibiteur concentré 10 fois sont ajoutés à 5 µL d'enzyme (20 ng final), 5 µL de peptide (1 µM final) et 10 µL d'ATP (80 µM final) sont ajoutés. La détection se fait après 20 minutes d'incubation à 25°C.

### ➢ Résultats obtenus exprimés en IC50 exprimées en micro-molaire

| | |
|---|---|
| Exemple 8 | 0,46 µM |

### 11) Test d'inhibition de l'activité de FAK

L'inhibition de l'activité kinase est déterminée par polarisation de fluorescence, l'autophosphorylation de FAK est détectée par l'ajout d'un anticorps anti-phosphoprotéine couplé à un marqueur fluorescent.

Le test est fait dans un volume final de 30 µl ; tous les réactifs sont préparés dans un tampon :
■ 50 m HEPES pH 7,5
■ 0,03 % triton X100
■ 10 mM MgCl2
■ 5 % Glycerol
■ 1 mM DTT
10 µl de l'inhibiteur concentré 10 fois sont ajoutés à 5 µL d'enzyme (100 ng final), 5 µL de tampon d'incubation et 10 µL d'ATP (1 µM final) sont ajoutés. La détection se fait après 10 minutes d'incubation à 25°C.

### ➢ Résultats obtenus exprimés en IC50 exprimées en micro-molaire

| | |
|---|---|
| Exemple 8 | 2 µM |
| Exemple 20 | 1,6 µM |
| Exemple 30 | 1 µM |

### 12) Test d'inhibition de l'activité de JNK3

L'inhibition de l'activité kinase est déterminée par polarisation de fluorescence, la kinase JNK3 phosphoryle un peptide détecté par l'ajout d'un anticorps anti-phospho peptide couplé à un marqueur fluorescent.

Le test est fait dans un volume final de 30 µl ; tous les réactifs sont préparés dans un tampon :
▩ 50 m HEPES pH 7,5
▩ 0,03 % triton X100
■ 10 mM MgCl2
▩ 5 % Glycerol
□ 1 mM DTT
10 µl de l'inhibiteur concentré 10 fois sont ajoutés à 5 µL d'enzyme (40 ng final), 5 µL de tampon d'incubation contenant 100 ng de substrat et 10 µL d'ATP (6 µM final) sont ajoutés. La détection se fait après 30 minutes d'incubation à 25°C.

### ➢ Résultats obtenus exprimés en IC50 exprimées en micro-molaire

| | |
|---|---|
| Exemple 8 | 0,84 µM |
| Exemple 1 | 0,29 µM |
| Exemple 15 | 0,5 µM |
| Exemple 38 | 0.42 µM |
| Exemple 26 | 0,31 µM |
| Exemple 27 | 0,29 µM |

### 13) Test d'inhibition de l'activité de GSK3beta

L'inhibition de l'activité kinase est déterminée par la mesure de la phosphorylation de son peptide substrat.

Le test est fait dans un volume final de 40 µl ; tous les réactifs sont préparés dans un tampon :
■ 50 m HEPES pH 7,5
■ 0,03 % triton X100
■ 10 mM MgCl2
■ 5 % Glycerol
■ 1 mM DTT
10 µl de l'inhibiteur concentré 10 fois sont ajoutés à 5 µL d'enzyme (20 ng final), 5 µL de peptide (1 µM final) puis 2,5 µL de tampon d'incubation contenant 16 µM ATP et de 2,5 µL d'ATP radio-marqué [33P]ATP (50 nCi) sont ajoutés. Après 15 minutes d'incubation à température ambiante, 10 µl de tampon d'incubation contenant 1 µM de substrat. La réaction est arrêtée après 30 minutes d'incubation à 30°C.

### ➢ Résultats obtenus exprimés en IC50 exprimées en micro-molaire

| | |
|---|---|
| Exemple 2 | 0,6 µM |
| Exemple 26 | 1,3 µM |
| Exemple 30 | 1,5 µM |

### 14) Test d'inhibition de l'activité de PLK1

L'inhibition de l'activité kinase est déterminée par la mesure de la phosphorylation de son peptide substrat.

Le test est fait dans un volume final de 40 µl ; tous les réactifs sont préparés dans un tampon :
■ 50 m HEPES pH 7,5
■ 0,03 % triton X100
■ 10 mM MgCl2
■ 5 % Glycerol
■ 1 mM DTT
10 µl de l'inhibiteur concentré 10 fois sont ajoutés à 5 µL d'enzyme (75 ng final), 5 µL de peptide (1 µM final) puis 2,5 µL de tampon d'incubation contenant 40 µM ATP et de 2,5 µL d'ATP radio-marqué [33P]ATP (500 nCi) sont ajoutés. Après 15 minutes d'incubation à température ambiante, 10 µl de tampon d'incubation contenant 192 nM de substrat. La réaction est arrêtée après 60 minutes d'incubation à 37°C.

### ➢ Résultats obtenus exprimés en IC50 exprimées en micro-molaire

| | |
|---|---|
| Exemple 18 | 0,16 µM |
| Exemple 1 | 0,21 µM |

### 15) Détermination de la concentration minimale inhibitrice pour tester la sensibilité des champignons aux antifongiques en milieu liquide: micro méthode

**Principe :** Un nombre constant de cellules d'une souche donnée est mis en présence de concentrations croissantes d'un antifongique, dans des conditions pris du NCCLS (National Committee for Clinical Laboratory Standards, 1997. Reference method for broth dilution antifungal susceptibility testing of yeasts. Approved Standard M27-A. NCCLS, Villanova, Pa.) ; la concentration minimale avec laquelle on observe une réduction du trouble visible de la croissance cellulaire (au moins 80 % par rapport à un contrôle sans produit) est la Concentration Minimale Inhibitrice (CMI) de l'antifongique vis-à-vis de la souche testée.

Supplémenter le milieu RPMI 1640 (liquide) sans L-glutamine avec de la L-glutamine (0,3 g/l ou 10,5 ml d'une solution à 200 mM) et tamponner avec 34,54 g/l (0,165 M) de M morpholinepropanesulfonic acid (MOPS). Stériliser le milieu par filtration. Distribuer 100 µl de milieu RPMI dans chaque puits d'une microplaque à 96 puits. Distribuer le volume adéquat de la solution d'antifongique dans la première colonne de la microplaque et compléter avec du milieu à 200 µl. Diluer de 2 en 2 de façon à établir une gamme de 11 concentrations dans chaque ligne de la microplaque. Le 12ème puits de chaque rangée servira de témoin de croissance. Préparer la suspension cellulaire à partir d'une culture (liquide ou agar) ou d'une ampoule congelée, la diluer en milieu RPMI pour obtenir une suspension cellulaire à 5x10³ - 2x10⁴ cellules/ml. Distribuer 100 µl de la suspension cellulaire dans la microplaque. La lecture de la CMI pour toutes espèces de Candida est faite après 24 - 48 h, pour Cryptococcus et Aspergillus après 48 - 72 h d'incubation à 37°C en atmosphère normale. La lecture de la CMI est faite par lecture visuelle en déterminant le puits qui contient la plus faible dose d'antifongique qui provoque une inhibition d'au moins 80 % de la croissance du champignon.

### Résultats des CMI

➢ Exemple 5 : CMI Candida glabrata 64 µg/ml ; Candida albicans 64 µg/ml
➢ Exemple 8 : CMI Candida glabrata 16 µg/ml ; Candida albicans 16 µg/ml ; Aspergillus fumigatus 32 µg/ml
➢ Exemple 19 : CMI Candida glabrata 32 µg/ml ; Candida albicans 16 µg/ml ; Aspergillus fumigatus 32 µg/ml
➢ Exemple 20 : CMI Candida glabrata 16 µg/ml ; Candida albicans 32 µg/ml ; Aspergillus fumigatus 32 µg/ml
➢ Exemple 30 : CMI Candida glabrata 64 µg/ml ; Candida albicans 32 µg/ml
➢ Exemple 32 : CMI Candida glabrata 64 µg/ml ; Candida albicans 64 µg/ml

## Revendications

1. Produits de formule (I) : 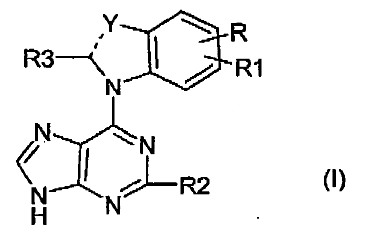 dans laquelle:
Y représente N, O, S, CHR3 ou =CR3
la ligne en pointillé sur le cycle indiquant que la liaison correspondante est simple ou double,
R et R1, identiques ou différents, représentent hydrogène, halogène, hydroxyle, alkyle, alcoxy, cyano, NO2, NR4R5, trifluorométhyle, trifluorométhoxy, aryle, hétéroaryle, -S(O)n-NR4R5, acyle, -NH-CO-alkyle ou -NH-CO-NH-phényle dans lesquels les radicaux alkyle et phényle sont éventuellement substitués par un ou plusieurs radicaux choisis parmi thiényle et phényle lui-même éventuellement substitué, ces radicaux phényle étant eux-même éventuellement substitués par un ou plusieurs radicaux choisis parmi les atomes d'halogène et les radicaux -NH2, -NHAlk et -N(Alk)2,
n représente un entier de 0 à 2,
R3 représente hydrogène, halogène, alkyle, cyano, N02, NR4R5, trifluorométhyle, aryle,
R2 représente un radical R4, OR4, SR4 ou NR4R5 dans lesquels R4 représente un atome d'hydrogène ou un radical alkyle, cycloalkyle ou aryle,
NR4R5 étant tel que soit R4 et R5, identiques ou différents, sont choisis parmi les valeurs de R4 soit R4 et R5 forment ensemble avec l'atome d'azote auxquels ils sont liés un radical cyclique renfermant 4 à 6 chaînons renfermant un ou plusieurs hétéroatomes identiques ou différents choisis parmi N, 0 et S,
tous les radicaux alkyle, alcoxy, cycloalkyle, aryle et hétérocyclique définis ci-dessus étant éventuellement substitués par un ou plusieurs radicaux choisis parmi les atomes d'halogène, les radicaux hydroxyle, cyano, trifluorométhyle, trifluorométhoxy, alcoxy, aryle, hétérocyclique, les radicaux à fonction acide et isostère d'acide et les radicaux -NHR4, -NalkR4, -COR4, -COOR4, CONalkR4 et-CONHR4 dans lesquels R4 a la signification indiquée ci-dessus et alk représente un radical alkyle, tous les radicaux aryle et hétérocyclique définis ci-dessus étant de plus éventuellement substitués par un ou plusieurs radicaux alkyle, hydroxyalkyle et phénylalkyle, tous les radicaux aryle définis ci-dessus étant de plus éventuellement substitués par un radical dioxol,
tous les radicaux alkyle et alcoxy définis ci-dessus étant linéaires ou ramifiés et renfermant au plus 6 atomes de carbone,
tous les radicaux cycloalkyle définis ci-dessus renfermant au plus 6 atomes de carbone,
étant entendu que R,R1,R2,R3 ne sont pas tous hydrogène, lesdits produits de formule (I) étant sous toutes les formes isomères possibles racémiques, énantiomères et diastéréo+isomères, ainsi que les sels d'addition avec les acides minéraux et organiques ou avec les bases minérales et organiques pharmaceutiquement acceptables desdits produits de formule (I).

2. Produits de formule (I) : 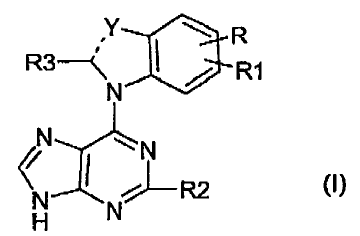 dans laquelle:
Y représente N, O, S, CHR3 ou =CR3
la ligne en pointillé sur le cycle indiquant que la liaison correspondante est simple ou double,
R et R1, identiques ou différents, représentent hydrogène, halogène, hydroxyle, alkyle, alcoxy, cyano, NO2, NR4R5, trifluorométhyle, trifluorométhoxy, aryle, hétéroaryle,
-S (O) n-NR4R5 ,
n représente un entier de 0 à 2,
R3 représente hydrogène, halogène, alkyle, cyano, NO2, NR4R5, trifluorométhyle, aryle,
R2 représente un radical R4, OR4, SR4 ou NR4R5 dans lesquels R4 représente un atome d'hydrogène ou un radical alkyle, cycloalkyle ou aryle,
NR4R5 étant tel que soit R4 et R5, identiques ou différents, sont choisis parmi les valeurs de R4 soit R4 et R5 forment ensemble avec l'atome d'azote auxquels ils sont liés un radical cyclique renfermant 4 à 6 chaînons renfermant un ou plusieurs hétéroatomes identiques ou différents choisis parmi N, 0 et S,
tous les radicaux alkyle, alcoxy, cycloalkyle, aryle et hétérocyclique définis ci-dessus étant éventuellement substitués par un ou plusieurs radicaux choisis parmi les atomes d'halogène, les radicaux hydroxyle, cyano, trifluorométhyle, trifluorométhoxy, alcoxy, aryle, hétérocyclique, les radicaux à fonction acide et isostère d'acide et les radicaux -NHR4, -NalkR4, -COR4, -COOR4, CONalkR4 et-CONHR4 dans lesquels R4 a la signification indiquée ci-dessus et alk représente un radical alkyle, tous les radicaux aryle et hétérocyclique définis ci-dessus étant de plus éventuellement substitués par un ou plusieurs radicaux alkyle, hydroxyalkyle et phénylalkyle, tous les radicaux aryle définis ci-dessus étant de plus éventuellement substitués par un radical dioxol,
tous les radicaux alkyle et alcoxy définis ci-dessus étant linéaires ou ramifiés et renfermant au plus 6 atomes de carbone,
tous les radicaux cycloalkyle définis ci-dessus renfermant au plus 6 atomes de carbone,
lesdits produits de formule (I) étant sous toutes les formes isomères possibles racémiques, énantiomères et diastéréo+isomères, ainsi que les sels d'addition avec les acides minéraux et organiques ou avec les bases minérales et organiques pharmaceutiquement acceptables desdits produits de formule (I).

3. Produits de formule (I) telle que définie à la revendication 1 répondant à la formule (Ia) : 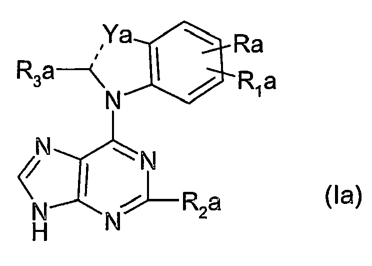 dans laquelle:
Ya représente N, O, S, CHR3a ou =CR3a
la ligne en pointillé sur le cycle indiquant que la liaison correspondante est simple ou double,
Ra et R1a, identiques ou différents, représentent hydrogène, halogène, hydroxyle, alkyle, alcoxy, cyano, N02, NR4aR5a, trifluorométhyle, trifluorométhoxy, phényle, hétéroaryle,
-S (O) n-NR4aR5a,
n représente un entier de 0 à 2,
R3a représente hydrogène, halogène, alkyle, cyano, NO2, amino, alkylamino, dialkylamino, trifluorométhyle et phényle,
R2a représente un radical R4a, OR4a, SR4a ou NR4aR5a dans lesquels R4a représente un atome d'hydrogène ou un radical alkyle, cycloalkyle ou phényle,
NR4aR5a étant tel que soit R4a et R5a, identiques ou différents, sont choisis parmi les valeurs de R4a soit R4a et R5a forment ensemble avec l'atome d'azote auxquels ils sont liés un radical pipéridyle, morpholinyle, pyrrolidinyle ou pipérazinyle éventuellement substitué, tous les radicaux alkyle, alcoxy, cycloalkyle, phényle, phénylalkyle et hétérocyclique (comme ceux formés par NR4aR5a) définis ci-dessus étant éventuellement substitués par un ou plusieurs radicaux choisis parmi les atomes d'halogène, les radicaux hydroxyle, cyano, trifluorométhyle, trifluorométhoxy, alcoxy, phényle, hétérocyclique éventuellement substitué sur N ou C par un radical carboxy libre, salifié ou estérifié par un radical alkyle, les radicaux SO3H, PO(OH)2, NH-SO2-CF3, NH-SO2-NH-V et NH-SO2-NH-CO-V dans lesquels V représente un radical phényle, alkyle ou alkényle, les radicaux alkényle étant linéaires ou ramifiés renfermant au plus 6 atomes de carbone, et les radicaux -NalkR4a, -NHR4a, - COR4a, -COOR4a , -CONalkR4a et-CONHR4a dans lesquels R4a a la signification indiquée ci-dessus et alk représente un radical alkyle,
tous les radicaux phényle et hétérocyclique définis ci-dessus étant de plus éventuellement substitués par un ou plusieurs radicaux alkyle, hydroxyalkyle et phénylalkyle, tous les radicaux phényle définis ci-dessus étant de plus éventuellement substitués par un radical dioxol,
tous les radicaux alkyle et alcoxy définis ci-dessus étant linéaires ou ramifiés et renfermant au plus 6 atomes de carbone,
tous les radicaux cycloalkyle définis ci-dessus renfermant 5 ou 6 atomes de carbone,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Ia).

4. Produits de formule (I) telle que définie à la revendication 1 répondant à la formule (Ib) : 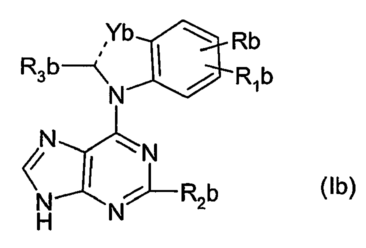 dans laquelle:
Yb représente N, CHR3b ou =CR3b
la ligne en pointillé sur le cycle indiquant que la liaison correspondante est simple ou double,
Rb et R1b, identiques ou différents, représentent hydrogène, halogène, hydroxyle, alkyle, alcoxy, cyano, NO2, trifluorométhyle, trifluorométhoxy, phényle,
-S (O) n-NR4bR5b,
n représente un entier de 0 à 2,
R3b représente hydrogène, halogène, alkyle, cyano, NO2, amino, alkylamino, dialkylamino, trifluorométhyle et phényle,
R2b représente un radical R4b ou NR4bR5b dans lesquels R4b représente un atome d'hydrogène ou un radical alkyle, cycloalkyle ou phényle,
NR4bR5b étant tel que soit R4b et R5b, identiques ou différents, sont choisis parmi les valeurs de R4b soit R4b et R5b forment ensemble avec l'atome d'azote auxquels ils sont liés un radical pipéridyle, morpholinyle, pyrrolidinyle éventuellement substitués,
tous les radicaux alkyle, alcoxy, cycloalkyle, phényle, phénylalkyle, hétérocyclique comme pipéridyle, morpholinyle et pyrrolidinyle définis ci-dessus étant éventuellement substitués par un ou deux radicaux choisis parmi les atomes d'halogène, les radicaux hydroxyle, cyano, trifluorométhyle, trifluorométhoxy, alcoxy, phényle, tétrahydropyranyle, pipéridyle eux-mêmes éventuellement substitués sur N ou C par un radical carboxy libre, salifié ou estérifié par un radical alkyle), et les radicaux -NalkR4a, -NHR4a et -COOR4a dans lesquels R4a a la signification indiquée ci-dessus et alk représente un radical alkyle,
tous les radicaux phényle et hétérocyclique définis ci-dessus étant de plus éventuellement substitués par un ou plusieurs radicaux alkyle, hydroxyalkyle et phénylalkyle, tous les radicaux alkyle et alcoxy définis ci-dessus étant linéaires ou ramifiés et renfermant au plus 4 atomes de carbone,
tous les radicaux cycloalkyle définis ci-dessus renfermant 5 ou 6 atomes de carbone,
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Ib).

5. Produits de formule (I) telle que définie à la revendication 1 répondant à la formule (Ic) : 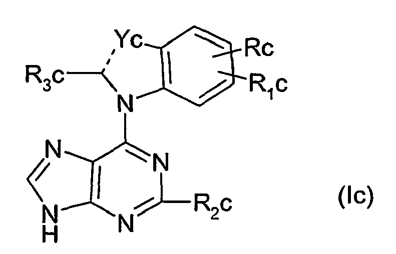 dans laquelle:
Yc représente N, CH2 ou CH=,
la ligne en pointillé sur le cycle indiquant que la liaison correspondante est simple ou double,
Rc et R1c, identiques ou différents, représentent hydrogène, halogène, hydroxyle, alkyle, alcoxy, phénylalcoxy, phénylalkyle, cyano, NO2, trifluorométhyle, trifluorométhoxy, phényle, -S(O)n-NH2 éventuellement substitué sur l'atome d'azote par un ou deux radicaux alkyle et n représente un entier de 0 à 2,
R3c représente hydrogène, halogène, alkyle, cyano, NO2, trifluorométhyle et phényle,
R2c représente un radical NR4cR5c dans lesquels soit R4c et R5c, identiques ou différents, sont tels que R4c représente un atome d'hydrogène ou un radical alkyle, cycloalkyle, phényle ou phénylalkyle,
les radicaux alkyle, cycloalkyle, phényle et phénylalkyle étant éventuellement substitués par un ou plusieurs radicaux choisis parmi hydroxyle, amino, carboxy libre, salifié ou estérifié par un radical alkyle, tétrahydropyranyle, pipéridyle éventuellement substitué sur N ou C par un radical carboxy libre, salifié ou estérifié par un radical alkyle,
et R5c représente un atome d'hydrogène ou un radical alkyle,
soit R4c et R5c forment ensemble avec l'atome d'azote auxquels ils sont liés un radical pipéridyle, morpholinyle ou pyrrolidinyle, ces radicaux éventuellement substitués par un radical alkyle, hydroxyalkyle, amino, monoalkylamino ou dialkylamino, tous les radicaux alkyle et alcoxy définis ci-dessus étant linéaires ou ramifiés renfermant au plus 4 atomes de carbone
lesdits produits de formule (Ic) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Ic).

6. Produits de formule (I) telle que définie à la revendication 1 répondant à la formule (Id) : 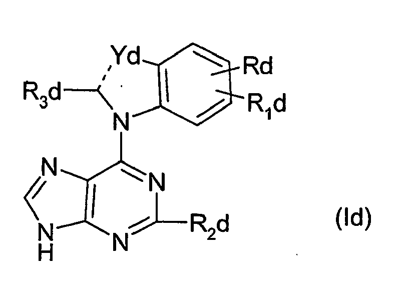 dans laquelle:
Yd représente N, CH2 ou CH=,
la ligne en pointillé sur le cycle indiquant que la liaison correspondante est simple ou double,
Rd et R1d, identiques ou différents, représentent hydrogène, halogène, alkyle, alcoxy, phénylalcoxy, NO2, dialkylaminosulfonyle,-NH2, trifluorométhyle, -CO-CH3, -NH-CO-alkyle ou -NH-CO-NH-phényle dans lesquels le radical alkyle est éventuellement substitué par un radical thiényle ou phényle et le radical phényle est éventuellement substitué par un ou plusieurs radicaux choisis parmi les atomes d'halogène et les radicaux -NH2, -NHAlk et -N(Alk)2,
R3d représente hydrogène ou alkyle,
R2d représente un radical NR4dR5d dans lesquels soit R4d et R5d, identiques ou différents, sont tels que R4d représente un atome d'hydrogène, un radical alkyle linéaire ou ramifié renfermant 1 à 4 atomes de carbone et éventuellement substitué par un ou deux hydroxyle, un radical cycloalkyle éventuellement substitué par un radical amino ou hydroxyle, phényle ou phénylalkyle (1à4C) avec phényle éventuellement substitué par un radical carboxy libre, salifié ou estérifié par un radical alkyle, tétrahydropyranalkyle (ex 28), piperidylalkyle (ex 31, 36) éventuellement substitué sur N ou C par un radical carboxy
et R5d représente un atome d'hydrogène ou un radical alkyle,
soit R4d et R5d forment ensemble avec l'atome d'azote auxquels ils sont liés un radical pipéridyle éventuellement substitué par un radical amino, morpholinyle, pyrrolidinyle (ex 34) éventuellement substitué par un radical hydroxyalkyle,
tous les radicaux alkyle et alcoxy définis ci-dessus étant linéaires ou ramifiés renfermant au plus 4 atomes de carbone
lesdits produits de formule (Id) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Id).

7. Produits de formule (I) telle que définie à la revendication 1 répondant à la formule (Id) : 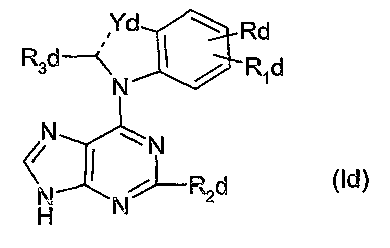 dans laquelle:
Yd représente N, CH2 ou CH=,
la ligne en pointillé sur le cycle indiquant que la liaison correspondante est simple ou double,
Rd et R1d, identiques ou différents, représentent hydrogène, halogène, alkyle, alcoxy, phénylalcoxy, NO2, dialkylaminosulfonyle
R3d représente hydrogène ou alkyle,
R2d représente un radical NR4dR5d dans lesquels soit R4d et R5d, identiques ou différents, sont tels que R4d représente un atome d'hydrogène, un radical alkyle linéaire ou ramifié renfermant 1 à 4 atomes de carbone et éventuellement substitué par un ou deux hydroxyle, un radical cycloalkyle éventuellement substitué par un radical amino ou hydroxyle, phényle ou phénylalkyle (1à4C) avec phényle éventuellement substitué par un radical carboxy libre, salifié ou estérifié par un radical alkyle, tétrahydropyranalkyle (ex 28), piperidylalkyle (ex 31, 36) éventuellement substitué sur N ou C par un radical carboxy
et R5d représente un atome d'hydrogène ou un radical alkyle,
soit R4d et R5d forment ensemble avec l'atome d'azote auxquels ils sont liés un radical pipéridyle éventuellement substitué par un radical amino, morpholinyle, pyrrolidinyle (ex 34) éventuellement substitué par un radical hydroxyalkyle,
tous les radicaux alkyle et alcoxy définis ci-dessus étant linéaires ou ramifiés renfermant au plus 4 atomes de carbone
lesdits produits de formule (Id) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Id).

8. Produits de formule (I) telle que définie à la revendication 1 dont les noms suivent:
- le Dichlorhydrate de trans-N-[6-(5,6-dichloro-2,3-dihydro-1H-indol-1-yl)-9H-purin-2-yl]-1,4-cyclohexanediamine
- le Dichlorhydrate de trans-N-[6-(1H-benzimidazol-1-yl)-9H-purin-2-yl]-1,4-cyclohexanediamine
- le Trans-N-[6-(5,6-diméthyl-1H-benzimidazol-1-yl)-9H-purin-2-yl]-1,4-cyclohexanediamine
- le Chlorhydrate de trans-N-[6-(5,6-dichloro-1H-benzimidazol-1-yl)-9H-purin-2-yl]-1,4-cyclohexanediamine
- le Dichlorhydrate de trans-N-[6-(5-méthoxy-1H-benzimidazol-1-yl)-9H-purin-2-yl]-1,4-cyclohexanediamine
- le Dichlorhydrate de trans-N-[6-(1H-indol-1-yl)-9H-purin-2-yl]-1,4-cyclohexanediamine
- le Trans-N-[6-[6-(phénylméthoxy)-1H-benzimidazol-1-yl] - 9H-purin-2-yl]-1,4-cyclohexanediamine
- le Trans-N-[6-[5-(phénylméthoxy)-1H-benzimidazol-1-yl]-9H-purin-2-yl]-1,4-cyclohexanediamine
- le Trans-4-[[6-(1H-benzimidazol-1-yl)-9H-purin-2-yl]amino]-cyclohexanol
- le Dichlorhydrate de trans-N-[6-(2,3-dihydro-5-nitro-1H-indol-1-yl)-9H-purin-2-yl]- 1,4-cyclohexanediamine
- le Trans-N-[6-[2,3-dihydro-6-(trifluorométhyl)-1H-indol-1-yl) -9H-purin-2-yl]-1,4-cyclohexanediamine ( Ex 40)
- le Trans-N-[1-[2-[(4-aminocyclohexyl)amino]-9H-purin-6-yl]-2,3-dihydro-1H-indol-5-yl]-2-thiophèneacétamide (Ex 41)
- le Trans-N-[6-(6-nitro-2,3-dihydro-1H-indol-1-yl)-9H-purin-2-yl]-1,4-cyclohexanediamine (Ex *44*)

9. Procédé de préparation des produits de formule (I), telle que définie à la revendication 1, **caractérisé en ce que** l'on soumet le composé de formule (II) : 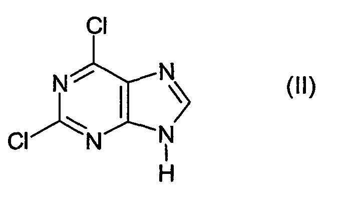 que l'on soumet à l'action d'un composé de formule (III) : 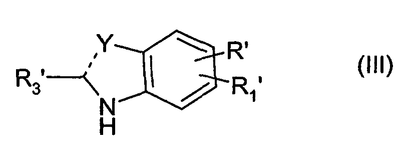 dans laquelle R', R1'et R3' ont les significations indiquées respectivement à la revendication 1 pour R, R1 et R3, dans lesquelles les éventuelles fonctions réactives sont éventuellement protégées par des groupements protecteurs, et Y a la signification indiquée à la revendication 1,
pour obtenir un produit de formule (IV) : 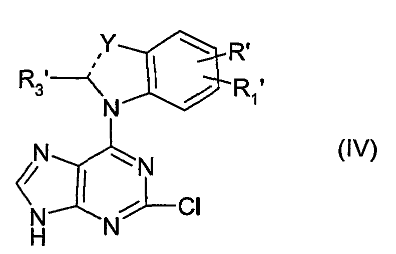 dans laquelle R', R1', R3' et Y ont les significations indiquées ci-dessus,
produit de formule (IV) que l'on soumet à une réaction avec un composé de formule (V) :
R2'-H (V)
dans laquelle R2' a la signification indiquée à la revendication 1 pour R2 dans laquelle les éventuelles fonctions réactives sont éventuellement protégées par des groupements protecteurs,
pour obtenir un produit de formule (I') : 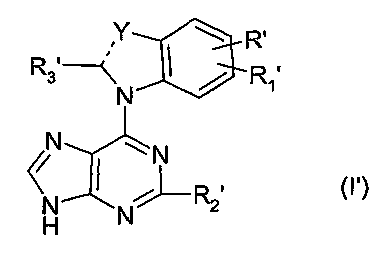 dans laquelle R', R1', R2', R3' et Y' ont les significations indiquées ci-dessus,
les produits de formule (I') ayant la signification indiquée à la revendication 1 pour les produits de formule (I) dans laquelle les éventuelles fonctions réactives sont éventuellement protégées par des groupements protecteurs,
produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

10. A titre de médicaments, les produits de formule (I) telle que définie à l'une quelconque des revendications 1 à 5 ainsi que les sels d'addition avec les acides minéraux et organiques ou avec les bases minérales et organiques pharmaceutiquement acceptables desdits produits de formule (Id).

11. A titre de médicaments, les produits de formule (I) telle que définie à la revendication 6 ainsi que les sels d'addition avec les acides minéraux et organiques ou avec les bases minérales et organiques pharmaceutiquement acceptables.

12. Les compositions pharmaceutiques contenant à titre de principe actif, l'un au moins des médicaments tels que définis à la revendication 8 ou 9.

13. Utilisation des produits de formule (I) telle que définie à l'une quelconque des revendications 1 à 6 et/ou de leurs sels pharmaceutiquement acceptables, pour la préparation de médicaments destinés à la prévention ou au traitement de maladies fongiques.

14. Utilisation des produits de formule (I) telle que définie à l'une quelconque des revendications 1 à 6 et/ou de leurs sels pharmaceutiquement acceptables, pour la préparation de médicaments destinés à la prévention ou au traitement de maladies fongiques telles que notamment les candidoses, aspergilloses, histoplasmoses et coccidoidoses.

15. Utilisation des produits de formule (I) tels que définis à l'une quelconque des revendications 1 à 6 et/ou de leurs sels pharmaceutiquement acceptables pour la préparation de médicaments destinés à la prévention ou au traitement de maladies causées par Candida albicans.

16. Utilisation des produits de formule (I) tels que définis à l'une quelconque des revendications 1 à 6 et/ou de leurs sels pharmaceutiquement acceptables pour la préparation de médicaments destinés à la prévention ou au traitement de la candidose systémique.

17. Produits de formule (I) telle que définie à la revendication 1 ayant des propriétés antifongiques comme inhibiteurs de protéines kinases CIV1 de Candida albicans.

18. Compositions pharmaceutiques renfermant à titre de principe actif au moins un inhibiteur de protéines kinases CIV1 de Candida albicans tels que définis à la revendication 17.

19. A titre de produits industriels nouveaux, les composés de formule (IV).

20. Compositions pharmaceutiques selon la revendication 16 **caractérisées en ce qu'**elles sont utilisées comme médicaments antimitotiques, en particulier pour la chimiothérapie de cancers ou encore pour le traitement de psoriasis, de parasitoses telles que celles dues à des champignons ou à des protistes ou de la maladie d'Alzheimer.

21. Compositions pharmaceutiques selon la revendication 16 **caractérisées en ce qu'**elles sont utilisées comme médicaments antineurodégénératifs notamment anti-apoptose neuronale.

22. Utilisation des produits de formule (I) telle que définie aux revendications 1 à 6, pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

23. Utilisation des produits de formule (I) et/ou de leurs sels pharmaceutiquement acceptables tels que définis aux revendications 1 à 6 pour la préparation de médicaments destinés à la prévention ou au traitement de maladies liées à un dérèglement de la sécrétion et/ou de l'activité de protéines tyrosines kinases et des sérine/thréonine kinases.

24. Utilisation des produits de formule (I) et/ou de leurs sels pharmaceutiquement acceptables tels que définis aux revendications 1 à 6 pour la préparation de médicaments destinés au traitement ou à la prévention de l'immunité, de l'infection, de l'allergie, et des maladies auto-immunes.

25. Utilisation des produits de formule (I) et/ou de leurs sels pharmaceutiquement acceptables tels que définis aux revendications 1 à 6 pour la préparation de médicaments destinés au traitement ou à la prévention de maladies telles que les maladies prolifératives, le cancer, la resténose, l'inflammation, les allergies ou les maladies cardiovasculaires.

## Claims

1. Products of formula (I): 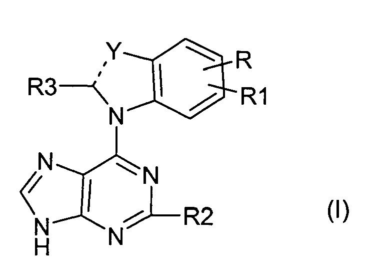 in which:
Y represents N, O, S, CHR3 or =CR3
the dashed line on the ring indicating that the corresponding bond is single or double,
R and R1, which may be identical or different, represent hydrogen, halogen, hydroxyl, alkyl, alkoxy, cyano, NO₂, NR4R5, trifluoromethyl, trifluoromethoxy, aryl, heteroaryl, -S(O)n-NR4R5, acyl, -NH-CO-alkyl or -NH-CO-NH-phenyl in which the alkyl and phenyl radicals are optionally substituted with one or more radicals chosen from thienyl and phenyl, itself optionally substituted, these phenyl radicals themselves being optionally substituted with one or more radicals chosen from halogen atoms and the radicals -NH₂, -NHAlk and -N (Alk) ₂,
n represents an integer of 0 to 2,
R3 represents hydrogen, halogen, alkyl, cyano, NO₂, NR4R5, trifluoromethyl, aryl,
R2 represents a radical R4, OR4, SR4 or NR4R5 in which R4 represents a hydrogen atom or an alkyl, cycloalkyl or aryl radical,
NR4R5 being such that either R4 and R5, which may be identical or different, are chosen from the values for R4 or R4 and R5 form, together with the nitrogen atom to which they are attached, a cyclic radical containing 4 to 6 ring members containing one or more hetero atoms, which may be identical or different, chosen from N, 0 and S,
all the alkyl, alkoxy, cycloalkyl, aryl and heterocyclic radicals defined above being optionally substituted with one or more radicals chosen from halogen atoms, hydroxyl, cyano, trifluoromethyl, trifluoromethoxy, alkoxy, aryl and heterocyclic radicals, radicals with an acid or acid isostere function and the radicals -NHR4, -NalkR4, -COR4, -COOR4, CONalkR4 and -CONHR4 in which R4 has the meaning given above and alk represents an alkyl radical,
all the aryl and heterocyclic radicals defined above also being optionally substituted with one or more alkyl, hydroxyalkyl and phenylalkyl radicals,
all the aryl radicals defined above also being optionally substituted with a dioxol radical,
all the alkyl and alkoxy radicals defined above being linear or branched and containing at most 6 carbon atoms,
all the cycloalkyl radicals defined above containing at most 6 carbon atoms,
it being understood that R, R1, R2, R3 are not all hydrogen,
said products of formula (I) being in all the possible racemic, enantiomeric and diastereoisomeric isomer forms, and also the pharmaceutically acceptable addition salts with inorganic and organic acids or with inorganic and organic bases of said products of formula (I).

2. Products of formula (I): 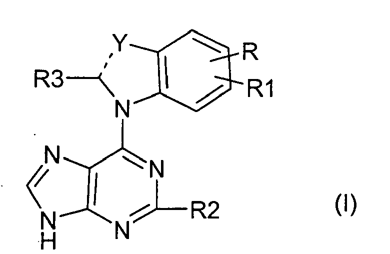 in which:
Y represents N, O, S, CHR3 or =CR3
the dashed line on the ring indicating that the corresponding bond is single or double,
R and R1, which may be identical or different, represent hydrogen, halogen, hydroxyl, alkyl, alkoxy, cyano, NO₂, NR4R5, trifluoromethyl, trifluoromethoxy, aryl, heteroaryl,
-S(O)n-NR4R5,
n represents an integer of 0 to 2,
R3 represents hydrogen, halogen, alkyl, cyano, NO₂, NR4R5, trifluoromethyl, aryl,
R2 represents a radical R4, OR4, SR4 or NR4R5 in which R4 represents a hydrogen atom or an alkyl, cycloalkyl or aryl radical,
NR4R5 being such that either R4 and R5, which may be identical or different, are chosen from the values for R4 or R4 and R5 form, together with the nitrogen atom to which they are attached, a cyclic radical containing 4 to 6 ring members containing one or more hetero atoms, which may be identical or different, chosen from N, 0 and S,
all the alkyl, alkoxy, cycloalkyl, aryl and heterocyclic radicals defined above being optionally substituted with one or more radicals chosen from halogen atoms, hydroxyl, cyano, trifluoromethyl, trifluoromethoxy, alkoxy, aryl and heterocyclic radicals, radicals with an acid or acid isostere function and the radicals -NHR4, -NalkR4, -COR4, -COOR4, CONalkR4 and -CONHR4 in which R4 has the meaning given above and alk represents an alkyl radical,
all the aryl and heterocyclic radicals defined above also being optionally substituted with one or more alkyl, hydroxyalkyl and phenylalkyl radicals,
all the aryl radicals defined above also being optionally substituted with a dioxol radical,
all the alkyl and alkoxy radicals defined above being linear or branched and containing at most 6 carbon atoms,
all the cycloalkyl radicals defined above containing at most 6 carbon atoms,
said products of formula (I) being in all the possible racemic, enantiomeric and diastereoisomeric isomer forms, and also the pharmaceutically acceptable addition salts with inorganic and organic acids or with inorganic and organic bases of said products of formula (I).

3. Products of formula (I) as defined in Claim 1, corresponding to formula (Ia): 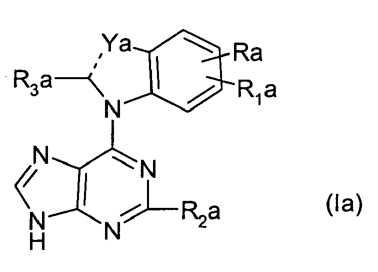 in which:
Ya represents N, O, S, CHR3a or =CR3a
the dashed line on the ring indicating that the corresponding bond is single or double,
Ra and R1a, which may be identical or different, represent hydrogen, halogen, hydroxyl, alkyl, alkoxy, cyano, NO₂, NR4aR5a, trifluoromethyl, trifluoromethoxy, phenyl, heteroaryl,
-S(O)n-NR4aR5a,
n represents an integer of 0 to 2,
R3a represents hydrogen, halogen, alkyl, cyano, NO₂, amino, alkylamino, dialkylamino, trifluoromethyl and phenyl,
R2a represents a radical R4a, OR4a, SR4a or NR4aR5a in which R4a represents a hydrogen atom or an alkyl, cycloalkyl or phenyl radical,
NR4aR5a being such that either R4a and R5a, which may be identical or different, are chosen from the values for R4a, or R4a and R5a form, together with the nitrogen atom to which they are attached, an optionally substituted piperidyl, morpholinyl, pyrrolidinyl or piperazinyl radical, all the alkyl, alkoxy, cycloalkyl, phenyl, phenylalkyl and heterocyclic radicals (such as those formed by NR4aR5a) defined above being optionally substituted with one or more radicals chosen from halogen atoms, hydroxyl, cyano, trifluoromethyl, trifluoromethoxy, alkoxy and phenyl radicals, a heterocyclic radical optionally substituted on N or C with a carboxyl radical which is free, salified or esterified with an alkyl radical, the radicals SO₃H, PO(OH)₂, NH-SO₂-CF₃, NH-SO₂-NH-V and NH-SO₂-NH-CO-V in which V represents a phenyl, alkyl or alkenyl radical, the alkenyl radicals being linear or branched containing at most 6 carbon atoms, and the radicals -NalkR4a, -NHR4a, -COR4a, -COOR4a , -CONalkR4a and -CONHR4a in which R4a has the meaning indicated above and alk represents an alkyl radical,
all the phenyl and heterocyclic radicals defined above also being optionally substituted with one or more alkyl, hydroxyalkyl or phenylalkyl radicals,
all the phenyl radicals defined above also being optionally substituted with a dioxol radical,
all the alkyl and alkoxy radicals defined above being linear or branched and containing at most 6 carbon atoms,
all the cycloalkyl radicals defined above containing 5 or 6 carbon atoms,
said products of formula (Ia) being in all the possible racemic, enantiomeric and diastereoisomeric isomer forms, and also the pharmaceutically acceptable addition salts with inorganic and organic acids or with inorganic and organic bases of said products of formula (Ia).

4. Products of formula (I) as defined in Claim 1, corresponding to formula (Ib: 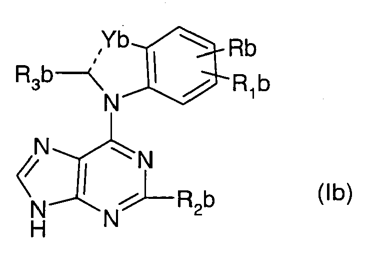 in which:
Yb represents N, CHR3b or =CR3b
the dashed line on the ring indicating that the corresponding bond is single or double,
Rb and R1b, which may be identical or different, represent hydrogen, halogen, hydroxyl, alkyl, alkoxy, cyano, NO₂, trifluoromethyl, trifluoromethoxy, phenyl,
-S(O)n-NR4bR5b,
n represents an integer of 0 to 2,
R3b represents hydrogen, halogen, alkyl, cyano, NO₂, amino, alkylamino, dialkylamino, trifluoromethyl and phenyl,
R2b represents a radical R4b or NR4bR5b in which R4b represents a hydrogen atom or an alkyl, cycloalkyl or phenyl radical,
NR4bR5b being such that either R4b and R5b, which may be identical or different, are chosen from the values for R4b, or R4b and R5b form, together with the nitrogen atom to which they are attached, an optionally substituted piperidyl, morpholinyl or pyrrolidinyl radical,
all the alkyl radicals, alkoxy radicals, cycloalkyl radicals, phenyl radicals, phenylalkyl radicals and heterocyclic radicals, such as piperidyl, morpholinyl and pyrrolidinyl, defined above being optionally substituted with one or two radicals chosen from halogen atoms, hydroxyl radicals, cyano radicals, trifluoromethyl radicals, trifluoromethoxy radicals, alkoxy radicals, phenyl radicals, tetrahydropyranyl radicals and piperidyl radicals, themselves optionally substituted on N or C with a carboxyl radical which is free, salified or esterified with an alkyl radical, and the radicals -NalkR4a, -NHR4a and -COOR4a in which R4a has the meaning indicated above and alk represents an alkyl radical,
all the phenyl and heterocyclic radicals defined above also being optionally substituted with one or more alkyl, hydroxyalkyl and phenylalkyl radicals,
all the alkyl and alkoxy radicals defined above being linear or branched and containing at most 4 carbon atoms,
all the cycloalkyl radicals defined above containing 5 or 6 carbon atoms,
said products of formula (Ib) being in all the possible racemic, enantiomeric and diastereoisomeric isomer forms, and also the pharmaceutically acceptable addition salts with inorganic and organic acids or with inorganic and organic bases of said products of formula (Ib).

5. Products of formula (I) as defined in claim 1, corresponding to formula (Ic): 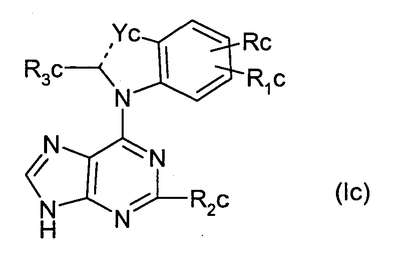 in which:
Yc represents N, CH₂ or CH=, the dashed line on the ring indicating that the corresponding bond is single or double,
Rc and R1c, which may be identical or different, represent hydrogen, halogen, hydroxyl, alkyl, alkoxy, phenylalkoxy, phenylalkyl, cyano, NO₂, trifluoromethyl, trifluoromethoxy, phenyl, -S(O)n-NH₂ optionally substituted on the nitrogen atom with one or two alkyl radicals and n represents an integer of 0 to 2,
R3c represents hydrogen, halogen, alkyl, cyano, NO₂, trifluoromethyl and phenyl,
R2c represents a radical NR4cR5c in which either R4c and R5c, which may be identical or different, are such that R4c represents a hydrogen atom or an alkyl, cycloalkyl, phenyl or phenylalkyl radical,
the alkyl, cycloalkyl, phenyl and phenylalkyl radicals being optionally substituted with one or more radicals chosen from hydroxyl, amino or carboxyl which is free, salified or esterified with an alkyl radical, tetrahydropyranyl radical or piperidyl radical, optionally substituted on N or C with a carboxyl radical which is free, salified or esterified with an alkyl radical,
and R5c represents a hydrogen atom or an alkyl radical, or R4c and R5c form, together with the nitrogen atom to which they are attached, a piperidyl, morpholinyl or pyrrolidinyl radical, these radicals being optionally substituted with an alkyl, hydroxyalkyl, amino, monoalkylamino or dialkylamino radical,
all the alkyl and alkoxy radicals defined above being linear or branched containing at most 4 carbon atoms, said products of formula (Ic) being in all the possible racemic, enantiomeric and diastereoisomeric isomer forms, and also the pharmaceutically acceptable addition salts with inorganic and organic acids or with inorganic and organic bases of said products of formula (Ic).

6. Products of formula (I) as defined in Claim 1, corresponding to formula (Id): 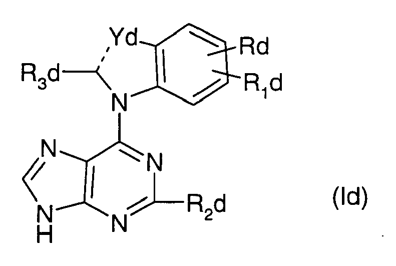 in which:
Yd represents N, CH₂ or CH=,
the dashed line on the ring indicating that the corresponding bond is single or double,
Rd and R1d, which may be identical or different, represent hydrogen, halogen, alkyl, alkoxy, phenylalkoxy, NO₂, dialkylaminosulfonyl, -NH₂, trifluoromethyl, -CO-CH₃, -NH-CO-alkyl or -NH-CO-NH-phenyl in which the alkyl radical is optionally substituted with a thienyl or phenyl radical and the phenyl radical is optionally substituted with one or more radicals chosen from halogen atoms and the radicals -NH₂, -NHAlk and -N(Alk)₂,
R3d represents hydrogen or alkyl,
R2d represents a radical NR4dR5d in which either R4d and R5d, which may be identical or different, are such that R4d represents a hydrogen atom, a linear or branched alkyl radical containing 1 to 4 carbon atoms and optionally substituted with one or two hydroxyl(s), a cycloalkyl radical optionally substituted with an amino or hydroxyl radical or a phenyl or phenylalkyl (1 to 4 C) radical with phenyl optionally substituted with a carboxyl radical which is free, salified or esterified with an alkyl radical, a tetrahydropyranalkyl (Ex. 28) radical or a piperidylalkyl (Ex. 31, 36) radical optionally substituted on N or C with a carboxyl radical,
and R5d represents a hydrogen atom or an alkyl radical, or R4d and R5d form, together with the nitrogen atom to which they are attached, a piperidyl radical optionally substituted with an amino radical, a morpholinyl radical or a pyrrolidinyl (ex 34) radical optionally substituted with a hydroxyalkyl radical,
all the alkyl and alkoxy radicals defined above being linear or branched containing at most 4 carbon atoms, said products of formula (Id) being in all the possible racemic, enantiomeric and diastereoisomeric isomer forms, and also the pharmaceutically acceptable addition salts with inorganic and organic acids or with inorganic and organic bases of said products of formula (Id) .

7. Products of formula (I) as defined in Claim 1, corresponding to formula (Id): 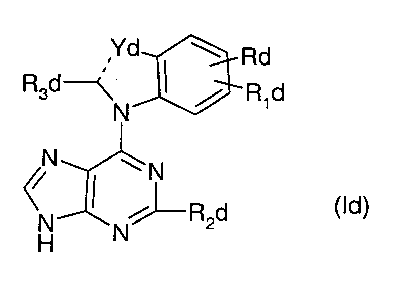 in which:
Yd represents N, CH₂ or CH=,
the dashed line on the ring indicating that the corresponding bond is single or double,
Rd and R1d, which may be identical or different, represent hydrogen, halogen, alkyl, alkoxy, phenylalkoxy, NO₂, dialkylaminosulfonyl,
R3d represents hydrogen or alkyl,
R2d represents a radical NR4dR5d in which either R4d and R5d, which may be identical or different, are such that R4d represents a hydrogen atom, a linear or branched alkyl radical containing 1 to 4 carbon atoms and optionally substituted with one or two hydroxyl(s), a cycloalkyl radical optionally substituted with an amino or hydroxyl radical or a phenyl or phenylalkyl (1 to 4 C) radical with phenyl optionally substituted with a carboxyl radical which is free, salified or esterified with an alkyl radical, a tetrahydropyranalkyl (Ex. 28) radical or a piperidylalkyl (Ex. 31, 36) radical optionally substituted on N or C with a carboxyl radical,
and R5d represents a hydrogen atom or an alkyl radical, or R4d and R5d form, together with the nitrogen atom to which they are attached, a piperidyl radical optionally substituted with an amino radical, a morpholinyl radical or a pyrrolidinyl (Ex. 34) radical optionally substituted with a hydroxyalkyl radical,
all the alkyl and alkoxy radicals defined above being linear or branched containing at most 4 carbon atoms, said products of formula (Id) being in all the possible racemic, enantiomeric and diastereoisomeric isomer forms, and also the pharmaceutically acceptable addition salts with inorganic and organic acids or with inorganic and organic bases of said products of formula (Id).

8. Products of formula (I) as defined in Claim 1, having the following names:
- trans-N-[6-(5,6-dichloro-2,3-dihydro-1H-indol-1-yl)-9H-purin-2-yl]-1,4-cyclohexanediamine dihydrochloride
- trans-N-[6-(1H-benzimidazol-1-yl)-9H-purin-2-yl]-1,4-cyclohexanediamine dihydrochloride
- trans-N-[6-(5,6-dimethyl-1H-benzimidazol-1-yl)-9H-purin-2-yl]-1,4-cyclohexanediamine
- trans-N-[6-(5,6-dichloro-1H-benzimidazol-1-yl)-9H-purin-2-yl]-1,4-cyclohexanediamine hydrochloride
- trans-N-[6-(5-methoxy-1H-benzimidazol-1-yl)-9H-purin-2-yl]-1,4-cyclohexanediamine dihydrochloride
- trans-N-[6-(1H-indol-1-yl)-9H-purin-2-yl]-1,4-cyclohexanediamine dihydrochloride
- trans-N-[6-[6-(phenylmethoxy)-1H-benzimidazol-1-yl]-9H-purin-2-yl]-1,4-cyclohexanediamine
- trans-N-[6-[5-(phenylmethoxy)-1H-benzimidazol-1-yl]-9H-purin-2-yl]-1,4-cyclohexanediamine
- trans-4-[[6-(1H-benzimidazol-1-yl)-9H-purin-2-yl]-amino]cyclohexanol
- trans-N-[6-(2,3-dihydro-5-nitro-1H-indol-1-yl)-9H-purin-2-yl]-1,4-cyclohexanediamine dihydrochloride
- trans-N-[6-(2,3-dihydro-6-(trifluoromethyl)-1H-indol-1-yl)-9H-purin-2-yl]-1,4-cyclohexanediamine (Ex. 40)
- trans-N-[1-[2-[(4-aminocyclohexyl)amino]-9H-purin-6-yl]-2,3-dihydro-1H-indol-5-yl]-2-thiopheneacetamide (Ex. 41)
- trans-N-[6-(6-nitro-2,3-dihydro-1H-indol-1-yl)-9H-purin-2-yl]-1,4-cyclohexanediamine (Ex. 44).

9. Method for preparing the products of formula (I), as defined in Claim 1, **characterized in that** the compound of formula (II): 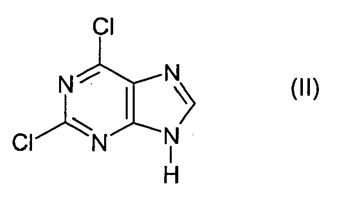 is subjected to the action of a compound of formula (III) : 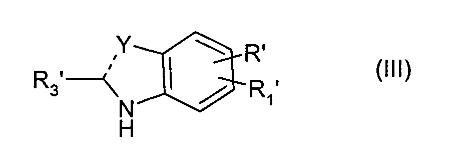 in which R', R1' and R3' have the meanings indicated respectively in claim 1 for R, R1 and R3, in which the optional reactive functions are optionally protected with protective groups, and Y has the meaning indicated in Claim 1,
so as to obtain a product of formula (IV): 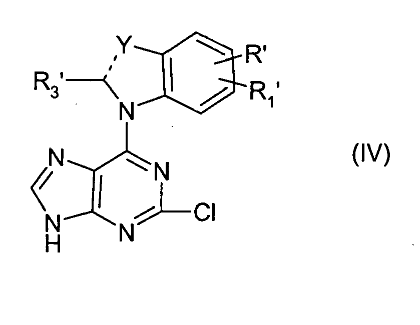 in which R', R1', R3' and Y have the meanings indicated above,
which product of formula (IV) is subjected to a reaction with a compound of formula (V):
R2'-H (V)
in which R2' has the meaning indicated in Claim 1 for R2 in which the optional reactive functions are optionally protected with protective groups,
so as to obtain a product of formula (I'): 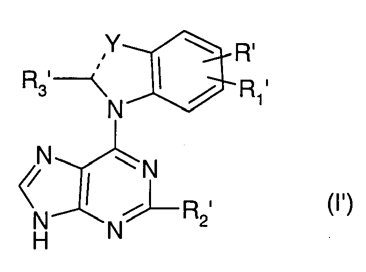 in which R', R1', R2', R3' and Y' have the meanings indicated above,
the products of formula (I') having the meaning indicated in Claim 1 for the products of formula (I) in which the optional reactive functions are optionally protected with protective groups,
which products of formula (I') can be products of formula (I) and which, so as to obtain products of formula (I) or other products of formula (I), can be subjected, if desired and if necessary, to one or more of the following conversion reactions, in any order:
a) a reaction of esterification of an acid function,
b) a reaction of saponification of an ester function to an acid function,
c) a reaction of oxidation of an alkylthio group to a corresponding sulfoxide or sulfone,
d) a reaction of conversion of a ketone function to an oxime function,
e) a reaction of reduction of the free or esterified carboxyl function to an alcohol function,
f) a reaction of conversion of an alkoxy function to a hydroxyl function, or else of a hydroxyl function to an alkoxy function,
g) a reaction of oxidation of an alkyl function to an aldehyde, acid or ketone function,
h) a reaction of conversion of a nitrile radical to a tetrazolyl,
i) a reaction of removal of protective groups which the protected reactive functions may carry,
j) a reaction of salification with an inorganic or organic acid or with a base so as to obtain the corresponding salt,
k) a reaction to resolve the racemic forms into resolved products,
said products of formula (I) thus obtained being in all the possible racemic, enantiomeric and diastereoisomeric isomer forms.

10. As medicinal products, the products of formula (I) as defined in any one of Claims 1 to 5, and also the pharmaceutically acceptable addition salts with inorganic and organic acids or with inorganic and organic bases of said products of formula (Id).

11. As medicinal products, the products of formula (I) as defined in Claim 6, and also the pharmaceutically acceptable addition salts with inorganic and organic acids or with inorganic and organic bases.

12. Pharmaceutical compositions containing, as active principle, at least one of the medicinal products as defined in Claim 8 or 9.

13. Use of the products of formula (I) as defined in any one of Claims 1 to 6, and/or of their pharmaceutically acceptable salts, for preparing medicinal products intended for the prevention or treatment of fungal diseases.

14. Use of the products of formula (I) as defined in any one of Claims 1 to 6, and/or of their pharmaceutically acceptable salts, for preparing medicinal products intended for the prevention or treatment of fungal diseases such as in particular candidiasis, aspergillosis, histoplasmosis and coccidiosis.

15. Use of the products of formula (I) as defined in any one of Claims 1 to 6, and/or of their pharmaceutically acceptable salts, for preparing medicinal products intended for the prevention or treatment of diseases caused by Candida albicans.

16. Use of the products of formula (I) as defined in any one of Claims 1 to 6, and/or of their pharmaceutically acceptable salts, for preparing medicinal products intended for the prevention or treatment of systemic candidiasis.

17. Products of formula (I) as defined in Claim 1 having antifungal properties, as inhibitors of Candida albicans CIV1 protein kinases.

18. Pharmaceutical compositions containing, as active principle, at least one inhibitor of Candida albicans CIV1 protein kinases as defined in claim 17.

19. As novel industrial products, the compounds of formula (IV).

20. Pharmaceutical compositions according to Claim 16, **characterized in that** they are used as antimitotic medicinal products, in particular for cancer chemotherapy or else for the treatment of psoriasis, of parasitic diseases, such as those due to fungi or to protists, or of Alzheimer's disease.

21. Pharmaceutical compositions according to Claim 16, **characterized in that** they are used as antineurodegenerative, in particular anti-neuronal apoptosis, medicinal products.

22. Use of the products of formula (I) as defined in Claims 1 to 6 for preparing medicinal products intended for cancer chemotherapy, for the treatment of psoriasis or of parasitic diseases such as those due to fungi or to protists, for the treatment of Alzheimer's disease or for the treatment of neurodegenerative disorders, in particular neuronal apoptosis.

23. Use of the products of formula (I), and/or of their pharmaceutically acceptable salts, as defined in Claims 1 to 6, for preparing medicinal products intended for the prevention or treatment of diseases associated with a disturbance of the secretion and/or of the activity of protein tyrosine kinases and of serine/threonine kinases.

24. Use of the products of formula (I), and/or of their pharmaceutically acceptable salts, as defined in Claims 1 to 6, for preparing medicinal products intended for the treatment or prevention of immunity, infection, allergy, and autoimmune diseases.

25. Use of the products of formula (I), and/or of their pharmaceutically acceptable salts, as defined in Claims 1 to 6, for preparing medicinal products intended for the treatment or prevention of diseases such as proliferative diseases, cancer, restenosis, inflammation, allergies or cardiovascular diseases.

## Patentansprüche

1. Produkte der Formel (I): 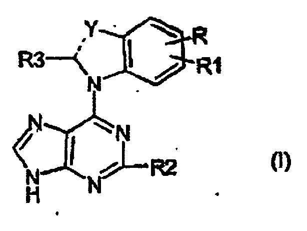 worin:
Y für N, O, S, CHR3 oder =CR3 steht
die gestrichelte Linie an dem Ring anzeigt, dass die entsprechende Bindung eine Einfach- oder Doppelbindung ist,
R und R1, die gleich oder verschieden sind, für Wasserstoff, Halogen, Hydroxyl, Alkyl, Alkoxy, Cyano, NO2, NR4R5, Trifluormethyl, Trifluormethoxy, Aryl, Heteroaryl, -S(O)n-NR4R5, Acyl, -NH-CO-Alkyl oder -NH-CO-NH-Phenyl stehen, wobei die Alkyl- und Phenylreste gegebenenfalls mit einem oder mehreren Resten substituiert sind, ausgewählt aus Thienyl und gegebenenfalls selbst substituiertem Phenyl, wobei diese Phenylreste gegebenenfalls selbst mit einem oder mehreren Resten substituiert sind, ausgewählt aus Halogenatomen und den Resten -NH2, -NHAlk und -N(Alk)2,
n für eine ganze Zahl von 0 bis 2 steht,
R3 für Wasserstoff, Halogen, Alkyl, Cyano, N02, NR4R5, Trifluormethyl, Aryl steht,
R2 für einen Rest R4, OR4, SR4 oder NR4R5 steht, worin R4 für ein Wasserstoffatom oder einen Alkyl-, Cycloalkyl- oder Arylrest steht,
NR4R5 derart ist, dass entweder R4 und R5, die gleich oder verschieden sind, aus den Werten für R4 ausgewählt sind, oder R4 und R5 zusammen mit dem Stickstoffatom, an das sie gebunden sind, einen cyclischen Rest mit 4 bis 6 Ringgliedern bilden, der ein oder mehrere gleiche oder verschiedene Heteroatome, ausgewählt aus N, O und S, enthält,
wobei alle vorstehend definierten Alkyl-, Alkoxy-, Cycloalkyl-, Aryl und heterocyclischen Reste gegebenenfalls mit einem oder mehreren Resten substituiert sind, ausgewählt aus Halogenatomen, Hydroxyl-, Cyano-, Trifluormethyl-, Trifluormethoxy-, Alkoxy-, Aryl-, heterocyclischen Resten, Resten mit Säurefunktion und Säureisosteren und den Resten -NHR4, -NAlkR4, -COR4, -COOR4, CONAlkR4 und -CONHR4, wobei R4 die vorstehend angegebene Bedeutung hat und Alk für einen Alkylrest steht,
wobei alle vorstehend definierten Aryl- und heterocyclischen Reste außerdem gegebenenfalls mit einem oder mehreren Alkyl-, Hydroxyalkyl- und Phenylalkylresten substituiert sind,
wobei alle vorstehend definierten Arylreste außerdem gegebenenfalls mit einem Dioxolrest substituiert sind,
wobei alle vorstehend definierten Alkyl- und Alkoxyreste gerade oder verzweigt sind und höchstens 6 Kohlenstoffatome enthalten,
wobei alle vorstehend definierten Cycloalkylreste höchstens 6 Kohlenstoffatome enthalten,
wobei selbstverständlich ist, dass R, R1, R2, R3 nicht sämtlich Wasserstoff sind,
wobei die Produkte der Formel (I) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (I).

2. Produkte der Formel (I): 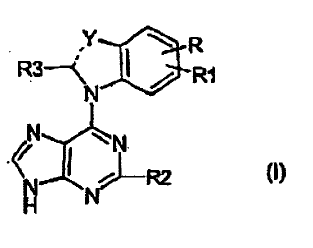 worin:
Y für N, O, S, CHR3 oder =CR3 steht
die gestrichelte Linie an dem Ring anzeigt, dass die entsprechende Bindung eine Einfach- oder Doppelbindung ist,
R und R1, die gleich oder verschieden sind, für Wasserstoff, Halogen, Hydroxyl, Alkyl, Alkoxy, Cyano, N02, NR4R5, Trifluormethyl, Trifluormethoxy, Aryl, Heteroaryl,
-S(O)n-NR4R5
stehen,
n für eine ganze Zahl von 0 bis 2 steht,
R3 für Wasserstoff, Halogen, Alkyl, Cyano, NO2, NR4R5, Trifluormethyl, Aryl steht,
R2 für einen Rest R4, OR4, SR4 oder NR4R5 steht, worin R4 für ein Wasserstoffatom oder einen Alkyl-, Cycloalkyl- oder Arylrest steht,
NR4R5 derart ist, dass entweder R4 und R5, die gleich oder verschieden sind, aus den Werten für R4 ausgewählt sind, oder R4 und R5 zusammen mit dem Stickstoffatom, an das sie gebunden sind, einen cyclischen Rest mit 4 bis 6 Ringgliedern bilden, der ein oder mehrere gleiche oder verschiedene Heteroatome, ausgewählt aus N, O und S, enthält,
wobei alle vorstehend definierten Alkyl-, Alkoxy-, Cycloalkyl-, Aryl und heterocyclischen Reste gegebenenfalls mit einem oder mehreren Resten substituiert sind, ausgewählt aus Halogenatomen, Hydroxyl-, Cyano-, Trifluormethyl-, Trifluormethoxy-, Alkoxy-, Aryl-, heterocyclischen Resten, Resten mit Säurefunktion und Säureisosteren und den Resten -NHR4, -NAlkR4, -COR4, -COOR4, CONAlkR4 und -CONHR4, wobei R4 die vorstehend angegebene Bedeutung hat und Alk für einen Alkylrest steht,
wobei alle vorstehend definierten Aryl- und heterocyclischen Reste außerdem gegebenenfalls mit einem oder mehreren Alkyl-, Hydroxyalkyl- und Phenylalkylresten substituiert sind,
wobei alle vorstehend definierten Arylreste außerdem gegebenenfalls mit einem Dioxolrest substituiert sind,
wobei alle vorstehend definierten Alkyl- und Alkoxyreste gerade oder verzweigt sind und höchstens 6 Kohlenstoffatome enthalten,
wobei alle vorstehend definierten Cycloalkylreste höchstens 6 Kohlenstoffatome enthalten,
wobei die Produkte der Formel (I) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (I).

3. Produkte der Formel (I) nach Anspruch 1, die der Formel (Ia) entsprechen: 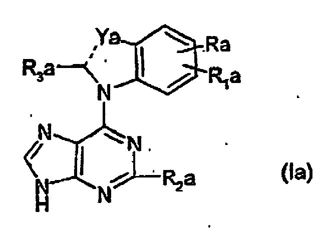 worin:
Ya für N, O, S, CHR3a oder =CR3a steht
die gestrichelte Linie an dem Ring anzeigt, dass die entsprechende Bindung eine Einfach- oder Doppelbindung ist,
Ra und R1a, die gleich oder verschieden sind, für Wasserstoff, Halogen, Hydroxyl, Alkyl, Alkoxy, Cyano, NO2, NR4aR5a, Trifluormethyl, Trifluormethoxy, Aryl, Heteroaryl,
-S(O)n-NR4aR5a
stehen,
n für eine ganze Zahl von 0 bis 2 steht,
R3a für Wasserstoff, Halogen, Alkyl, Cyano, NO2, Amino, Alkylamino, Dialkylamino, Trifluormethyl und Phenyl steht,
R2a für einen Rest R4a, OR4a, SR4a oder NR4aR5a steht, worin R4a für ein Wasserstoffatom oder einen Alkyl-, Cycloalkyl- oder Phenylrest steht,
NR4aR5a derart ist, dass entweder R4a und R5a, die gleich oder verschieden sind, aus den Werten für R4a ausgewählt sind, oder R4a und R5a zusammen mit dem Stickstoffatom, an das sie gebunden sind, einen gegebenenfalls substituierten Piperidyl-, Morpholinyl-, Pyrrolidinyl- oder Piperazinylrest bilden, wobei alle vorstehend definierten Alkyl-, Alkoxy-, Cycloalkyl-, Phenyl-, Phenylalkyl- und heterocyclischen Reste (wie die von NR4aR5a gebildeten) gegebenenfalls mit einem oder mehreren Resten substituiert sind, ausgewählt aus Halogenatomen, Hydroxyl-, Cyano-, Trifluormethyl-, Trifluormethoxy-, Alkoxy-, Phenyl-, heterocyclischen Resten, die gegebenenfalls an N oder C mit einem freien, versalzten oder mit einem Alkylrest veresterten Carboxyrest substituiert sind, den Resten S03H, PO(OH)2, NH-SO2-CF3, NH-SO2-NH-V und NH-S02-NH-CO-V, in denen V für einen Phenyl-, Alkyl- oder Alkenylrest steht, wobei die Alkenylreste gerade oder verzweigt sind und höchstens 6 Kohlenstoffatome enthalten, und den Resten -NAlkR4a, -NHR4a, -COR4a, -COOR4a, CONAlkR4a und -CONHR4a, worin R4a die vorstehend angegebene Bedeutung hat und Alk für einen Alkylrest steht,
wobei alle vorstehend definierten Phenyl- und heterocyclischen Reste außerdem gegebenenfalls mit einem oder mehreren Alkyl-, Hydroxyalkyl- und Phenylalkylresten substituiert sind,
wobei alle vorstehend definierten Phenylreste außerdem gegebenenfalls mit einem Dioxolrest substituiert sind,
wobei alle vorstehend definierten Alkyl- und Alkoxyreste gerade oder verzweigt sind und höchstens 6 Kohlenstoffatome enthalten,
wobei alle vorstehend definierten Cycloalkylreste 5 oder 6 Kohlenstoffatome enthalten,
wobei die Produkte der Formel (Ia) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (Ia).

4. Produkte der Formel (I) nach Anspruch 1, die der Formel (Ib) entsprechen: 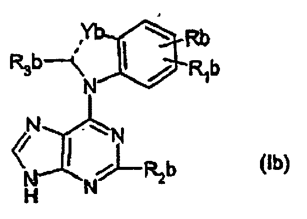 worin:
Yb für N, CHR3b oder =CR3b steht
die gestrichelte Linie an dem Ring anzeigt, dass die entsprechende Bindung eine Einfach- oder Doppelbindung ist,
Rb und R1b, die gleich oder verschieden sind, für Wasserstoff, Halogen, Hydroxyl, Alkyl, Alkoxy, Cyano, N02, Trifluormethyl, Trifluormethoxy, Phenyl,
-S(O)n-NR4bR5b
stehen,
n für eine ganze Zahl von 0 bis 2 steht,
R3b für Wasserstoff, Halogen, Alkyl, Cyano, N02, Amino, Alkylamino, Dialkylamino, Trifluormethyl und Phenyl steht,
R2b für einen Rest R4b oder NR4bR5b steht, worin R4b für ein Wasserstoffatom oder einen Alkyl-, Cycloalkyl- oder Phenylrest steht,
NR4bR5b derart ist, dass entweder R4b und R5b, die gleich oder verschieden sind, aus den Werten für R4b ausgewählt sind, oder R4b und R5b zusammen mit dem Stickstoffatom, an das sie gebunden sind, einen gegebenenfalls substituierten Piperidyl-, Morpholinyl-, Pyrrolidinyl- oder Piperazinylrest bilden,
wobei alle vorstehend definierten Alkyl-, Alkoxy-, Cycloalkyl-, Phenyl-, Phenylalkyl-, heterocyclischen Reste, wie Piperidyl, Morpholinyl und Pyrrodidinyl, gegebenenfalls mit einem oder zwei Resten substituiert sind, ausgewählt aus Halogenatomen, Hydroxyl-, Cyano-, Trifluormethyl-, Trifluormethoxy-, Alkoxy-, Phenyl-, Tetrahydropyranyl-, Piperiylresten, die gegebenenfalls selbst an N oder C mit einem freien, versalzten oder mit einem Alkylrest veresterten Carboxyrest substituiert sind, und den Resten -NAlkR4a, -NHR4a und -COOR4a, worin R4a die vorstehend angegebene Bedeutung hat und Alk für einen Alkylrest steht,
wobei alle vorstehend definierten Phenyl- und heterocyclischen Reste außerdem gegebenenfalls mit einem oder mehreren Alkyl-, Hydroxyalkyl- und Phenylalkylresten substituiert sind,
wobei alle vorstehend definierten Alkyl- und Alkoxyreste gerade oder verzweigt sind und höchstens 4 Kohlenstoffatome enthalten,
wobei alle vorstehend definierten Cycloalkylreste 5 oder 6 Kohlenstoffatome enthalten,
wobei die Produkte der Formel (Ib) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (Ib).

5. Produkte der Formel (I) nach Anspruch 1, die der Formel (Ic) entsprechen: 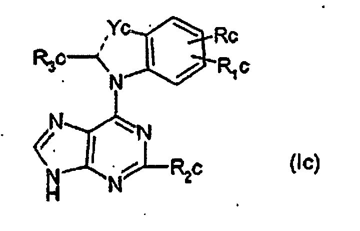 worin:
Yc für N, CH2 oder CH= steht
die gestrichelte Linie an dem Ring anzeigt, dass die entsprechende Bindung eine Einfach- oder Doppelbindung ist,
Rc und R1c, die gleich oder verschieden sind, für Wasserstoff, Halogen, Hydroxyl, Alkyl, Alkoxy, Phenylalkoxy, Phenylalkyl, Cyano, NO2, Trifluormethyl, Trifluormethoxy, Phenyl, -S(O)n-NH2 stehen, das gegebenenfalls am Stickstoffatom mit einem oder zwei Alkylresten substituiert ist, und n für eine ganze Zahl von 0 bis 2 steht,
R3c für Wasserstoff, Halogen, Alkyl, Cyano, N02, Trifluormethyl und Phenyl steht,
R2c für einen Rest NR4cR5c steht, worin R4c und R5c, die gleich oder verschieden sind, derart sind, dass entweder R4c für ein Wasserstoffatom oder einen Alkyl-, Cycloalkyl-, Phenyl- oder Phenylalkylrest steht,
wobei die Alkyl-, Cycloalkyl-, Phenyl- und Phenylalkylreste gegebenenfalls mit einem oder mehreren Resten substituiert sind, ausgewählt aus Hydroxyl, Amino, freiem, versalztem oder mit einem Alkyl verestertem Carboxy, Tetrahydropyranyl, Piperidyl, das gegebenenfalls an N oder C mit einem freien, versalzten oder mit einem Alkylrest veresterten Carboxyrest substituiert ist,
und R5c für ein Wasserstoffatom oder einen Alkylrest steht,
oder R4c und R5c zusammen mit dem Stickstoffatom, an das sie gebunden sind, einen Piperidyl-, Morpholinyl- oder Pyrrolidinylrest bilden, wobei diese Reste gegebenenfalls mit einem Alkyl-, Hydroxyalkyl-, Amino-, Monoalkylamino- oder Dialkylaminorest substituiert sind,
wobei alle vorstehend definierten Alkyl- und Alkoxyreste gerade oder verzweigt sind und höchstens 4 Kohlenstoffatome enthalten,
wobei die Produkte der Formel (Ic) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (Ic).

6. Produkte der Formel (I) nach Anspruch 1, die der Formel (Id) entsprechen: 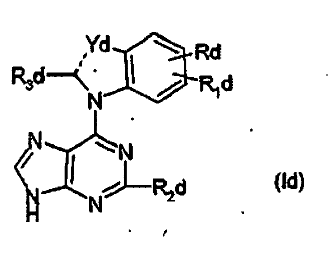 worin:
Yd für N, CH2 oder CH= steht
die gestrichelte Linie an dem Ring anzeigt, dass die entsprechende Bindung eine Einfach- oder Doppelbindung ist,
Rd und R1d, die gleich oder verschieden sind, für Wasserstoff, Halogen, Alkyl, Alkoxy, Phenylalkoxy, NO2, Dialkylaminosulfonyl, -NH2, Trifluormethyl, -CO-CH3, -NH-CO-Alkyl oder -NH-CO-NH-Phenyl stehen, in denen der Alkylrest gegebenenfalls mit einem Thienyl- oder Phenylrest substituiert ist und der Phenylrest gegebenenfalls mit einem oder mehreren Resten substituiert ist, ausgewählt aus Halogenatomen und den Resten -NH2, -NHAlk und -N(alk)2,
R3d für Wasserstoff oder Alkyl steht,
R2d für einen Rest NR4dR5d steht, worin entweder R4d und R5d, die gleich oder verschieden sind, derart sind, dass R4d für ein Wasserstoffatom, einen geraden oder verzweigten Alkylrest mit 1 bis 4 Kohlenstoffatomen, der gegebenenfalls mit einer oder zwei Hydroxylgruppen substituiert ist, einen Cycloalkylrest, der gegebenenfalls mit einem Amino- oder Hydroxylrest substituiert ist, Phenyl oder Phenylalkyl (1 bis 4 C) steht, wobei Phenyl gegebenenfalls mit einem freien, versalzten oder einem Carboxyrest substituiert ist, der mit einem Alkyl-, Tetrahydropyranalkyl- (Bsp. 28), Piperidylalkylrest (Bsp. 31, 36), der gegebenenfalls an N oder C mit einem Carboxyrest substituiert ist, verestert ist,
und R5d für ein Wasserstoffatom oder einen Alkylrest steht,
oder R4d und R5d zusammen mit dem Stickstoffatom, an das sie gebunden sind, einen gegebenenfalls mit einem Aminorest substituierten Piperidyl-, einen Morpholinyl-, einen Pyrrolidinylrest (Bsp. 34), der gegebenenfalls mit einem Hydroxyalkylrest substituiert ist, bilden,
wobei alle vorstehend definierten Alkyl- und Alkoxyreste gerade oder verzweigt sind und höchstens 4 Kohlenstoffatome enthalten,
wobei die Produkte der Formel (Id) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (Id).

7. Produkte der Formel (I) nach Anspruch 1, die der Formel (Id) entsprechen: 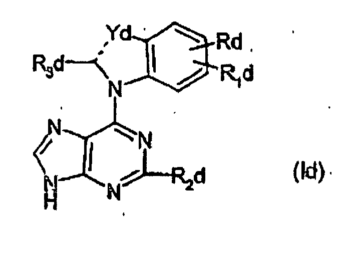 worin:
Yd für N, CH2 oder CH= steht
die gestrichelte Linie an dem Ring anzeigt, dass die entsprechende Bindung eine Einfach- oder Doppelbindung ist,
Rd und R1d, die gleich oder verschieden sind, für Wasserstoff, Halogen, Alkyl, Alkoxy, Phenylalkoxy, NO2, Dialkylaminosulfonyl stehen,
R3d für Wasserstoff oder Alkyl steht,
R2d für einen Rest NR4dR5d steht, worin entweder R4d und R5d, die gleich oder verschieden sind, derart sind, dass R4d für ein Wasserstoffatom, einen geraden oder verzweigten Alkylrest mit 1 bis 4 Kohlenstoffatomen, der gegebenenfalls mit einer oder zwei Hydroxylgruppen substituiert ist, einen Cycloalkylrest, der gegebenenfalls mit einem Amino- oder Hydroxylrest substituiert ist, Phenyl oder Phenylalkyl (1 bis 4 C) steht, wobei Phenyl gegebenenfalls mit einem freien, versalzten oder mit einem Carboxyrest substituiert ist, der mit einem Alkyl-, Tetrahydropyranalkyl- (Bsp. 28), Piperidylalkylrest (Bsp. 31, 36), der gegebenenfalls an N oder C mit einem Carboxyrest substituiert ist, verestert ist,
und R5d für ein Wasserstoffatom oder einen Alkylrest steht,
oder R4d und R5d zusammen mit dem Stickstoffatom, an das sie gebunden sind, einen gegebenenfalls mit einem Aminorest substituierten Piperidyl-, einen Morpholinyl-, einen Pyrrolidinylrest (Bsp. 34), der gegebenenfalls mit einem Hydroxyalkylrest substituiert ist, bilden,
wobei alle vorstehend definierten Alkyl- und Alkoxyreste gerade oder verzweigt sind und höchstens 4 Kohlenstoffatome enthalten,
wobei die Produkte der Formel (Id) in allen möglichen racemischen, enantiomeren und diastereoisomeren, isomeren Formen vorliegen, sowie die pharmazeutisch annehmbaren Additionssalze mit mineralischen und organischen Säuren oder mit mineralischen und organischen Basen der Produkte der Formel (Id).

8. Produkte der Formel (I) nach Anspruch 1 mit folgenden Bezeichnungen:
- das Dihydrochlorid von trans-N-[6-(5,6-Dichlor-2,3-dihydro-1H-indol-1-yl)-9H-purin-2-yl]-1,4-cyclohexandiamin
- das Dihydrochlorid von trans-N-[6-(1H-Benzimidazol-1-yl)-9H-purin-2-yl]-1,4-cyclohexandiamin
- trans-N-[6-(5,6-Dimethyl-1H-benzimidazol-1-yl)-9H-purin-2-yl]-1,4-cyclohexandiamin
- das Hydrochlorid von trans-N-[6-(5,6-Dichlor-1H-benzimidazol-1-yl)-9H-purin-2-yl]-1,4-cyclohexandiamin
- das Dihydrochlorid von trans-N-[6-(5-Methoxy-1H-benzimidazol-1-yl)-9H-purin-2-yl]-1,4-cyclohexandiamin
- das Dihydrochlorid von trans-N-[6-(1H-indol-1-yl)-9H-purin-2-yl]-1,4-cyclohexandiamin
- trans-N-[6-[6-(Phenylmethoxy)-1H-benzimidazol-1-yl]-9H-purin-2-yl]-1,4-cyclohexandiamin
- trans-N-[6-[5-(Phenylmethoxy)-1H-benzimidazol-1-yl]-9H-purin-2-yl]-1,4-cyclohexandiamin
- trans-4-[[6-(1H-Benzimidazol-1-yl)-9H-purin-2-yl]-amino]-cyclohexanol
- das Dihydrochlorid von trans-N-[6-(2,3-Dihydro-5-nitro-1H-indol-1-yl)-9H-purin-2-yl]-1,4-cyclohexandiamin
- trans-N-[6-[2,3-Dihydro-6-(trifluormethyl)-1H-indol-1-yl)-9H-purin-2-yl]-1,4-cyclohexandiamin (Bsp. 40)
- trans-N-[1-[2-[(4-Aminocyclohexyl)amino]-9H-purin-6-yl]-2,3-dihydro-1H-indol-5-yl]-2-thiophenacetamid (Bsp. 41)
- trans-N-[6-(6-Nitro-2,3-dihydro-1H-indol-1-yl)-9H-purin-2-yl]-1,4-cyclohexandiamin (Bsp. 44)

9. Verfahren zur Herstellung der Produkte der Formel (I) nach Anspruch 1, **dadurch gekennzeichnet, dass** man die Verbindung der Formel (II): 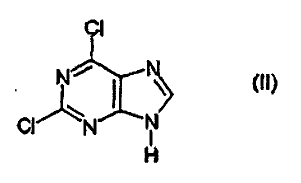 mit einer Verbindung der Formel (III): 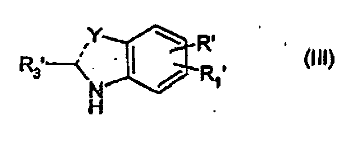 umsetzt, worin R', R1' und R3' jeweils die im Anspruch 1 für R, R1 und R3 angegebenen Bedeutungen haben, wobei die möglichen reaktiven Funktionen gegebenenfalls mit Schutzgruppen geschützt sind, und Y die im Anspruch 1 angegebene Bedeutung hat,
um ein Produkt der Formel (IV): 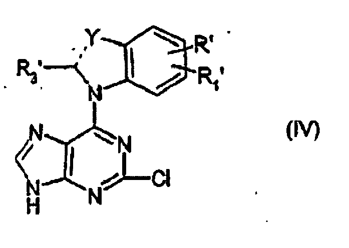 zu erhalten, worin R', R1', R3' und Y die vorstehend angegebenen Bedeutungen haben,
wobei man das Produkt der Formel (IV) einer Reaktion mit einer Verbindung der Formel (V):
R2'-(H) (V)
unterzieht, worin R2' die im Anspruch 1 für R2 angegebene Bedeutung hat, wobei die möglichen reaktiven Funktionen gegebenenfalls mit Schutzgruppen geschützt sind,
um ein Produkt der Formel (I'): 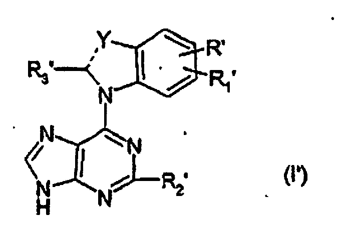 zu erhalten, worin R', R1', R2', R3' und Y' die vorstehend angegebenen Bedeutungen haben,
wobei die Produkte der Formel (I') die im Anspruch 1 für die Produkte der Formel (I) angegebene Bedeutung haben, worin die möglichen reaktiven Funktionen gegebenenfalls mit Schutzgruppen geschützt sind,
wobei die Produkte der Formel (I') Produkte der Formel (I) sein können und man sie, um andere Produkte der Formel (I) zu erhalten, wenn gewünscht und notwendig, einer oder mehreren der folgenden Umwandlungsreaktionen in beliebiger Reihenfolge unterziehen kann:
a) einer Veresterungsreaktion der Säurefunktion,
b) einer Verseifungsreaktion der Esterfunktion in eine Säurefunktion,
c) einer Oxidationsreaktion der Alkylthiogruppe in das entsprechende Sulfoxid oder Sulfon,
d) einer Umwandlungsreaktion der Ketonfunktion in eine Oximfunktion,
e) einer Reduktionsreaktion der freien oder veresterten Carboxyfunktion in eine Alkoholfunktion,
f) einer Umwandlungsfunktion der Alkoxyfunktion in eine Hydroxylfunktion oder auch der Hydroxylfunktion in eine Alkoxyfunktion,
g) einer Oxidationsreaktion der Alkoholfunktion in eine Aldehyd-, Säure- oder Ketonfunktion,
h) einer Umwandlungsreaktion des Nitrilrestes in Tetrazolyl,
i) einer Eliminationsreaktion der Schutzgruppen, welche die geschützten reaktiven Funktionen tragen können,
j) einer Versalzungsreaktion mit einer mineralischen oder organischen Säure oder einer Base, um das entsprechende Salz zu erhalten,
k) einer Auftrennungsreaktion der racemischen Formen in die aufgetrennten Produkte,
wobei diese so erhaltenen Produkte der Formel (I) in allen möglichen racemischen, enantiomeren und diastereoisomeren, isomeren Formen vorliegen.

10. Produkte der Formel (I) nach einem der Ansprüche 1 bis 5 sowie die pharmazeutisch annehmbaren Additionssalze der Produkte der Formel (Id) mit mineralischen und organischen Säuren oder mit mineralischen und organischen Basen als Arzneimittel.

11. Produkte der Formel (I) nach Anspruch 6 sowie die pharmazeutisch annehmbaren Additionssalze mit mineralischen und organischen Säuren oder mit mineralischen und organischen Basen als Arzneimittel.

12. Pharmazeutische Zusammensetzungen, die als Wirkstoff mindestens eines der Arzneimittel nach Anspruch 8 oder 9 enthalten.

13. Verwendung der Produkte der Formel (I) nach einem der Ansprüche 1 bis 6 und/oder ihrer pharmazeutisch annehmbaren Salze zur Herstellung von Arzneimitteln zur Vorbeugung oder Behandlung von Pilzerkrankungen.

14. Verwendung der Produkte der Formel (I) nach einem der Ansprüche 1 bis 6 und/oder ihrer pharmazeutisch annehmbaren Salze zur Herstellung von Arzneimitteln zur Vorbeugung oder Behandlung von Pilzerkrankungen, wie insbesondere Candidosen, Aspergillosen, Histoplasmosen und Coccidoidosen.

15. Verwendung der Produkte der Formel (I) nach einem der Ansprüche 1 bis 6 und/oder ihrer pharmazeutisch annehmbaren Salze zur Herstellung von Arzneimitteln zur Vorbeugung oder Behandlung von Erkrankungen, die durch Candida albicans hervorgerufen werden.

16. Verwendung der Produkte der Formel (I) nach einem der Ansprüche 1 bis 6 und/oder ihrer pharmazeutisch annehmbaren Salze zur Herstellung von Arzneimitteln zur Vorbeugung oder Behandlung von systemischer Candidose.

17. Produkte der Formel (I) nach Anspruch 1 mit fungiziden Eigenschaften als Inhibitoren von CIV1-Kinaseproteinen von Candida albicans.

18. Pharmazeutische Zusammensetzungen, die als Wirkstoff mindestens einen Inhibitor von CIV1-Kinaseproteinen von Candida albicans nach Anspruch 17 enthalten.

19. Verbindungen der Formel (IV) als neue industrielle Produkte.

20. Pharmazeutische Zusammensetzungen nach Anspruch 16, **dadurch gekennzeichnet, dass** sie als antimitotische Arzneimittel, insbesondere zur Chemotherapie von Krebserkrankungen oder auch zur Behandlung von Psoriasis, Parasitosen, wie denjenigen aufgrund von Pilzen oder Protisten, oder von Alzheimer-Krankheit, verwendet werden.

21. Pharmazeutische Zusammensetzungen nach Anspruch 16, **dadurch gekennzeichnet, dass** sie als antineurodegenerative Arzneimittel, insbesondere als Mittel gegen neuronale Apoptose, verwendet werden.

22. Verwendung von Produkten der Formel (I) nach den Ansprüchen 1 bis 6 zur Herstellung von Arzneimitteln zur Chemotherapie von Krebserkrankungen, zur Behandlung von Psoriasis, Parasitosen, wie denjenigen aufgrund von Pilzen oder Protisten, zur Behandlung von Alzheimer-Krankheit oder zur Behandlung von neurodegenerativen Störungen, insbesondere neuronaler Apoptose.

23. Verwendung von Produkten der Formel (I) und/oder ihrer pharmazeutisch annehmbaren Salze nach den Ansprüchen 1 bis 6 zur Herstellung von Arzneimitteln zur Vorbeugung oder Behandlung von Erkrankungen, die mit einer Funktionsstörung der Sekretion und/oder der Aktivität von Tyrosinkinase- und Serin/Threonin-Kinase-Proteinen zusammenhängen.

24. Verwendung von Produkten der Formel (I) und/oder ihrer pharmazeutisch annehmbaren Salze nach den Ansprüchen 1 bis 6 zur Herstellung von Arzneimitteln zur Behandlung oder zur Vorbeugung von Immunität, Infektion, Allergie und Autoimmunerkrankungen.

25. Verwendung von Produkten der Formel (I) und/oder ihrer pharmazeutisch annehmbaren Salze nach den Ansprüchen 1 bis 6 zur Herstellung von Arzneimitteln zur Behandlung oder Vorbeugung von Erkrankungen, wie proliferativen Erkrankungen, Krebs, Restenose, Entzündung, Allergien oder kardiovaskuläre Erkrankungen.
